# EUROPÄISCHE PATENTSCHRIFT

(11) **EP 1 827 423 B1**
(45) Veröffentlichungstag und Bekanntmachung des Hinweises auf die Patenterteilung: **30.06.2010**
(21) Anmeldenummer: 05811330.9
(22) Anmeldetag: 01.12.2005
(51) Int. Cl.: A61K 31/4174, A61K 31/433

(54) **NEUE ZYKLISCHE HARNSTOFFE ALS INHIBITOREN VON METALLOPROTEASEN**
NOVEL CYCLIC UREAS USED AS INHIBITORS OF METALLOPROTEASES
NOUVELLES UREES CYCLIQUES UTILISEES COMME INHIBITEURS DE METALLOPROTEASES

(30) Priorität: 15.12.2004 DE 102004060229
(43) Veröffentlichungstag der Anmeldung: 05.09.2007
(73) Patentinhaber: Sanofi-Aventis Deutschland GmbH, 65929 Frankfurt am Main (DE)
(72) Erfinder: LINDENSCHMIDT, Andreas, 65812 Bad Soden (DE); WAGNER, Holger, 88400 Biberach/Mettenberg (DE); BENINGA, Jochen, 55129 Mainz (DE); GRUENEBERG, Sven, 65779 Kelkheim (DE); WEITHMANN, Klaus-Ulrich, 65719 Hofheim (DE)
(86) Internationale Anmeldenummer: PCT/EP2005/012799
(87) Internationale Veröffentlichungsnummer: WO 2006/066693

(56) Entgegenhaltungen:
- WO-A-00/59285
- WO-A-02/28846
- WO-A-98/32748
- WO-A-99/06340
- WO-A-2005/077937
- US-B1- 6 376 506

## Beschreibung

Die Erfindung betrifft zyklische Harnstoffe, die geeignet sind als Inhibitoren von Metalloproteasen, insbesondere von ADAMTS Proteasen und TNF-α converting enzyme (TACE), eingesetzt zu werden, Verfahren zu deren Herstellung und deren Verwendung zur Behandlung von Erkrankungen wie Osteoarthrose und rheumatoider Arthritis.

Im Krankheitsbild der Osteoarthrose stellt die Degradation des Aggrecans, des Hauptproteoglykans des artikulären Knorpels, ein sehr frühes und entscheidendes Ereignis dar. Der pathologische Verlust des Knorpel-Aggrecans resultiert aus proteolytischen Spaltungen in dessen interglobulärer Domäne. Aminosäuresequenzanalysen von Proteoglykanmetaboliten, isoliert aus der Synovialflüssigkeit von Patienten, die an einer Gelenkschädigung, an Osteoarthrose oder an einer entzündlichen Gelenkerkrankung leiden, zeigten, daß eine proteolytische Spaltung bevorzugt zwischen den Aminosäuren Glu³⁷³ und Ala³⁷⁴ in der interglobulären Domäne des humanen Aggrecans stattfindet (Lohmander et al., Arthritis Rheum. 36, (1993), 1214-1222). Die proteolytische Aktivität, die für diese Spaltung verantwortlich ist, wird als "Aggrecanase" bezeichnet und kann zur Superfamilie der Metalloproteinasen (MP) gerechnet werden.

Bei den Metalloproteinasen ist Zink im katalytisch aktiven Zentrum essentiell. MP spalten Kollagen, Laminin, Proteoglykane, Elastin oder Gelatin unter physiologischen Bedingungen und spielen daher eine wichtige Rolle im Knochen und Bindegewebe. Eine Vielzahl von verschiedenen MP-Inhibitoren sind bekannt (J.S.Skotnicki et al., Ann. N.Y. Acad. Sci. 878, 61-72 [1999]; EP 0 606 046; WO94/28889). Einige dieser Inhibitoren sind bezüglich ihrer Spezifität wenig charakterisiert; andere sind mehr oder weniger selektiv vor allem gegen Matrix-Metallproteinasen (MMPs) gerichtet.

Aggrecanase unterscheidet sich von den Matrix-Metallproteinasen (MMPs) durch ihre andersartige, gegen bestimmte, im Aggrecan vorkommende und von MMPs nicht bevorzugte Spaltstellen gerichtete Spezifität. Durch die Spaltung entstehen charakteristische Fragmente, die unter Verwendung geeigneter Antikörper nachgewiesen werden können.

Nachteil der bekannten Inhibitoren der MMPs ist häufig die mangelnde Spezifität der Hemmung für nur eine Klasse der MMPs. Daher hemmen die meisten MMP-Inhibitoren mehrere MMPs gleichzeitig.

In dem Bestreben, wirksame Verbindungen zur Behandlung von Bindegewebserkrankungen zu finden, wurde nun gefunden, dass die Verbindungen der Formel I starke Inhibitoren der Matrix-Metalloproteinasen wie Aggrecanase, beispielsweise ADAMTS-4, ADATMS-5 oder ADAMTS-1 und Tissue Necoris Factor α (TNF-α) converting enzyme sind.

Die Erfindung betrifft daher eine Verbindung der Formel I 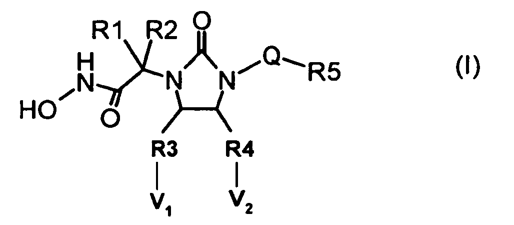 und/oder alle stereoisomeren Formen der Verbindung der Formel I und/oder Gemische diese Formen in jedem Verhältnis, und/oder ein physiologisch verträgliches Salz der Verbindung der Formel I, wobei
R1 und R2 gleich oder verschieden sind und unabhängig voneinander für
   a) Wasserstoffatom,
   b) -(C₁-C₆)-Alkyl,
   c) -(C₃-C₆)-Cycloalkyl,
   d) -(C₂-C₄)-Alkyl-Het, worin Het für einen mono- oder bizyklischen 4- bis 15-gliedrigen Heterozyklus steht, der mindestens ein Kohlenstoffatom und ein, zwei, drei oder vier Heteroatome aus der Reihe Stickstoff, Schwefel oder Sauerstoff enthält, worin der Heterozyklus unsubstituiert oder ein-, zwei- oder dreifach durch R8 substituiert ist, oder
   e) -(C₂-C₄)-Alkyl-(C₆-C₁₄)-Aryl, worin Aryl unsubstituiert oder ein- oder zweifach durch R8 substituiert ist, stehen, oder
R1 und R2 bilden zusammen mit dem Kohlenstoffatom an das sie jeweils gebunden sind
   a) -(C₃-C₆)-Cycloalkyl oder
   b) einen mono- oder bizyklischen 4- bis 15-gliedrigen Heterozyklus, der mindestens ein Kohlenstoffatom und ein, zwei, drei oder vier Heteroatome aus der Reihe Stickstoff, Schwefel oder Sauerstoff enthält, worin der Heterozyklus unsubstituiert oder ein-, zwei- oder dreifach durch R8 substituiert ist,
R3 und R4 gleich oder verschieden sind und unabhängig voneinander für
   eine kovalente Bindung, -(CH₂)ₘ-, -(C₁-C₃)-Alkylen-O-(C₀-C₃)-Alkylen-, -(C₀-C₃)-Alkylen-C(O)-O-(CH₂)ₙ-, -(C₀-C₃)-Alkylen-C(O)-NR¹⁰-(CH₂)ₙ-, -(CH₂)ₘ-CH(OH)-(CH₂)ₙ-, -(C₁-C₃)-Alkylen-N(R¹⁰)-(CH₂)ₙ-, -(CH₂)ₘ-NR¹⁰-C(O)-(CH₂)ₙ-, -(CH₂)ₘ-NR¹⁰-C(O)-NR¹⁰-(CH₂)ₙ-, -(CH₂)ₘ-O-C(O)-NR¹⁰-(CH₂)ₙ-, -(CH₂)ₘ-NR¹⁰-C(O)-O-(CH₂)ₙ-, -(CH₂)ₘ-S-(CH₂)ₙ-, -(C₁-C₃)-Alkylen-S(O)-(CH₂)ₙ-, -(C₁-C₃)-Alkylen-SO₂-(CH₂)ₙ-, -(C₁-C₃)-Alkylen-SO₂-NH-(R¹⁰) , -(CH₂)ₘ-SO₂-NR¹⁰-(CH₂)ₙ-, -(CH₂)ₘ-NR¹⁰-SO₂-(CH₂)ₙ- oder -(CH₂)ₘ-NR¹⁰-SO₂-NR¹⁰-(CH₂)ₙ- stehen, worin
   n und m unabhängig voneinander gleich oder verschieden sind und m für die ganzen Zahlen 1, 2, 3, 4, 5 oder 6 steht und n für die ganzen Zahlen Null, 1, 2, 3, 4, 5 oder 6 steht und worin die Alkylenreste, die durch -(CH₂)ₘ- oder -(CH₂)ₙ- gebildet werden unsubstituiert oder ein-, zwei- oder dreifach substituiert sind durch Halogen, -NH₂ oder -OH oder ein -(C₃-C₆)-Cycloalkyl bilden, worin Cycloalkyl unsubstituiert oder ein-, zwei- oder dreifach durch Halogen, -NH₂ oder -OH substituiert ist,
   R¹⁰ für Wasserstoffatom, -(C₁-C₆)-Alkyl, -(C₀-C₄)-Alkyl-OH,
      -(C₀-C₄)-Alkyl-O-(C₁-C₄)-Alkyl oder -(C₁-C₃)-Perfluoralkyl steht,
V_{1,} V₂ und R5 gleich oder verschieden sind und unabhängig voneinander für
   a) Wasserstoffatom,
   b) -(C₆-C₁₄)-Aryl, worin Aryl unsubstituiert oder ein- oder zweifach durch R8 oder den Rest -G-M substituiert ist oder
   c) einen mono- oder bizyklischen 4- bis 15-gliedrigen Heterozyklus stehen, der mindestens ein Kohlenstoffatom und ein, zwei, drei oder vier Heteroatome aus der Reihe Stickstoff, Schwefel oder Sauerstoff enthält, worin der Heterozyklus unsubstituiert oder ein-, zwei- oder dreifach durch R8 oder den Rest -G-M substituiert ist,
M für
   a) Wasserstoffatom,
   b) -(C₆-C₁₄)-Aryl, worin Aryl unsubstituiert oder ein- oder zweifach durch R8 substituiert ist oder
   c) einen mono- oder bizyklischen 4- bis 15-gliedrigen Heterozyklus steht, der mindestens ein Kohlenstoffatom und ein, zwei, drei oder vier Heteroatome aus der Reihe Stickstoff, Schwefel oder Sauerstoff enthält und worin der Heterozyklus unsubstituiert oder ein-, zwei- oder dreifach durch R8 substituiert ist,
      R8 für
      1) Halogen,
      2) -NO₂,
      3) -CN,
      4) -C(O)-NH₂,
      5) -SO₂-NH₂,
      6) -OH,
      7) -NH₂,
      8) -O-CF₃,
      9) -(C₆-C₁₄)-Aryl, worin Aryl unsubstituiert oder ein- oder zweifach durch Halogen oder -O-(C₁-C₈)-Alkyl substituiert ist,
      10) -(C₁-C₈)-Alkyl, worin Alkyl unsubstituiert oder ein-, zwei- oder dreifach durch Halogen, NH₂, -OH oder Methoxy substituiert ist,
      11) -O-(C₁-C₈)-Alkyl, worin Alkyl unsubstituiert oder ein-, zwei- oder dreifach durch Halogen, NH₂, -OH oder Methoxy substituiert ist,
      12) -SO₂-CH₃ oder
      13) -SO₂-CF₃ steht,
G für kovalente Bindung, -(CH₂)ₒ-, -(C₀-C₃)-Alkylen-O-(C₀-C₃)-Alkylen-, -(C₀-C₃)-Alkylen-C(O)-O-(CH₂)ₚ-, -(C₀-C₃)-Alkylen-C(O)-NR¹⁰-(CH₂)ₚ-, -(CH₂)ₒ-CH(OH)-(CH₂)ₚ-, -(C₀-C₃)-Alkylen-N(R¹⁰)-(CH₂)ₚ-, -(CH₂)ₒ-NR¹⁰-C(O)-(CH₂)ₚ-, -(CH₂)ₒ-NR¹⁰-C(O)-NR¹⁰-(CH₂)ₚ-, -(CH₂)₀-O-C(O)-NR¹⁰-(CH₂)ₚ-, -(CH₂)ₒ-NR¹⁰-C(O)-O-(CH₂)ₚ-, -(CH₂)ₒ-S-(CH₂)ₚ-, -(C₀-C₃)-Alkylen-S(O)-(CH₂)ₚ-, -(C₀-C₃)-Alkylen-SO₂-(CH₂)ₚ-, -(C₀-C₃)-Alkylen-SO₂-NH-(R¹⁰), -(CH₂)ₒ-SO₂-NR¹⁰-(CH₂)ₚ-, -(CH₂)ₒ-NR¹⁰-SO₂-(CH₂)ₚ-, -(C₀-C₃)-Alkylen-O-(C₂-C₄)-Alkenylen- oder -(CH₂)ₒ-NR¹⁰-SO₂-NR¹⁰-(CH₂)ₚ- steht, worin o und p gleich oder verschieden sind und unabhängig voneinander für die ganzen Zahlen Null, 1, 2, 3, 4, 5 oder 6 stehen und worin die Alkylenreste die durch -(CH₂)ₒ- oder -(CH₂)ₚ- gebildet werden unsubstituiert oder ein-, zwei- oder dreifach substituiert sind durch Halogen, -NH₂ oder -OH oder ein -(C₃-C₆)-Cycloalkyl bilden, worin Cycloalkyl unsubstituiert oder ein-, zwei- oder dreifach durch Halogen, -NH₂ oder -OH substituiert ist, und R10 wie oben definiert ist, und
Q für kovalente Bindung, -(C₁-C₃)-Alkylen oder -(C₃-C₆)-Cycloalkyl steht, unter der Bedingung, dass wenigstens einer der Reste V₁, V₂ oder R5 für
   -(C₆-C₁₄)-Aryl oder einen mono- oder bizyklischen 4- bis 15-gliedrigen Heterozyklus steht, worin Aryl oder Heterozyklus unsubstituiert oder ein- oder zweifach durch R8 oder den Rest -G-M substituiert sind.

2) Ein weiterer Gegenstand der Erfindung ist die Verbindung der Formel I, wobei
   R1 und R2 gleich oder verschieden sind und unabhängig voneinander für Wasserstoffatom oder-(C₁-C₄)-Alkyl stehen, oder
   R1 und R2 bilden zusammen mit dem Kohlenstoffatom an das sie jeweils gebunden sind -(C₃-C₆)-Cycloalkyl,
   R3 und R4 gleich oder verschieden sind und unabhängig voneinander für eine kovalente Bindung, -(CH₂)ₘ-, -(C₁-C₃)-Alkylen-O-(C₀-C₃)-Alkylen-, -(C₀-C₃)-Alkylen-C(O)-O-(CH₂)ₙ-, -(C₀-C₂)-Alkylen-C(O)-NR¹⁰-(CH₂)ₙ-, -(CH₂)ₘ-CH(OH)-(CH₂)ₙ-, -(C₁-C₃)-Alkylen-N(R¹⁰)-(CH₂)ₙ-, -(CH₂)ₘ-NR¹⁰-C(O)-(CH₂)ₙ-, -(CH₂)ₘ-NR¹⁰-C(O)-NR¹⁰-(CH₂)ₙ-, -(CH₂)ₘ-O-C(O)-NR¹⁰-(CH₂)ₙ-, -(CH₂)ₘ-NR¹⁰-C(O)-O-(CH₂)ₙ-, -(CH₂)ₘ-S-(CH₂)ₙ-, -(C₁-C₃)-Alkylen-S(O)-(CH₂)ₙ-, -(C₁-C₃)-Alkylen-SO₂-(CH₂)ₙ-, -(C₁-C₃)-Alkylen-SO₂-NH-(R¹⁰), -(CH₂)ₘ-SO₂-NR¹⁰-(CH₂)ₙ-, -(CH₂)ₘ-NR¹⁰-SO₂-(CH₂)ₙ- oder -(CH₂)ₘ-NR¹⁰-SO₂-NR¹⁰-(CH₂)ₙ- stehen, worin n und m unabhängig voneinander gleich oder verschieden sind und m für die ganzen Zahlen 1, 2, 3, 4, 5 oder 6 steht und n für die ganzen Zahlen Null, 1, 2, 3, 4, 5 oder 6 steht und worin die Alkylenreste die durch -(CH₂)ₘ- oder -(CH₂)ₙ- gebildet werden unsubstituiert oder ein-, zwei- oder dreifach substituiert sind durch Halogen, -NH₂ oder -OH oder ein -(C₃-C₆)-Cycloalkyl bilden, worin Cycloalkyl unsubstituiert oder ein-, zwei- oder dreifach durch Halogen, -NH₂ oder -OH substituiert ist,
      R¹⁰ für Wasserstoffatom, -(C₁-C₆)-Alkyl, -(C₀-C₄)-Alkyl-OH,
      -(C₀-C₄)-Alkyl-O-(C₁-C₄)-Alkyl oder -(C₁-C₃)-Perfluoralkyl steht,
   V₁, V₂ und R5 gleich oder verschieden sind und unabhängig voneinander für
      a) Wasserstoffatom,
      b) -(C₆-C₁₄)-Aryl, worin Aryl ein Rest aus der Reihe Phenyl, Naphthyl, 1-Naphthyl, 2-Naphthyl, Anthryl oder Fluorenyl bedeutet unsubstituiert oder ein- oder zweifach durch R8 oder den Rest -G-M substituiert ist oder c) einen mono- oder bizyklischen 4- bis 15-gliedrigen Heterozyklus stehen, worin Heterozyklus ein Rest aus der Reihe Acridinyl, Azetidinyl, Benzimidazolyl, Benzodioxol, Benzodiazin, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, beta-Carbolinyl, Chinazolinyl, Chinolinyl, 4H-Chinolizinyl, Chinoxalinyl, Chinuclidinyl, Chromanyl, Chromenyl, Decahydrochinolinyl, Dihydrofuran, Dithiazinyl, Dithiazolly, Fuaranyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl, Isothiazolyl, Isoxazolyl, Morpholinyl, Naphthyridinyl, Octahydr-isochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, Pyrrolyl, Tetrahydrofuranyl, Tetrahydroisochinolinyl, Tetrahydrochinolinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Triazinyl, 1,2,3-Triazolyl, 1,2,4-Triazolyl, 1,2,5-Triazolyl, 1,3,4-Triazolyl und Xanthenyl bedeutet, worin der Heterozyklus unsubstituiert oder ein-, zwei- oder dreifach durch R8 oder den Rest -G-M substituiert ist,
   M für
      a) Wasserstoffatom,
      b) -(C₆-C₁₄)-Aryl, worin Aryl wie oben definiert ist und unsubstituiert oder ein- oder zweifach durch R8 substituiert ist oder
      c) einen mono- oder bizyklischen 4- bis 15-gliedrigen Heterozyklus steht, worin Heterozyklus wie oben definiert ist und worin der Heterozyklus unsubstituiert oder ein-, zwei- oder dreifach durch R8 substituiert ist,
   R8 für
      1) Halogen,
      2) -NO₂,
      3) -CN,
      4) -C(O)-NH₂,
      5) -SO₂-NH₂,
      6) -OH,
      7) -NH₂,
      8) -O-CF₃,
      9) -(C₆-C₁₄)-Aryl, worin Aryl unsubstituiert oder ein- oder zweifach durch Halogen oder-O-(C₁-C₈)-Alkyl substituiert ist,
      10) -(C₁-C₈)-Alkyl, worin Alkyl unsubstituiert oder ein-, zwei- oder dreifach durch Halogen, NH₂, -OH oder Methoxy substituiert ist,
      11) -O-(C₁-C₈)-Alkyl, worin Alkyl unsubstituiert oder ein-, zwei- oder dreifach durch Halogen, NH₂, -OH oder Methoxy substituiert ist,
      12) -SO₂-CH₃ oder
      13) -SO₂-CF₃ steht,
   G für kovalente Bindung, -(CH₂)ₒ-, -(C₀-C₃)-Alkylen-O-(C₀-C₃)-Alkylen-, -(CH₂)ₒ-CH(OH)-(CH₂)ₚ-, -(C₀-C₃)-Alkylen-C(O)-O-(CH₂)ₚ-, -(C₀-C₃)-Alkylen-C(O)-NR₁₀-(CH₂)ₚ-, -(C₀-C₃)-Alkylen-N(R¹⁰)-(CH₂)ₚ-, -(CH₂)ₒ-NR¹⁰-C(O)-(CH₂)ₚ-, -(CH₂)₀-O-C(O)-NR¹⁰-(CH₂)ₚ-, -(CH₂)ₒ-NR¹⁰-C(O)-NR¹⁰-(CH₂)ₚ-, -(CH₂)ₒ-NR¹⁰-C(O)-O-(CH₂)ₚ-, -(CH₂)ₒ-S-(CH₂)p-, -(C₀-C₃)-Alkylen-S(O)-(CH₂)ₚ-, -(C₀-C₃)-Alkylen-SO₂-(CH₂)ₚ-, -(C₀-C₃)-Alkylen-SO₂-NH-(R¹⁰), -(CH₂)ₒ-SO₂-NR¹⁰-(CH₂)ₚ-, -(CH₂)ₒ-NR¹⁰-SO₂-(CH₂)ₚ-, -(C₀-C₃)-Alkylen-O-(C₂-C₄)-Alkenylen- oder -(CH₂)ₒ-NR¹⁰-SO₂-NR¹⁰-(CH₂)ₚ- steht, worin
      o und p gleich oder verschieden sind und unabhängig voneinander für die ganzen Zahlen Null, 1, 2, 3, 4, 5 oder 6 stehen und worin die Alkylenreste die durch -(CH₂)ₒ- oder -(CH₂)ₚ- gebildet werden unsubstituiert oder ein-, zwei- oder dreifach substituiert sind durch Halogen, -NH₂ oder -OH oder ein -(C₃-C₆)-Cycloalkyl bilden, worin Cycloalkyl unsubstituiert oder ein-, zwei- oder dreifach durch Halogen, -NH₂ oder -OH substituiert ist, und R10 wie oben definiert ist, und
   Q für kovalente Bindung, -(C₁-C₃)-Alkylen oder -(C₃-C₆)-Cycloalkyl steht,
      unter der Bedingung, dass wenigstens einer der Reste V₁, V₂ oder R5 für -(C₆-C₁₄)-Aryl oder einen mono- oder bizyklischen 4- bis 15-gliedrigen Heterozyklus steht, worin Aryl oder Heterozyklus unsubstituiert oder ein- oder zweifach durch R8 oder den Rest -G-M substituiert sind.
3) Ein weiterer Gegenstand der Erfindung ist die Verbindung der Formel I, wobei
   R1 und R2 gleich oder verschieden sind und unabhängig voneinander für Wasserstoffatom oder-(C₁-C₄)-Alkyl stehen, oder
   R1 und R2 bilden zusammen mit dem Kohlenstoffatom an das sie jeweils gebunden sind -(C₃-C₆)-Cycloalkyl,
   R3 und R4 gleich oder verschieden sind und unabhängig voneinander für eine kovalente Bindung, -(CH₂)ₘ- oder -(C₁-C₃)-Alkylen-O-(C₀-C₃)-Alkylen-, worin
      m für die ganze Zahl 1 steht, und worin der Alkylenrest, der durch -(CH₂)ₘ- gebildet wird unsubstituiert ist oder einfach mit -OH substituiert ist,
   V₂ für Wasserstoffatom steht,
   V₁ und R5 gleich oder verschieden sind und unabhängig voneinander für
      a) Wasserstoffatom,
      b) -(C₆-C₁₄)-Aryl, worin Aryl Phenyl bedeutet und unsubstituiert oder ein- oder zweifach durch R8 oder den Rest -G-M substituiert ist oder
      c) einen mono- oder bizyklischen 4- bis 15-gliedrigen Heterozyklus stehen, worin Heterozyklus ein Rest aus der Reihe Benzodioxol, Chinolinyl oder Pyridyl, bedeutet, worin der Heterozyklus unsubstituiert oder ein-, zwei- oder dreifach durch R8 oder den Rest -G-M substituiert ist,
   M für
      a) Wasserstoffatom,
      b) -(C₆-C₁₄)-Aryl, worin Aryl Phenyl bedeutet und unsubstituiert oder ein- oder zweifach durch R8 substituiert ist oder
      c) einen mono- oder bizyklischen 4- bis 15-gliedrigen Heterozyklus steht, worin Heterozyklus wie oben definiert ist und worin der Heterozyklus unsubstituiert oder ein-, zwei- oder dreifach durch R8 substituiert ist,
   R8 für Halogen, -OH oder -(C₁-C₄)-Alkyl oder -O-(C₁-C₄)-Alkyl steht,
   G für kovalente Bindung, -(C₀-C₃)-Alkylen-O-(C₀-C₃)-alkylen- oder -(C₀-C₃)-Alkylen-O-(C₂-C₄)-Alkenylen- steht, und
   Q für kovalente Bindung oder -(C₁-C₃)-Alkylen steht,
   unter der Bedingung, dass wenigstens einer der Reste V₁ oder R5 für -(C₆-C₁₄)-Aryl oder einen mono- oder bizyklischen 4- bis 15-gliedrigen Heterozyklus steht, worin Aryl oder Heterozyklus unsubstituiert oder ein- oder zweifach durch R8 oder den Rest -G-M substituiert sind.
4) Ein weiterer Gegenstand der Erfindung sind Verbindungen der Formel I aus der Reihe
   2-[3-(4-Benzyloxy-benzyl)-2-oxo-imidazolidin-1-yl]-N-hydroxy-3-methyl-butyramid,

   2-[3-(4-Benzyloxy-phenyl)-2-oxo-imidazolidin-1-yl]-N-hydroxy-3-methyl-butyramid,
   2-[3-(3-Benzyloxy-benzyl)-2-oxo-imidazolidin-1-yl]-N-hydroxy-3-methyl-butyramid,
   N-Hydroxy-3-methyl-2-[2-oxo-3-(4-phenoxy-benzyl)-imidazolidin-1-yl]-butyramid,
   2-[3-(6-Benzyloxy-pyridin-3-ylmethyl)-2-oxo-imidazolidin-1-yl]-N-hydroxy-3-methyl-butyramid,
   2-(3-Biphenyl-4-ylmethyl-2-oxo-imidazolidin-1-yl)-N-hydroxy-3-methyl-butyramid,
   2-[3-Benzyl-5-(4-methoxy-phenyl)-2-oxo-imidazolidin-1-yl]-N-hydroxy-3-methyl-butyramid,
   N-Hydroxy-2-[3-(4-hydroxy-benzyl)-2-oxo-imidazolidin-1-yl]-3-methyl-butyramid,
   N-Hydroxy-2-[3-(3-hydroxy-benzyl)-2-oxo-imidazolidin-1-yl]-3-methyl-butyramid,
   N-Hydroxy-3-methyl-2-{2-oxo-3-[4-(pyridin-4-ylmethoxy)-benzyl]-imidazolidin-1-yl}-butyramid mit TFA,
   N-Hydroxy-3-methyl-2-{2-oxo-3-[4-(pyrid in-3-ylmethoxy)-benzyl]-imidazolidin-1-yl}-butyramid mit TFA,
   N-Hydroxy-3-methyl-2-{2-oxo-3-[4-(pyridin-2-ylmethoxy)-benzyl]-imidazolidin-1-yl}-butyramid mit TFA,
   2-[3-(4-But-2-ynyloxy-benzyl)-2-oxo-imidazolidin-1-yl]-N-hydroxy-3-methyl-butyramid,
   N-Hydroxy-3-methyl-2-{3-[4-(2-methyl-quinolin-4-ylmethoxy)-benzyl]-2-oxo-imidazolidin-1-yl}-butyramid mit TFA,
   2-[3-Benzyl-5-(4-methoxy-benzyl)-2-oxo-imidazolidin-1-yl]-N-hydroxy-3-methyl-butyramid,
   2-[3-(4-Benzyloxy-benzyl)-5-(4-methoxy-benzyl)-2-oxo-imidazolidin-1-yl]-N-hydroxy-3-methyl-butyramid,
   N-Hydroxy-2-[5-(4-methoxy-benzyl)-3-methyl-2-oxo-imidazolidin-1-yl]-3-methyl-butyramid,
   2-[5-Benzo[1,3]dioxol-5-ylmethyl-3-(4-benzyloxy-benzyl)-2-oxo-imidazolidin-1-yl]-N-hydroxy-3-methyl-butyramid oder
   2-(5-Benzo[1,3]dioxol-5-ylmethyl-3-benzyl-2-oxo-imidazolid in-1-yl)-N-hydroxy-3-methyl-butyramid.

Unter dem Begriff "(C₁-C₆)-Alkyl" werden Kohlenwasserstoffreste verstanden, deren Kohlenstoffkette geradkettig oder verzweigt ist und 1 bis 6 Kohlenstoffatome enthält, beispielsweise Methyl, Ethyl, Propyl, Iso-Propyl, Butyl, Iso-Butyl, tertiär-Butyl, Pentyl, Iso-Pentyl, Neopentyl, Hexyl, 2,3-Dimethylbutan oder Neohexyl.

Unter dem Begriff "-(CH₂)ₒ-, worin o die Zahl Null, 1, 2, 3, 4, 5 oder 6 bedeutet" wird für o gleich Null eine kovalente Bindung, o gleich 1 der Rest Methylen, o gleich 2 der Rest Ethylen, o gleich 3 Propylen, o gleich 4 Butenylen, o gleich 5 Pentylen und o gleich 6 Hexylen verstanden. Die Bedeutungen des Begriffs "-(CH₂)ₚ-, worin p die Zahl Null, 1, 2, 3, 4, 5 oder 6 bedeutet" sind analog zum Begriff -(CH₂)ₒ-.

Unter dem Begriff "-(CH₂)ₙ-, worin n die ganze Zahl Null, 1, 2, 3, 4, 5 oder 6 bedeutet" wird für n gleich Null eine kovalente Bindung, n gleich 1 der Rest Methylen, n gleich 2 der Rest Ethylen, n gleich 3 Propylen, n gleich 4 Butenylen, n gleich 5 Pentylen und n gleich 6 Hexylen verstanden. Die Bedeutungen des Begriffs "-(CH₂)ₘ-, worin m die Zahl 1, 2, 3, 4, 5 oder 6 bedeutet" sind analog zum Begriff -(CH₂)ₙ-.

Unter dem Begriff "-(C₀-C₃)-Alkylen" werden Kohlenwasserstoffreste verstanden, deren Kohlenstoffkette geradkettig oder verzweigt ist und 1 bis 3 Kohlenstoffatome enthält wie die Reste Methylen, Ethylen oder Propylen. Unter dem Begriff "-Co-Alkylen-" wird eine kovalente Bindung verstanden.

Unter dem Begriff "-(C₂-C₄)-Alkenylen," werden Kohlenwasserstoffreste verstanden, deren Kohlenstoffkette geradkettig oder verzweigt ist und 2 bis 4 Kohlenstoffatome enthält und je nach Kettenlänge 1 oder 2 Doppelbindungen aufweisen, beispielsweise Ethenylen, Propenylen, Iso-Propenylen, Iso-Butenylen oder Butenylen; die Substituenten an der Doppelbindung können, sofern die prinzipielle Möglichkeit besteht, E- oder Z-ständig angeordnet sein.

Unter dem Begriff "Halogen" wird Fluor, Chlor, Brom oder Jod verstanden.

Unter dem Begriff "-(C₃-C₆)-Cycloalkyl" werden Reste verstanden wie Verbindungen, die sich von 3- bis 6-gliedrige Monocyclen wie Cyclopropyl, Cyclobutyl, Cyclopentyl oder Cyclohexyl herleiten.

Unter dem Begriff "-(C₁-C₃)-Perfluoralkyl" werden teilweise oder vollständig fluoriierte Alkyl-reste verstanden, die sich von den folgenden Resten ableiten wie -CF₃, -CHF₂, -CH₂F, -CHF-CF₃, -CHF-CHF₂, -CHF-CH₂F, -CH₂-CF₃, -CH₂-CHF₂, -CH₂-CH₂F, -CF₂-CF₃, -CF₂-CHF₂, -CF₂-CH₂F, -CH₂-CHF-CF₃, -CH₂-CHF-CHF₂, -CH₂-CHF-CH₂F, -CH₂-CH₂-CF₃, -CH₂-CH₂-CHF₂, -CH₂-CH₂-CH₂F, -CH₂-CF₂-CF₃, -CH₂-CF₂-CHF₂, -CH₂-CF₂-CH₂F, -CHF-CHF-CF₃, -CHF-CHF-CHF₂, -CHF-CHF-CH₂F, -CHF-CH₂-CF₃, -CHF-CH₂-CHF₂, -CHF-CH₂-CH₂F, -CHF-CF₂-CF₃, -CHF-CF₂-CHF₂, -CHF-CF₂-CH₂F, -CF₂-CHF-CF₃, -CF₂-CHF-CHF₂, -CF₂-CHF-CH₂F, -CF₂-CH₂-CF₃, -CF₂-CH₂-CHF₂, -CF₂-CH₂-CH₂F, -CF₂-CF₂-CF₃, -CF₂-CF₂-CHF₂ oder -CF₂-CF₂-CH₂F.

Unter dem Begriff "(C₆-C₁₄)-Aryl" werden aromatische Kohlenstoffreste verstanden mit 6 bis 14 Kohlenstoffatomen im Ring. -(C₆-C₁₄)-Arylreste sind beispielsweise Phenyl, Naphthyl, zum Beispiel 1-Naphthyl, 2-Naphthyl, Biphenylyl, zum Beispiel 2-Biphenylyl, 3-Biphenylyl und 4-Biphenylyl, Anthryl oder Fluorenyl. Biphenylylreste, Naphthylreste und insbesondere Phenylreste sind bevorzugte Arylreste.

Unter dem Begriff "einen mono- oder bizyklischen 4- bis 15-gliedrigen Heterozyklus, der mindestens ein Kohlenstoffatom und ein, zwei, drei oder vier Heteroatome aus der Reihe Stickstoff, Schwefel oder Sauerstoff enthält" oder "Het" werden Reste wie Acridinyl, Azetidinyl, Benzimidazolyl, Benzodioxol, Benzodiazin, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, beta-Carbolinyl, Chinazolinyl, Chinolinyl, 4H-Chinolizinyl, Chinoxalinyl, Chinuclidinyl, Chromanyl, Chromenyl, Decahydrochinolinyl, Dihydrofuran, Dithiazinyl, Dithiazolly, Fuaranyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl, Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purinyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, Pyrrolyl, Tetrahydrofuranyl, Tetrahydroisochinolinyl, Tetrahydrochinolinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Triazinyl, 1,2,3-Triazolyl, 1,2,4-Triazolyl, 1,2,5-Triazolyl, 1,3,4-Triazolyl und Xanthenyl verstanden.

Bevorzugte Heterozyklusreste sind Benzodioxol, Benzofuranyl, Benzothiophenyl, 1,3-Benzodioxolyl, Benzimidazolyl, Benzoxazolyl, Benzothiazolyl, Chinolinyl, Chromanyl, Isochromanyl, Chinazolinyl, Chinoxalinyl, Furyl, Imidazolyl, Indazolyl, Indolyl, Isochinolinyl, Isoindolyl, Isothiazolyl, Isoxazolyl, Oxazolyl, Phthalazinyl, Pteridinyl, Purinyl, Pyrazinyl, Pyrazolyl, Pyridazinyl, Pyridoimidazolyl, Pyridopyridinyl, Pyridopyrimidinyl, Pyridyl, Pyrimidinyl, Pyrrolyl, Tetrazolyl, Thiazolyl und Thienyl. Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze, oder Hydrochloride, Hydrobromide, Sulfate, Hemisulfate, alle möglichen Phosphate sowie Salze der Aminosäuren, natürlicher Basen oder Carbonsäuren.

Die Herstellung physiologisch verträglicher Salze aus zur Salzbildung befähigten Verbindungen der Formel I, einschließlich deren stereoisomeren Formen, gemäß Verfahrensschritt d) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansutfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron- Palmitin-, oder TFA in Frage.

Die Erfindung betrifft ferner ein Verfahren zur Herstellung der Verbindung der Formel I und/oder einer stereoisomeren Form der Verbindung der Formel I und/oder eines physiologisch verträglichen Salzes der Verbindung der Formel I, das dadurch gekennzeichnet ist, dass man
a) eine Verbindung der Formel II 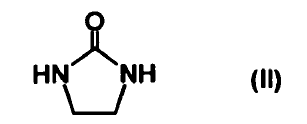 mit einer Verbindung X-Q-R5, worin Q und R5 wie in der Verbindung der Formel I definiert sind und X ein Halogen bedeutet, in eine Verbindung der Formel III überführt, 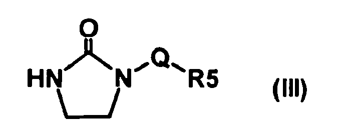 und mit einer Verbindung der Formel IV, 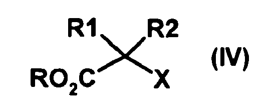 worin R1 und R2 wie in Formel I definiert sind, X ein Halogen und R eine Carboxyl-Schutzgruppe bedeutet,
   in eine Verbindung der Formel V überführt, 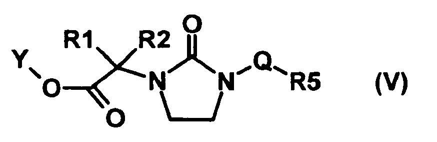 und anschließend die Verbindung der Formel V in die Hydroxamsäure, worin Y = NH-OH ist, der Formel I umwandelt, oder
b) eine Verbindung der Formel VI 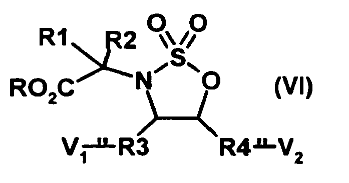 worin R1, R2, R3 und V1 wie in Formel I definiert sind und R eine CarboxySchutzgruppe bedeutet,
   mit einer Verbindung NH₂-Q-R5, worin Q und R5 wie in der Verbindung der Formel I definiert sind, in eine Verbindung der Formel VII überführt, 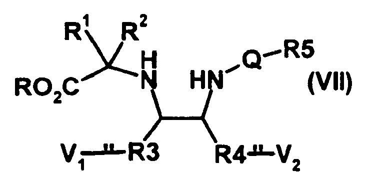 und anschließend mit COCl₂ in eine Verbindung der Formel VIII überführt, 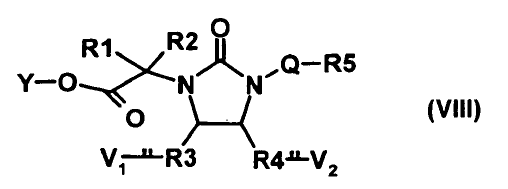 und anschließend die Verbindung der Formel VIII in die Hydroxamsäure, worin Y = NH-OH ist, der Formel I umwandelt, oder
c) eine nach Verfahren a) oder b) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
d) die nach den Verfahren a), b), oder c) hergestellte Verbindung der Formel I entweder in freier Form isoliert oder im Falle des Vorliegens von sauren oder basischen Gruppen in physiologisch verträgliche Salze umwandelt.

Synthesen von Verbindungen der Formel III werden im Stand der Technik beschrieben, beispielsweise für Q = CH₂ und R5 = 2-Chloropyridyl in J. Med. Chem. 1999, 42(12), 2227. Die Umsetzung mit einem Halogenid der Formel X-Q-R5 läuft ab in Gegenwart einer Base wie Kaliumcarbonat, Cäsiumcarbonat, Natrium Hydrid, Lithiumdiisopropylamid oder Lithium-bis(trimethylsilyl)amid.

Verbindungen der Formel V können aus Verbindungen der Formel III durch Deprotonierung mit einer Base wie Lithium-bis(trimethylsilyl)amid, Lithiumdiisopropylamid, Kaliumcarbonat, Cäsiumcarbonat oder Natriumhydrid und Alkylierung mit Verbindungen der Formel IV dargestellt werden, beispielsweise beschrieben in Bioorg. Med. Chem. Lett. 2002, 12(1), 25.

Die Umwandlung von Verbindungen der Formel V in die Hydroxamsäure der Formel I erfolgt durch Entschützung der Carboxylfunktion in geeigneter Weise und Überführung der freien Carbonsäure analog zu den bekannten Methoden wie beschrieben in WO97/18194 oder Tetrahedron Lett. 1992, 33(14), 1827. Geeignete Carboxylschutzgruppen für Verbindungen der Formel IV stellen beispielsweise Ester dar, wie t-Butyl-, Benzyl-, Isopropyl-, Ethyl- oder Methylester. Deren Spaltung sowie weitere geeignete Schutzgruppen für die Carboxylfunktion werden in "Protective Groups in Organic Synthesis" T. W. Greene, P. G. M. Wuts, John Wiley & Sons, Inc., 1999, Seiten 369-431, beschrieben.

Durch Umsetzung mit Aminen des Typs NH₂-Q-R5 in Anwesenheit einer Base wie Cäsiumcarbonat, Kaliumcarbonat, Triethylamin, Diisopropylethylamin lassen sich Verbindungen der Formel VI in Verbindungen der Formel VII überführen. Anstelle einer zusätzlichen Base kann das Amin NH₂-Q-R5 auch im Überschuß (größer zwei Moleäquivalente) eingesetzt werden wie es in Tetrahedron Lett. 1999, 40(43), 7687, beschrieben wird.

Vorschriften für die Überführung von Verbindungen der Formel VII in Verbindungen der Formel VIII sind bekannt. So ist dies beispielsweise möglich, durch die Umsetzung mit Phosgen, Triphosgen oder Carbonyldiimidazol, wie es in J. Med. Chem. 1992, 35(5), 823, beschrieben wird. Für die anschließende Oxidation stehen mehrere literaturbekannte Methoden zur Verfügung wie sie in J. Med. Chem. 1981, 24(11), 1300 oder Tetrahedron Lett. 2001, 42(8), 1433, beschrieben werden.

Verbindungen der Formel VIII können analog zu Verbindungen der Formel V in Hydroxamsäuren der Formel I überführt werden.

Im Verfahrensschritt c) wird die Verbindung der Formel I, sofern sie als Gemisch von Diastereomeren oder Enantiomeren auftritt oder bei der gewählten Synthese als deren Gemische anfällt, in die reinen Stereoisomeren getrennt, entweder durch Chromatographie an einem gegebenenfalls chiralen Trägermaterial, oder, sofern die racemische Verbindung der Formel I zur Salzbildung befähigt ist, durch fraktionierte Kristallisation der mit einer optisch aktiven Base oder Säure als Hilfsstoff gebildeten diastereomeren Salze. Als chirale Stationärphasen für die dünnschicht- oder säulenchromatographische Trennung von

Enantiomeren eignen sich zum Beispiel modifizierte Kieselgelträger (sogenannte Pirkle-Phasen) sowie hochmolekulare Kohlenhydrate wie Triacetylcellulose. Für analytische Zwecke sind nach entsprechender, dem Fachmann bekannter Derivatisierung, auch gaschromatographische Methoden an chiralen Stationärphasen anwendbar. Zur Enantiomerentrennung der racemischen Carbonsäuren werden mit einer optisch aktiven, in der Regel kommerziell erhältlichen Base wie (-)-Nicotin, (+)- und (-)-Phenylethylamin, Chininbasen, L-Lysin oder L-und D-Arginin die unterschiedlich löslichen diastereomeren Salze gebildet, die schwerer lösliche Komponente als Feststoff isoliert, das leichter lösliche Diastereomer aus der Mutterlauge abgeschieden und aus den so gewonnenen diastereomeren Salzen die reinen Enantiomeren gewonnen. Auf prinzipiell gleiche Weise kann man die racemischen Verbindungen der Formel I, die eine basische Gruppe wie eine Aminogruppe enthalten, mit optisch aktiven Säuren, wie (+)-Campher-10-sulfonsäure, D- und L- Weinsäure, D-und L- Milchsäure sowie (+) und (-)-mandelsäure in die reinen Enantiomeren überführen. Auch kann man chirale Verbindungen, die Alkohol- oder Amin-funktionen enthalten, mit entsprechend aktivierten oder gegebenenfalls N-geschützten enantiomerenreinen Aminosäuren in die entsprechenden Ester oder Amide, oder umgekehrt chirale Carbonsäuren mit carboxygeschützten enantiomerenreinen Aminosäuren in die Amide oder mit enantiomerenreinen Hydroxycarbonsäuren wie Milchsäure, in die entsprechenden chiralen Ester überführen. Sodann kann die Chiralität des in enantiomerenreiner Form eingebrachten Aminosäure- oder Alkoholrestes zur Trennung der Isomeren genutzt werden, indem man eine Trennung der nunmehr vorliegenden Diastereomeren durch Kristallisation oder Chromatographie an geeigneten Stationärphasen vornimmt und danach den mitgeführte chiralen Molekülteil mittels geeigneter. Methoden wieder abspaltet.

Weiterhin ergibt sich bei einigen der erfindungsgemäßen Verbindungen die Möglichkeit; zur Herstellung der Gerüststrukturen diastereo- oder enantiomerenrreine Ausgangsprodukte einzusetzen. Dadurch können ggf. auch andere oder vereinfachte Verfahren zur Aufreinigung der Endprodukte eingesetzt werden. Diese Ausgangsprodukte wurden zuvor nach Literaturbekannten Verfahren enantiomeren- oder diastereomerenrein hergestellt. Das kann insbesondere bedeuten, daß in der Synthese der Grundgerüste entweder enantioselektive Verfahren zum Einsatz kommen, oder aber eine Enantiomeren- (oder Diastereomeren-) Trennung auf früher Synthesestufe und nicht erst auf der Stufe der Endprodukte durchgeführt wird. Ebenso kann eine Vereinfachung der Trennungen dadurch erreicht werden, dass zwei- oder mehrstufig vorgegangen wird.

Saure oder basische Produkte der Verbindung der Formel I können in Form ihrer Salze oder in freier Form vorliegen. Bevorzugt sind pharmakologisch verträgliche Salze, beispielsweise Alkali- oder Erdalkalimetallsalze, oder Hydrochloride, Hydrobromide, Sulfate, Hemisulfate, alle möglichen Phosphate sowie Salze der Aminosäuren, natürlicher Basen oder Carbonsäuren. Die Herstellung physiologisch verträglicher Salze aus zur Salzbildung befähigten Verbindungen der Formel I, einschließlich deren stereoisomeren Formen, gemäß Verfahrensschritt d) erfolgt in an sich bekannter Weise. Die Verbindungen der Formel I bilden mit basischen Reagenzien wie Hydroxiden, Carbonaten, Hydrogencarbonaten, Alkoholaten sowie Ammoniak oder organischen Basen, beispielsweise Trimethyl- oder Triethylamin, Ethanolamin, Diethanolamin oder Triethanolamin, Trometamol oder auch basischen Aminosäuren, etwa Lysin, Ornithin oder Arginin, stabile Alkali-, Erdalkali- oder gegebenenfalls substituierte Ammoniumsalze. Sofern die Verbindungen der Formel I basische Gruppen aufweisen, lassen sich mit starken Säuren auch stabile Säureadditionssalze herstellen. Hierfür kommen sowohl anorganische als auch organische Säuren wie Chlorwasserstoff-, Bromwasserstoff-, Schwefel-, Hemischwefel-, Phosphor-, Methansulfon-, Benzolsulfon-, p-Toluolsulfon-, 4-Brombenzol-sulfon-, Cyclohexylamidosulfon-, Trifluormethylsulfon-, 2-Hydroxyethansulfon-, Essig-, Oxal-, Wein-, Bernstein-, Glycerolphosphor-, Milch-, Äpfel-, Adipin-, Citronen-, Fumar-, Malein-, Glucon-, Glucuron- Palmitin-, oder TFA in Frage.

Die Erfindung betrifft auch Arzneimittel, gekennzeichnet durch einen wirksamen Gehalt an mindestens einer Verbindung der Formel I und/oder eines physiologisch verträglichen Salzes der Verbindung der Formel I und/oder eine gegebenenfalls stereoisomere Form der Verbindung der Formel I, zusammen mit einem pharmazeutisch geeigneten und physiologisch verträglichen Trägerstoff, Zusatzstoff und/oder anderen Wirk- und Hilfsstoffen.

Aufgrund der pharmakologischen Eigenschaften eignen sich die erfindungsgemäßen Verbindungen zur selektiven Prophylaxe und Therapie all solcher Erkrankungen, an deren Verlauf eine verstärkte Aktivität der Metalloproteinasen wie Aggrecanase oder TNF-α converting enzyme beteiligt sind. Dazu gehören degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen. Ferner gehören dazu auch Erkrankungen des Bindegewebes wie Kollagenosen, Periodontalerkrankungen, Wundheitungsstörungen und chronische Erkrankungen des Bewegungsapparates wie entzündliche, immunologisch oder stoffwechselbedingte akute und chronische Arthritiden, Arthropathien, Myalgien und Störungen des Knochenstoffwechsels. Ferner eignen sich die Verbindungen der Formel I zur Behandlung der Ulceration, Atherosklerose und Stenosen. Weiterhin eignen sich die Verbindungen der Formel I zur Behandlung von Entzündungen, Krebserkrankungen, Tumormetastasenbildung, Kachexie, Anorexie und septischem Schock.

Unter dem Begriff "Osteoarthrose" wird eine Erkrankung verstanden, die vorwiegend bei einem Missverhältnis zwischen Beanspruchung und Belastbarkeit der einzelnen Gelenkanteile und Gelenkgewebe entsteht, die mit einer zunehmenden Knorpeldestruktion verbunden ist und vorwiegend nicht entzündlich ist. Im Vordergrund der Pathologie stehen Schädigungen des Gelenkknorpels wie Auffaserung, Demarkierung oder Hyalinisierung, gefolgt von reaktiven Veränderungen des subchondralen Knochens sowie Kapselveränderungen.

Unter dem Begriff "Spondylose" wird eine Arthrose der Wirbelkörper verstanden, die durch einen nicht entzündlichen Knorpelschwund der Wirbelkörper und Bandscheiben gekennzeichnet ist.

Die Applikation der erfindungsgemäßen.Arzneimittel kann durch orale, inhalative, rektale oder transdermale Applikation oder durch subkutane, intraartikuläre, intraperitoneale oder intravenöse Injektion erfolgen. Bevorzugt ist die orale Applikation.

Die Erfindung betrifft auch ein Verfahren zur Herstellung eines Arzneimittels, das dadurch gekennzeichnet ist, dass man mindestens eine Verbindung der Formel I mit einem pharmazeutisch geeigneten und physiologisch verträglichen Träger und gegebenenfalls weiteren geeigneten Wirk-, Zusatz- oder Hilfsstoffen in eine geeignete Darreichungsform bringt.

Geeignete feste oder galenische Zubereitungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel wie Trägerstoffe, Spreng-, Binde-, Überzugs-, Quellungs-, Gleit- oder Schmiermittel, Geschmacksstoffe, Süßungsmittel und Lösungsvermittler Verwendung finden. Als häufig verwendete Hilfsstoffe seien Magnesiumcarbonat, Titandioxid, Laktose, Mannit und andere Zucker, Talkum, Milcheiweiß, Gelatine, Stärke, Cellulose und ihre Derivate, tierische und pflanzliche Öle wie Lebertran, Sonnenblumen-, Erdnuß- oder Sesamöl, Polyethylen-glykol und Lösungsmittel wie etwa steriles Wasser und ein- oder mehrwertige Alkohole wie Glycerin, genannt.

Vorzugsweise werden die pharmazeutischen Präparate in Dosierungseinheiten hergestellt und verabreicht, wobei jede Einheit als aktiven Bestandteil eine bestimmte Dosis der erfindungsgemäßen Verbindung der Formel I enthält. Bei festen Dosierungseinheiten wie Tabletten, Kapseln, Dragees oder Suppositorien, kann diese Dosis bis zu etwa 1000 mg, bevorzugt jedoch etwa 50 bis 300 mg und bei Injektionslösungen in Ampullenform bis zu etwa 300 mg, vorzugsweise aber etwa 10 bis 100 mg, betragen.

Für die Behandlung eines erwachsenen, etwa 70 kg schweren Patienten sind je nach Wirksamkeit der Verbindung gemäß Formel I, Tagesdosen von etwa 20 mg bis 1000 mg Wirkstoff, bevorzugt etwa 100 mg bis 500 mg indiziert. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Produkte werden in der Regel durch massenspektroskopische Methoden (FAB-, ESI-MS)-angegeben sind jeweils der Hauptpeak oder die beiden Hauptpeaks und durch ihre Retentionszeit (Rₜ) im LC/MS (die verwendete Methode ist jeweils vermerkt) charakterisiert. Temperaturangaben in Grad Celsius, RT bedeutet Raumtemperatur (22 °C bis 26 °C). Verwendete Abkürzungen sind entweder erläutert oder entsprechen den üblichen Konventionen.

Nachfolgend ist die Erfindung an Hand von Beispielen näher erläutert.

### Beispiele

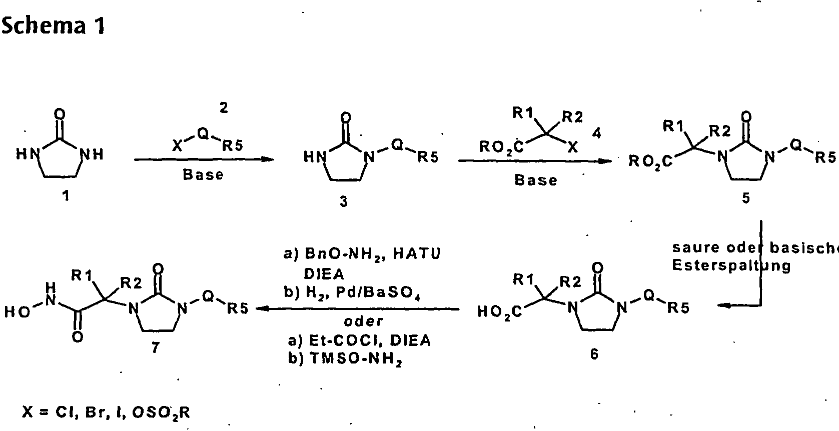

### 1.1 1-(4-Benzyloxy-benzyl)-imidazolidin-2-on (3)

Imidazolidin-2-on (2,0 g; 23,23 mmol) wurde in Dimethylsulfoxid (DMSO; 30 ml) gelöst. Dazu wurde Kaliumcarbonat (3,10 g; 23,23 mmol), Kaliumiodid (0,95 g; 5,80 mmol) und 4-Benzyloxybenzylchlorid (5,4 g; 23,23 mmol) gegeben. Die Mischung wurde 3 Stunden (h) auf 100 °C erhitzt. Nach Abkühlen auf RT wurde zwischen Wasser (100 ml) und Ethylacetat (EtOAc, 100 ml) verteilt. Die Phasen wurden getrennt und die wässrige Phase wurde mit EtOAc (3x; 30 ml) extrahiert. Die vereinten organischen Phasen wurden mit gesättigter NaCl-Lösung (80 ml) gewaschen und über M₉SO₄ getrocknet. Das Lösungsmittel wurde unter vermindertem Druck am Rotationsverdampfer entfernt. Nach Ausrühren des Rückstands aus EtOAc (10 ml) ausgerührt wurde 1-(4-Benzyloxy-benzyl)-imidazolidin-2-on (2,10 g; 7,43 mmol) erhalten. MS: 283,15 (M+H); Rₜ: 1,38 min [Methode: Gradient 0 min 90% H₂O (0,05% TFA) 1,9 min 95% Acetonitril, 95% Acetonitril bis 2,4 min, 10% Acetonitril 2,45 min; Flussrate 1 ml/min; Säule 0,4 µL (YMC J'sphere ODS H80 20X2 1,4 µ); 30 °C]

### 1.2 2-[3-(4-Benzyloxy-benzyl)-2-oxo-imidazolidin-1-yl]-3-methyl-buttersäure-ethylester (5)

1-(4-Benzyloxy-benzyl)-imidazolidin-2-on (1,0 g; 3,54 mmol) wurde in Dimethylformamid (DMF, 10 ml)) gelöst. Es wurde NaH (60 %-ig in Mineralöl; 0,085 g; 3,54 mol) zugegeben und die Mischung wurde 1 h auf 40 °C erhitzt. Danach wurden Ethyl-2-Bromoisovalerat (0,74 g; 3,54 mmol) zugegeben und für weitere 4 h auf 60 °C erhitzt. Die Reaktion wurde durch langsame Zugabe von Wasser (1 ml) beendet. Das Lösungsmittel wurde unter vermindertem Druck entfernt und der Rückstand zwischen Wasser (50 ml) und EtOAc (50 ml) verteilt. Es wurde soviel verdünnte HCl zugegeben, dass der pH-Wert der wässrigen Phase etwa 4 betrug. Dann wurden die Phasen getrennt und die wässrige Phase wurde mit EtOAc (2x; 30 ml) extrahiert. Die vereinigten organischen Phasen wurden über MgSO₄ getrocknet. Das Lösungsmittel wurde unter vermindertem Druck entfernt. Nach Reinigung des Rückstands durch Säulenchromatographie an Silicagel (SiO₂) wurde 2-[3-(4-Benzyloxy-benzyl)-2-oxo-imidazolidin-1-yl]-3-methyl-buttersäure-ethylester (0,38 G; 0,92 mmol) erhalten. MS: 411,15 (M+H); Rₜ: 1,87 min min [Methode: Gradient 0 min 90% H₂O (0,05% TFA) 1,9 min 95% Acetonitril, 95% Acetonitril bis 2,4 min, 10% Acetonitril 2,45 min; Flussrate 1 ml/min; Säule 0,4 µL (YMC J'sphere ODS H80 20X2 1,4 µ); 30 °C]

### 1.3 2-[3-(4-Benzyloxy-benzyl)-2-oxo-imidazolidin-1-yl]-3-methyl-buttersäure (6)

2-[3-(4-Benzyloxy-benzyl)-2-oxo-imidazolidin-1 -yl]-3-methyl-buttersäureethylester _{'}(0,330 g; 0,80 mmol) wurde in Methanol (MeOH; 6 ml) gelöst. Man gab NaOH-Lösung (1 N; 3ml) hinzu und ließ 3 h bei RT rühren. Das Lösungsmittel wurde unter vermindertem Druck entfernt und zum Rückstand wurde gesättigte NaH₂PO₄- Lösung (2 ml) hinzugegeben. Nachdem der Feststoff abgesaugt und unter vermindertem Druck bei 60 °C getrocknet wurde, wurde 2-[3-(4-Benzyloxy-benzyl)-2-oxo-imidazolidin-1-yl]-3-methyl-buttersäure (0,25 g; 0,66 mmol) erhalten. MS: 383,10 (M+H); Rₜ: 1,57 min [Methode: Gradient 0 min 90% H₂O (0,05% TFA) 1,9 min 95% Acetonitril, 95% Acetonitril bis 2,4 min, 10%Acetonitril 2,45 min; Flussrate 1 ml/min; Säule 0,4 µL (YMC J'sphere ODS H80 20X2 1,4 µ); 30 °C]

### 1.4 2-[3-(4-Benzyloxy-benzyl)-2-oxo-imidazolidin-1-yl]-N-hydroxy-3-methyl-butyramid (7)

Es wurde 2-[3-(4-Benzyloxy-benzyl)-2-oxo-imidazolidin-1-yl]-3-methyl-buttersäure (50 mg; 0,13 mmol) in Tetrahydrofuran (THF; 2 ml) gelöst. Nacheinander wurde bei 0 °C

Diisopropylethylamin (DIEA; 69 µl; 0,39 mmol) und Ethylchloroformat (25 µl; 0,26 mmol) zugegeben. Man ließ innerhalb von 2 h von 0 °C auf RT kommen und gab dann O-Trimethylsilylhydroxylamin (50 µl; 0,65 mmol) hinzu. Nach weiteren 3 h Rühren bei RT wurde zwischen verdünnter HCl (10 ml) und EtOAc (10 ml) verteilt. Die Phasen wurden getrennt und die wäßrige Phase wurde mit EtOAc (3x; 5 ml) extrahiert. Die vereinten organischen Phasen wurden über MgSO₄ getrocknet. Das Lösungsmittel wurde unter vermindertem Druck entfernt. Durch Ausrühren des Rückstands aus EtOAc (2 ml) wurde 2-[3-(4-Benzyloxy-benzyl)-2-oxo-imidazolidin-1-yl]-N-hydroxy-3-methyl-butyramide (25 mg; 0,06 mmol) erhalten. MS: 398,15 (M+H); Rₜ: 1,30 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

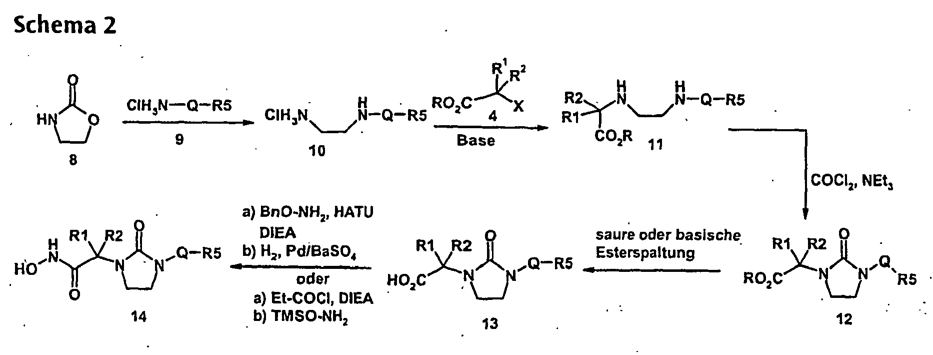

### 2.1 N-1-(4-Benzyloxy-phenyl)-ethane-1,2-diamin Hydrochlorid (10)

4-Benzyloxyanilin Hydrochlorid (10,0 g; 42,42 mmol) wurde in Diethylenglykolmonomethylether (40 ml) gelöst. Dazu wurde 2-Oxazolidon (4,43 g; 50,90 mmol) gegeben und 6 h auf 180 °C erhitzt. Nach Abkühlen auf RT wurde der Feststoff abgesaugt. Nachdem mit Diethylether (Et₂O) gewaschen wurde, wurde N-1-(4-Benzyloxyphenyl)-ethane-1,2-diamin Hydrochlorid (3,50 g; 12,55 mmol) erhalten. MS: 243,15 (M_{freie Base}+H); Rₜ: 0.96 min [Methode: Gradient 0 min 90% H₂O (0,05% TFA, 1,9 min 95% Acetonitril, 95% Acetonitril bis 2,4 min, 10% Acetonitril 2,45 min; Flussrate 1 ml/min; Säule 0,4 µL (YMC j'sphere ODS H80 20X21,4 µ); 30 °C]

### 2.2 2-[2-(4-Benzyloxy-phenylamino)-ethylamino]-3-methyl-buttersäure-ethylester (11)

N-1-(4-Benzyloxy-phenyl)-ethan-1,2-diamin Hydrochlorid (1.7 g; 6,09 mmol) wurde in DMF (15 ml) gelöst. Man erhitzte auf 50 °C und gab Triethylamin (NEt₃; 2,55 ml) hinzu. Danach wurde Ethyl-2-Bromoisovalerat (1,66 g; 7,92 mmol) hinzugegeben und 3 h auf 100 °C erhitzt. Das Lösungsmittel wurde unter vermindertem Druck entfernt. Nach Reinigung des Rückstands mittels Säulenchromatographie (SiO₂) erhielt man 2-[2-(4-Benzyloxy-phenylamino)-ethylamino]-3-methyl-buttersäure-ethylester (0,30 g; 0,809 mmol). MS: 371,20 (M+H); Rt: 1,29 min [Methode: Gradient 0 min 90% H₂O (0,05% TFA) 1,9 min 95% Acetonitril, 95% Acetonitril bis 2,4 min, 10% Acetonitril 2,45 min; Säule 0,4 µL (YMC J'sphere ODS H80 20X2 1,4 µ); 30 °C]

### 2.3 2-[3-(4-Benzyloxy-phenyl)-2-oxo-imidazolidin-1-yl]-3-methyl-buttersäure-ethylester (12)

2-[2-(4-Benzyloxy-phenylamino)-ethylamino]-3-methyl-buttersäure-ethylester (100 mg; 0,27 mmol) wurde in Toluol (5 ml) gelöst und auf 0 °C gekühlt. Man gab DIEA (141 µl; 0,81 mmol) und Phosgen (20 %-ig in Toluol; 202 µl; 0,40 mmol) hinzu. Innerhalb von 3 h ließ man auf RT kommen und entfernte dann das Lösungsmittel wurde unter vermindertem Druck entfernt. Der Rückstand wurde zwischen Wasser (10 ml) und EtOAc (10 ml) verteilt. Die Phasen wurden getrennt und die wässrige Phase wurde mit EtOAc (2x; 5 ml) extrahiert. Die vereinten organischen Phasen wurden über MgSO₄ getrocknet. Nach Entfernen des Lösungsmittels wurde unter vermindertem Druck erhielt man 2-[3-(4-Benzyloxy-phenyl)-2-oxo-imidazolidin-1-yl]-3-methyl-buttersäure-ethylester (90 mg; 0,22 mmol). MS: 397,15 (M+H); Rₜ: 1,90 min [Methode: Gradient 0 min 90% H₂O (0,05% TFA) 1,9 min 95% Acetonitril, 95% Acetonitril bis 2,4 min, 10% Acetonitril 2,45 min; Flussrate 1 ml/min; Säule 0,4 µL (YMC j'sphere ODS H80 20X2 1,4 µ); 30 °C]

### 2.4 2-[3-(4-Benzyloxy-phenyl)-2-oxo-imidazolidin-1-yl]-3-methyl-buttersäure (13)

2-[3-(4-Benzyloxy-phenyl)-2-oxo-imidazolidin-1-yl]-3-methyl-buttersäure-ethylester (110 mg; 0,27 mmol) wurde bei 0°C in Methanol (3 ml) gelöst. Dann wurde Natronlauge (1 N, 1,5 ml) zugegeben und 4h gerührt, wobei es der Reaktionsmischung erlaubt wurde, langsam auf RT zu kommen. Das Methanol wurde am Rotationsverdampfer entfernt und die restliche Lösung mit gesättigter NaH₂PO₄-Lösung neutralisiert. Die ausfallende 2-[3-(4-Benzyloxy-phenyl)-2-oxo-imidazolidin-1-yl]-3-methyl-buttersäure (91 mg, 0,25 mmol) wurde abgesaugt. MS: 369,20 (M+H); Rₜ: 1,58 min [Methode: Gradient 0 min 90% H₂O (0,05% TFA) 1,9 min 95% Acetonitril, 95% Acetonitril bis 2,4 min, 10% Acetonitril 2,45 min; Flussrate 1 ml/min; Säule 0,4 µL (YMC J'sphere ODS H80 20X2 1,4 µ); 30 °C]

### 2.5 2-[3-(4-Benzyloxy-phenyl)-2-oxo-imidazolidin-1-yl]-N-hydroxy-3-methyl-butyramid (14)

Es wurde 2-[3-(4-Benzyloxy-phenyl)-2-oxo-imidazolidin-1-yl]-3-methyl-buttersäure (66 mg; 0,18 mmol) in THF (2 ml) gelöst. Nacheinander wurde bei 0 °C Diisopropylethylamin (DIEA; 124 µl; 0,71 mmol) und Ethylchloroformat (51 µl; 0,53 mmol) zugegeben. Man ließ innerhalb von 2 h von 0 °C auf RT kommen und gab dann O-Trimethylsilylhydroxylamin (80,43 µl; 1,07 mmol) hinzu. Nach weiteren 3 h Rühren bei RT wurde zwischen verdünnter HCl (10 ml) und EtOAc (10 ml) verteilt. Die Phasen wurden getrennt und die wässrige Phase wurde mit EtOAc (3x; 5 ml) extrahiert. Die vereinten organischen Phasen wurden über MgSO₄ getrocknet. Das Lösungsmittel wurde unter vermindertem Druck entfernt. Durch Ausrühren des Rückstands aus EtOAc (2 ml) wurde 2-[3-(4-Benzyloxy-phenyl)-Z-oxo-imidazolidin-1-yl]-N-hydroxy-3-methyl-butyramid (22 mg; 0,06 mmol) erhalten. MS: 384,25 (M+H); Rₜ: 1,94 min [Methode: Gradient Acetonitril + 0,08% Ameisensäure : H₂O + 0,1% Ameisensäure von 5:95 (0 min) bis 95:5 (2,5 min) bis 95:5 (3 min); Flussrate 1,3 ml/min; Säule YMC Jsphere 33*2.1]

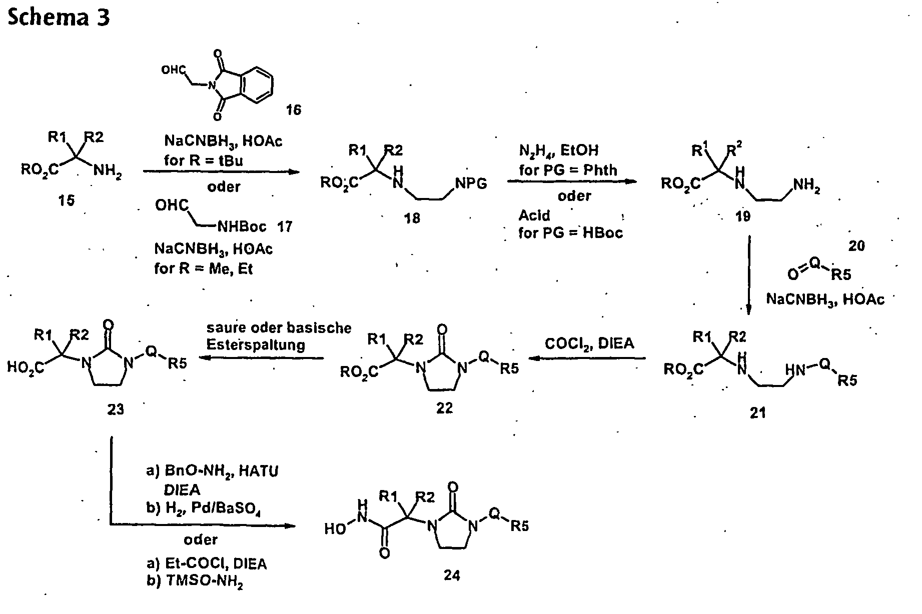

### 3.1 2-[2-(1,3-Dioxo-1,3-dihydro-isoindol-2-yl)-ethylamin]-3-methyl-buttersäure-t-butylester (18)

(R)-Valin-t-Butylester (4,9 g; 28,28 mmol) wurde in Methanol (100 ml) gelöst. Dazu wurde Essigsäure (1,62 ml; 28,28 mmol) und (1,3-Dioxo-1,3-dihydro-isoindol-2-yl)-acetaldehyd (5,40 g; 28,28 mmol) gegeben. Danach wurde Natriumcyanoborhydrid (1,95 g; 31,11 mmol) gelöst in THF (20 ml) zugegeben. Man ließ 6 h bei RT rühren und gab dann gesättigte NaHCO₃-Lösung (200 ml) zu. Methanol wurde unter vermindertem Druck entfernt und der Rückstand wurde mit EtOAc (3x; 100 ml) extrahiert. Die vereinten organischen Phasen wurden über MgSO₄ getrocknet. Nach Entfernen des Lösungsmittels unter vermindertem Druck und Reinigung des Rückstands mittels Säulenchromatographie (SiO') wurde (R)-2-[2-(1,3-Dioxo-1,3-dihydroisoindol-2-yl)-ethylamino]-3-methyl-buttersäure-t-butylester (6,60 g; 19,05 mmol) erhalten. MS: 347,20 (M+H); Rₜ: 1,02 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### 3.2 2-(2-Amino-ethylamino)-3-methyl-buttersäure-t-butylester (19)

(R)-2-[2-(1,3-Dioxo-1,3-dihydro-isoindol-2-yl)-ethylamino]-3-methyl-buttersäure-t-butylester (6,60 g; 19,05 mmol) wurde in Ethanol (100 ml) gelöst. Hydrazin Hydrat (4,62 ml; 95,25 mmol) wurde zugegeben und die Reaktionsmischung 2 h unter Rückfluss erhitzt. Der Feststoff wurde über Kieselgur abgesaugt und mit Ethanol (100 ml) gewaschen. Das Lösungsmittel wurde entfernt und der Rückstand zwischen gesättigter NaHCO₃-Lösung (150 ml) und CH₂Cl₂ (150 ml) verteilt. Die Phasen wurden getrennt und die wässrige Phase wurde mit CH₂Cl₂ (2x; 100 ml) extrahiert. Die vereinten organischen Phasen wurden über MgSO₄ getrocknet. Nach Entfernen des Lösungsmittels unter vermindertem Druck wurde (R)-2-(2-Amino-ethylamino)-3-methyl-buttersäure-t-butylester (4,60 g; 19,05 mmol) erhalten. MS: 217,25 (M+H); Rₜ: 0,69 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### 3.3 2-[2-(3-Benzyloxy-benzylamino)-ethylamin]-3-methyl-buttersäure-t-butylester (21)

(R)-2-(2-Amino-ethylamino)-3-methyl-buttersäure-t-butylester (100 mg; 0,46 mmol) wurde in Methanol (2 ml) gelöst. Man gab Essigsäure (8 µl; 0,14 mmol), 3-Benzyloxybenzaldehyd (98 mg; 0,46 mmol) und Natriumcyanoborhydrid (32 mg; 0,50 mmol) zu. Nach 6 h Rühren bei RT wurde zwischen gesättigter NaHCO₃-Lösung (8 ml) und CH₂Cl₂ (8 ml) verteilt. Die Phasen wurden getrennt und die wässrige Phase wurde mit CH₂Cl₂ (2x; 5 ml) extrahiert. Die vereinten organischen Phasen wurden über MgSO₄ getrocknet und das Lösungsmittel wurde unter vermindertem Druck entfernt. Reinigung mit präparativer HPLC ergab (R)-2-[2-(3-Benzyloxybenzylamino)-ethylamin]-3-methyl-buttersäure-t-butylester (155 mg; 0,37 mmol) MS: 413,25 (M+H); Rₜ: 1,11 min [Methode: Gradient 0 min 96% H₂O (0,05% TFA) 2,0 min 95% Acetonitril, 95% Acetonitril bis 2,4 min, 4% Acetonitril 2,45 min; Säule 0,4 µL (YMC J'sphere ODS H80 20X2 1,4 µ); 30 °C]

### 3.4 2-[3-(3-Benzyloxy-benzyl)-2-oxo-imidazolidin-1-yl]-3-methyl-buttersäure-t-butylester (22)

(R)-2-[2-(3-Benzyloxy-benzylamino)-ethylamino]-3-methyl-buttersäure-t-butylester (155 mg; 0,37 mmol) wurde in Toluol (5 ml) gelöst und auf 0 °C gekühlt. Man gab NaOH (1N; 5 ml; 5 mmol) hinzu, sowie Phosgen (20% in Toluol; 0,37 ml; 0,75 mmol). Man ließ 6 h bei 0 °C rühren, trennte die Phasen und extrahierte die wäßrige Phase mit CH₂Cl₂ (2x; 5 ml). Die vereinten organischen Phasen wurden über MgSO₄ getrocknet und die Lösungsmittel wurden unter vermindertem Druck entfernt. Nach Reinigung des Rückstands mittels Säulenchromatographie (SiO₂) wurden (R)-2-[3-(3-Benzyloxy-benzyl)-2-oxo-imidazolidin-1-yl]-3-methyl-buttersäure-t-butylester (102 mg; 0,23 mmol) erhalten. MS: 439,25 (M+H); Rₜ: 2,12 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### 3.5 2-[3-(3-Benzyloxy-benzyl)-2-oxo-imidazolidin-1-yl]-N-hydroxy-3-methyl-butyramid (24)

(R)-2-[3-(3-Benzyloxy-benzyl)-2-oxo-imidazolidin-1-yl]-3-methyl-buttersäure-t-butylester (102 mg; 0,23 mmol) wurde in CH₂Cl₂ (2 ml) gelöst und auf 0 °C gekühlt. Man gab Trifluoressigsäure (TFA; 1 ml) hinzu und ließ 2 h rühren. Die Lösungsmittel wurden unter vermindertem Druck entfernt und der Rückstand in Tetrahydrofuran (THF; 2 ml) aufgenommen. Es wurde DIEA (0,16 ml; 0,93 mmol) und Ethylchlorformat (67 µl; 0,69 mmol) hinzugegeben. Man ließ 2 h bei RT rühren und gab dann O-Trimethylsilylhydroxylamin (0,10 ml; 1,39 mmol) hinzu. Man ließ 15 h rühren, gab HCl (6N; 0,30 ml) hinzu und entfernte dann die Lösungsmittel unter vermindertem Druck. Nach Reinigung des Rückstands durch präparative HPLC wurde (R)-2-[3-(3-Benzyloxybenzyl)-2-oxo-imidazolidin-1-yl]-N-hydroxy-3-methyl-butyramid (8 mg, 0,02 mmol) erhalten. MS: 398,15 (M+H); Rₜ: 1,46 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X21,4 µ); 30°C]

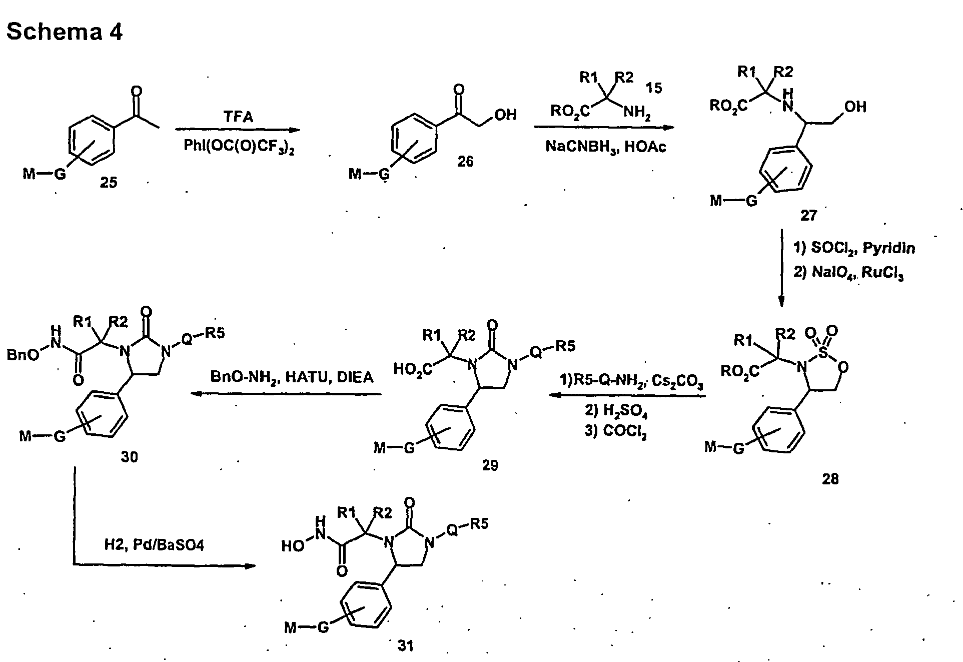

### 4.1 2-Hydroxy-1-(4-methoxy-phenyl)-ethanon (26)

4-Methoxyacetophenon (10 g; 66,59 mmol) wurde zu einer Lösung aus Acetonitril (350 ml), Wasser (70 ml) und Trifluoressigsäure (TFA) (10,26 ml; 133,20 mmol) gegeben. Dann wurde [Bis(trifluoroacetoxy)iodo]benzol zugegeben und die Reaktionsmischung 3 h unter Rückfluss erhitzt. Danach wurde das Acetonitril am Rotationsverdampfer abgezogen und die Reaktionsmischung in NaHCO₃-Lösung/CH₂Cl₂ verteilt. Es wurde mit CH₂Cl₂ (2x) extrahiert, die vereinigten organischen Phasen über MgSO₄ getrocknet und das Lösemittel am Rotationsverdampfer entfernt. Nach Reinigung mittels Säulenchromatographie (CH₂Cl₂/ MeOH 50:1) wurde 2-Hydroxy-1-(4-methoxy-phenyl)-ethanon (6,0 g; 36,1 mmol) erhalten. MS: 167,15 (M+H); Rₜ: 0,83 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X21,4 µ); 30 °C]

### 4.2 2-[2-Hydroxy-1-(4-methoxy-phenyl)-ethylamino]-3-methyl-buttersäure-t-butylester (27)

Valin-t-butylester Hydrochlorid (3,85 g; 18,38 mmol) wurde in 1 N NaOH/CH₂Cl₂ verteilt, mit CH₂Cl₂ extrahiert (2x) und die vereinigten, organischen Phasen über MgSO₄ getrocknet. Nach dem Einengen am Rotationsverdampfer wurde das so erhaltene freie Amin in 1,2-Dichlorethan (30 ml) aufgenommen. Dann wurde 2-Hydroxy-1-(4-methoxy-phenyl)-ethanon (2,35 g; 14,14 mmol) und Essigsäure (0,48 ml; 8,48 mmol) zugegeben. Es wurde 1 h bei RT gerührt, dann NaBH(OAc)₃ (3,89 g; 18,38 mmol) zugegeben und weitere 4 h bei RT gerührt. Die Reaktionsmischung wurde in NaHCO₃-Lsg./CH₂Cl₂ verteilt und mit CH₂Cl₂ (2x) extrahiert. Die vereinigten organischen Phasen wurden über MgSO₄ getrocknet und das Lösemittel am Rotationsverdampfer entfernt. Nach chromatographischer Reinigung wurde 2-[2-Hydroxy-1-(4-methoxy-phenyl)-ethylamino]-3-methyl-buttersäure-t-butylester (2,5 g; 7,73 mmol) erhalten. MS: 324,20 (M+H); Rₜ: 1,07 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### 4.3 2-[4-(4-Methoxy-phenyl)-2,2-dioxo-[1,2,3]oxathiazolidin-3-yl]-3-methyl-buttersäure-t-butylester (28)

2-[2-Hydroxy-1-(4-methoxy-phenyl)-ethylamino]-3-methyl-buttersäure-t-butylester (2,5 g; 7,73 mmol) wurde in CH₂Cl₂ (200 ml) vorgelegt und die Lösung auf-78 °C gekühlt. Nach Zugabe von Pyridin (3,12 ml; 38,65 mmol) wurde langsam Thionylchlorid (0,67 ml; 9,27 mmol) zugetropft. Die Reaktionsmischung wurde 1 h gerührt, wobei man die Temperatur auf 0 °C steigen ließ. Die Reaktionsmischung wurde zwischen wässriger 1%-iger HCl/CH₂Cl₂ verteilt und mit CH₂Cl₂ (2x) extrahiert. Die vereinigten organischen Phasen wurden mit gesättigter NaHCO₃-Lsg. gewaschen, über MgSO₄ getrocknet und das Lösemittel am Rotationsverdampfer entfernt. Der Rückstand wurde in Acetonitril (20 ml) aufgenommen und auf 0 °C gekühlt. Dann wurde NalO₄ (1,82 g; 8,5 mmol), RuCl3·H₂O (14,43 mg; 0,077 mmol) und Wasser (20 ml) zugegeben. Die Reaktionsmischung wurde 5 Minuten bei 0 °C gerührt und dann weitere 20 Minuten bei RT. Dann wurde zwischen gesättigter NaHCO₃-Lsg. /CH₂Cl₂ verteilt und mit CH₂Cl₂ (2x) extrahiert. Die vereinigten organischen Phasen wurden mit gesättigter NaHCO₃-Lösung gewaschen, über MgSO₄ getrocknet und das Lösemittel am Rotationsverdampfer entfernt. Nach chromatographischer Reinigung wurde 2-[4-(4-Methoxy-phenyl)-2,2-dioxo-[1,2,3]oxathiazolidin-3-yl]-3-methyl-buttersäure-t-butylester (2,5 g; 6,48 mmol) erhalten. MS: 403,15 (M+NH₄⁺);_{:}Rₜ: 1,82 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 p); 30 °C]

### 4.4 2-[3-Benzyl-5-(4-methoxy-phenyl)-2-oxo-imidazolidin-1-yl]-3-methyl-buttersäure (29)

2-[4-(4-Methoxy-phenyl)-2,2-dioxo-[1,2,3]oxathiazolidin-3-yl]-3-methyl-buttersäure-t-butylester (0,30 g; 0,778 mmol) wurde in Acetonitril (5 ml) vorgelegt. Dazu wurde Benzylamin (0,10 g; 0,93 mmol) und Cs₂CO₃ (0,50 g; 1,55 mmol) gegeben und die Reaktionsmischung wurde 5 h bei 55°C gerührt. Dann wurde über Kieselgur filtriert und der Rückstand mit einer Lösung von Methanol (3%) in Acetonitril gewaschen. Das Filtrat wurde am Rotationsverdampfer eingeengt und der Rückstand in Dioxan (10 ml) und konzentrierter, wässriger H₂SO₄ (10 ml) aufgenommen. Es wurde 2 h bei 70°C gerührt und dann am Rotationsverdampfer zur Trockene eingeengt. Der Rückstand wurde in Toluol aufgenommen und mit 1 N NaOH (10 ml) versetzt. Nachdem die Reaktionsmischung auf 0 °C gekühlt wurde, wurde Phosgen (20% in Toluol; 0,58 ml; 1,16 mmol) langsam zugetropft. Dann wurde 2 h bei 0 °C gerührt. Die Reaktionsmischung wurde mit verdünnter, wässriger HCl auf pH 1-2 eingestellt, mit EtOAc versetzt und mit EtOAc (2x) extrahiert. Die vereinigten organischen Phasen wurden über MgSO₄ getrocknet und das Lösemittel am Rotationsverdampfer entfernt. Nach Reinigung mittels Säulenchromatographie (CH₂Cl/ MeoH, Gradient) wurde 2-[3-Benzyl-5-(4-methoxy-phenyl)-2-oxo-imidazolidin-1-yl]-3-methyl-buttersäure (0,15 g; 0,39 mmol) erhalten. MS: 383,15 (M+H); Rt: 1,52 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µ (YMC J'sphere ODS H80 20X2 1,4 µ; 30 °C]

### 4.5 2-[3-Benzyl-5-(4-methoxy-phenyl)-2-oxo-imidazolidin-1-yl]-benzyloxy-3-methyl butyramid (30)

2-[3-Benzyl-5-(4-methoxy-phenyl)-2-oxo-imidazolidin-1-y]-3-methyl-buttersäure (150 mg; 0,39 mmol) wurde in DMF (10 ml) vorgelegt und auf 0 °C gekühlt. Dann wurde N,N-Disiopropylethylamin (202 mg; 1,56 mmol), 0-Benzylhydroxylaminhydrochlorid (125 mg, 0,78 mmol) und 0-(7-Azabenzotriazol-1-yl)-N,N,N',N'-tetramethyluronium-hexafluorophosphat (HATU; 223 mg; 0,58 mmol) zugegeben und 2 h bei 0 °C gerührt. Danach wurde die Reaktionslösung eingeengt und zwischen verdünnter HCl-Lsg./EtOAc verteilt und mit EtOAc (2x) extrahiert. Die vereinigten organischen Phasen wurden über MgSO₄ getrocknet und das Lösemittel am Rotationsverdampfer entfernt. Nach chromatographischer Reinigung wurde 2-[3-Benzyl-5-(4-methoxy-phenyl)-2-oxo-imidazolidin-1-yl]-N-benzyloxy-3-methyl-butyramid (116 mg; 0,24 mmol) erhalten. MS: 488,25 (M+H); Rₜ: 3,50 min [Methode: Gradient 95% H₂O (0,05% TFA) bis 95% Acetonitril über 3,5 min, 95% Acetonitril für 1,0 min, 5% Acetonitril 1,0 min; Flussrate 0,5 ml/min; Säule 1 µL (Merck Purospher 5 µ 2x55 mm); 30 °C]

### 4.6 2-[3-Benzyl-5-(4-methoxy-phenyl)-2-oxo-imidazolidin-1-yl]-N-hydroxy-3-methyl-butyramid (31)

2-[3-Benzyl-5-(4-methoxy-phenyl)-2-oxo-imidazolidin-1-yl]-N-benzyloxy-3-methyl-butyramid (120 mg; 0,24 mmol) wurde in Methanol (10 ml) vorgelegt und mit Pd/BaSO₄ (50 mg) versetzt. Es wurde 4 h bei RT mit H₂ (1 atm) hydriert. Dann wurde die Reaktionsmischung über Kieselgur filtriert, der Rückstand mit Methanol gewaschen und das Filtrat am Rotationsverdampfer eingeengt. Nach Reinigung über präperative HPLC wurde 2-[3-Benzyl-5-(4-methoxy-phenyl)-2-oxo-imidazolidin-1-yl]-N-hydroxy-3-methyl-butyramid (60 mg, 0,15 mmol) erhalten. MS: 398,15 (M+H); Rₜ: 1,31 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

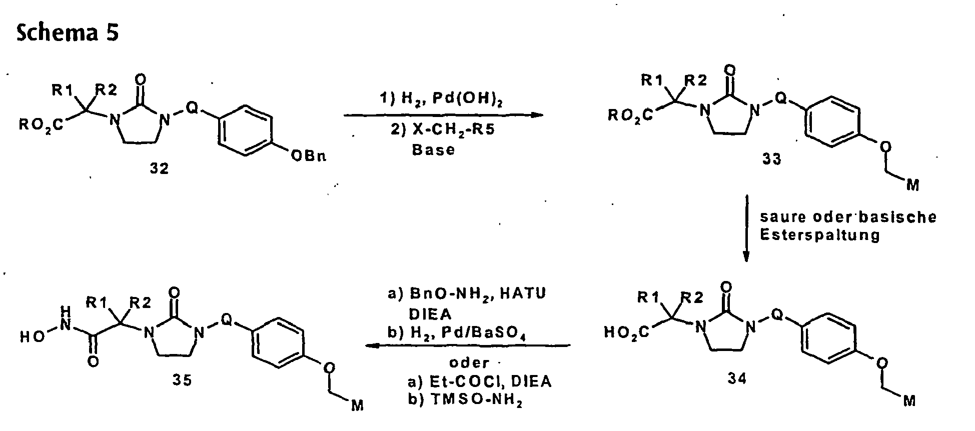

### 5.1 2-[3-(4-Hydroxy-benzyl)-2-oxo-imidazolidin-1-yl]-3-methyl-buttersäure-ethylester (32)

2-[3-(4-Benzyloxy-benzyl)-2-oxo-imidazolidin-1-yl]-3-methyl-butytersäure-ethylester (400 mg; 0,97 mmol) wurde in Ethanol (20 ml) gelöst. Man gab Pd(OH)₂ (100 mg; 0,71 mmol) hinzu und hydrierte für 3 h bei etwa 1 atm. Wasserstoff. Der Katalysator wurde über Kieselgur abfiltriert und mit Ethanol (2x; 20 ml) gewaschen. Das Lösungsmittel wurde unter vermindertem Druck entfernt. Nach Reinigung des Rückstands mittels Säulenchromatographie (SiO₂) erhielt man 2-[3-(4-Hydroxy-benzyl)-2-oxo-imidazolidin-1-yl]-3-methyl-buttersäure-ethylester (300 mg; 0,94 mol). MS: 321,35 (M+H); Rt: 1,24 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### 5.2 3-Methyl-2-{2-oxo-3-[4-(pyridin-4-ylmethoxy)-benzyl]-imidazolidin-1-yl}-buttersäure-ethylester (33)

2-[3-(4-Hydroxy-benzyl)-2-oxo-imidazolidin-1-yl]-3-methyl-buttersäure-ethylester (50 mg; 0,15 mmol) wurde in DMSO (1 ml) gelöst. Dazu wurde nacheinander 4-Chlormethylpyridin Hydrochlorid (39 mg; 0,23 mmol), Kaliumcarbonat (86 mg; 0,62 mmol) und Kaliumiodid (8 mg, 04 mmol) gegeben. Man erhitzte 3 h auf 50 °C. Nach Abkühlen auf RT wurde zwischen Wasser (10 ml) und EtOAc (10 ml) verteilt. Die Phasen wurden getrennt und die wässrige Phase wurde mit EtOAc (3x; 5ml) extrahiert. Die vereinten organischen Phasen wurden über MgSO₄ getrocknet und das Lösungsmittel wurde unter vermindertem Druck entfernt. Nach Reinigung des Rückstands mittels Säulenchromatographie (SiO₂) wurde 3-Methyl-2-{2-oxo-3-(4-(pyridin-4-ylmethoxy)-benzyl)-imidazolidin-1-yl}-buttersäure-ethylester (45 mg; 0,11 mmol) erhalten. MS: 412,50 (M+H); Rₜ: 1,04 min [Methode: Gradient 0 min 96% H₂O (0,05% TFA) 2,0 min 95% Acetonitril, 95% Acetonitril bis 2,4 min, 4%Acetonitril 2,45 min; Flussrate 1 ml/min; Säule 0,4 µL (YMC J'sphere ODS H80 20X21,4 µ); 30 °C]

### 5.3 3-Methyl-2-{2-oxo- 3-[4-(pyridin-4-ylmethoxy)-benzyl]-imidazolidin-1-yl}-buttersäure (34)

3-Methyl-2-{2-oxo-3-[4-(pyridin-4-ylmethoxy)-benzyl]-imidazolidin-1-yl}-buttersäure-ethylester (45 mg; 0,11 mmol) wurde in Methanol (1,5 ml) gelöst und auf 0°C gekühlt. Man gab NaOH (1N, 5 ml) hinzu und ließ innerhalb von 3 h auf RT erwärmen. Dann wurde das Methanol unter vermindertem Druck entfernt und zu dem Rückstand Wasser (1,5 ml) und gesättigte NaH₂PO₄-Lösung (3 ml) gegeben. Der Feststoff wurde abgesaugt und mit Wasser gewaschen. Nach Trocknung bei 60 °C unter vermindertem Druck erhielt man 3-Methyl-2-{2-oxo-3-[4-(pyridin-4-ylmethoxy)-benzyl]-imidazolidin-1-yl}-buttersäure (36 mg; 0,09 mmol). MS: 384,45 (M+H); Rₜ: 0,87 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X21,4 µ); 30 °C]

### 5.4 N-Hydroxy-3-methyl-2-{2-oxo-3-[4-(pyridin-4-ylmethoxy)-benzyl]-imidazolidin-1-yl}-butyramid; Verbindung mit TFA (35)

3-Methyl-2-{2-oxo-3-[4-(pyridin-4-ylmethoxy)-benzyl]-imidazolidin-1-yl}-buttersäure (36 mg; 0,09 mmol) wurden in THF (5 ml) gelöst und auf 0°C gekühlt. Man gab dazu nacheinander DIEA (66 µl; 0,37 mmol) und Ethylchloroformat (27 µl; 0,28 mmol). Innerhalb von 3 h ließ man auf RT kommen. Dann wurde O-(Trimethylsilyl)hydroxylamin (42 µl; 0,56 mmol) zugegeben und weitere 15 h bei RT gerührt. Danach entfernte man das Lösungsmittel unter vermindertem Druck. Der Rückstand wurde in verdünnter HCl (2 ml) aufgenommen und 10 min gerührt. Man neutralisierte mit verdünnter NaOH und extrahierte mit CHCl₃/i-Propanol (4:1; 3x; 10 ml). Die vereinten organischen Phasen wurden über MgSO₄ getrocknet und die Lösungsmittel unter vermindertem Druck entfernt. Nach Reinigung des Rückstands durch präparative HPLC gereinigt wurde N-Hydroxy-3-methyl-2-{2-oxo-3-[4-(pyridin-4-ylmethoxy)-benzyl]-imidazolidin-1-yl}-butyramid als Trifluoracetat (9 mg; 0,02 mmol) erhalten. MS: 399,40 (M+H); Rₜ: 0,78 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X21,4 µ); 30 °C]

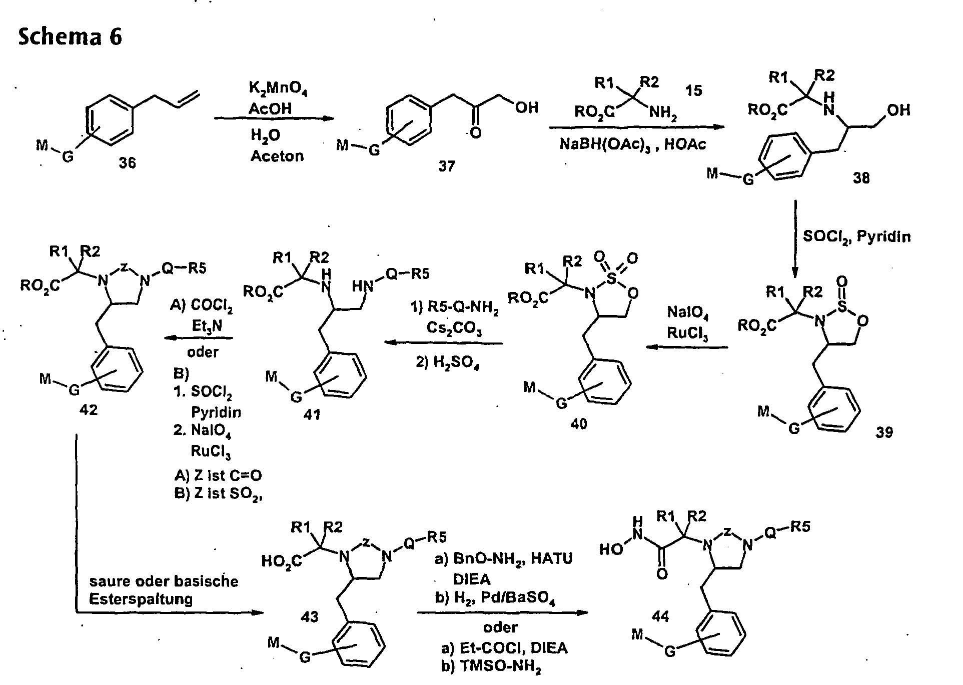

### 6.1 1-Hydroxy-3-(4-methoxy-phenyl)-propan-2-on (37)

4-Allylanisol (98%; 3,0 g; 19,86 mmol) wurde in Eisessig (180 ml), Wasser (180 ml) und Aceton (180 ml) gelöst. Innerhalb 1 h wurde KMnO4 (4,7 g; 29,79 mmol) zugegeben. Anschließend wurde 1 h bei Raumtemperatur (RT) gerührt. Dann wurde mit gesättigter NaHCO₃-Lösung entfärbt. Das Reaktionsvolumen wurde am Rotationsverdampfer reduziert. Danach wurde mit -CH₂Cl₂-extrahiert, die organische Phase mit Wasser gewaschen und über Na₂SO₄ getrocknet. Das Lösemittel wurde dann am Rotationsverdampfer entfernt. Der ölige Rückstand wurde mittels Säulenchromatographie (SiO₂; EtOAc/n-Heptan:1:2) gereinigt. 1-Hydroxy-3-(4-methoxy-phenyl)-propan-2-on wurde als weißes, kristallines Pulver erhalten. MS: 181,20 (M+H); Rₜ: 0,83 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X21,4 µ); 30 °C]

### 6.2 2-[1-Hydroxymethyl-2-(4-methoxy-phenyl)-ethylamino]-3-methyl-buttersäure-t-butylester (38)

Valin-t-butylester Hydrochlorid (302 mg; 1,45 mmol) wurde in wenig 1N NaOH gelöst, mit CH₂Cl₂ extrahiert (3x) und die vereinigten, organischen Phasen über Na₂SO₄ getrocknet. Nach dem Einengen am Rotationsverdampfer wurde das so erhaltene freie Amin in 1,2-Dichlorethan (2,4 ml) gelöst. Dann wurde 1-Hydroxy-3-(4-methoxy-phenyl)-propan-2-on (200 mg, 1,1 mmol) und Eisessig (38 µl) zugegeben. Es wurde 1 h bei RT gerührt. NaBH(OAc)₃ (303 mg; 1,45 mmol) wurde zugegeben. Danach wurde 4 h bei RT gerührt. Die Reaktionsmischung wurde in CH₂Cl₂ aufgenommen, mit gesättigter NaHCO₃-Lösung gewaschen und die organische Phase über Na₂SO₄ getrocknet. Nach dem Einengen am Rotationsverdampfer wurde ein öliger Rückstand erhalten, der mit präperativer HPLC gereinigt wurde. Von den vereinigten Produktfraktionen wurde am Rotationsverdampfer das Acetonitril entfernt und gesättigte NaHCO₃-Lösung zugegeben. Dann wurde mit CH₂Cl₂ extrahiert, über Na₂SO₄ getrocknet. Nach dem Einengen am Rotationsver-dampfer wurde 2-[1-Hydroxymethyl-2-(4-methoxy-phenyl)-ethylamino]-3-methyl-buttersäure-t-butylester (74 mg; 0,22 mmol) erhalten. MS: 338,20 (M+H); Rₜ: 1,05 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### 6.3 2-[4-(4-Methoxy-benzyl)-2-oxo-[1,2,3]oxathiazolidin-3-yl]-3-methyl-buttersäure-t-butylester (39)

2-[1-Hydroxymethyl-2-(4-methoxy-phenyl)-ethylamino]-3-methyl-buttersäure-t -butylester (2,49 g; 7,4 mmol) wurde in CH₂Cl₂ (180 ml) gelöst. Die Reaktionslösung würde auf-78°C gekühlt. Dann wurde Pyridin (3 ml; 37 mmol) zugegeben und danach Thionylchlorid (0.64 ml; 8,88 mmol) zugetropft. Die Reaktionsmischung wurde 1 h gerührt, wobei man die Temperatur bis auf 0 °C kommen ließ. Es wurde mit CH₂Cl₂ aufgenommen, mit wässriger HCl (1%; 2x) und NaHCO₃-Lösung gewaschen. Die organische Phase würde über Na₂SO₄ getrocknet und am Rotationsverdampfer eingeengt. Das Rohprodukt wurde durch Filtration über Silicagel (100 g) gereinigt. 2-[4-(4-Methoxy-benzyl)-2-oxo-[1,2,3]oxathiazolidin-3-yl]-3-methyl-buttersäure-t-butylester (2,35 g; 0,61 mmol) wurde als dunkelgelbes Öl erhalten. MS: 384,20 (M+H); Rₜ: 1,76 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### 6.4 2-[4-(4-Methoxy-benzyl)-2,2-dioxo-[1,2,3]oxathiazolidin-3-yl]-3-methyl-buttersäure-t-butylester (40)

2-[4-(4-Methoxy-benzyl)-2-oxo-[1,2,3]oxathiazolidin-3-yl]-3-methyl-buttersäure-t-butylester (2,2 g; 5,7 mmol) wurde in Acetonitril (15 ml) gelöst und die Reaktionslösung wurde auf 0 °C gekühlt. Dann wurde NalO₄ (1,47 g; 6,8 mmol), RuCl₃·H₂O (128,5 mg; 0,57 mmol) und Wasser (15 ml) zugegeben. Die Reaktionsmischung wurde 5 Minuten bei 0 °C und dann 30 Minuten bei RT gerührt. Dann wurde NaHCO₃-Lösung zugegeben, mit CH₂Cl₂ (3x) extrahiert, die organische Phase über NaSO₄ getrocknet und am Rotationsverdampfer eingeengt. Das Rohprodukt wurde über ein SiO₂-Kartusche (10 g) filtriert. Es wurde 2-[4-(4-Methoxy-benzyl)-2,2-dioxo-[1,2,3]oxathiazolidin-3-yl]-3-methyl-buttersäure-t-butylester (2,02 g; 5,0 mmol) als gelbes Öl erhalten. MS: 417,45 (M+NH₄⁺); Rt: 1,86 min [Methode: Gradient 0 min 96% H₂O (0,05%TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X21,4 p); 30 °C]

### 6.5 2-[2-Benzylamino-1-(4-methoxy-benzyl)-ethylamino]-3-methyl-buttersäure-t-butylester (41)

2-[4-(4-Methoxy-benzyl)-2,2-dioxo-[1,2,3]oxathiazolidin-3-yl]-3-methyl-buttersäure-t-butylester (1,0 g; 2.5 mmol) wurde in Acetonitril (15 ml) gelöst. Es wurde Cs₂CO₃ (1,63 g; 5 mmol) und Benzylamin (0,5 ml; 4,5 mmol) zugegeben. Die Reaktionsmischung wurde 4 h bei 55 °C gerührt. Danach ließ man auf RT kommen und filtrierte die Reaktionsmischung über eine Klärschicht. Der Rückstand wurde mit Acetonitril gewaschen. Das Filtrat wurde eingeengt und in CH₂Cl₂ (10 ml) aufgenommen. Es wurde wässrige H₂SO₄ (20%; 5 ml) zugegeben und bei RT 1,5 h gerührt. Darin wurden die Phasen getrennt, die wässrige Phase wurde mit CH₂Cl₂ (2x) extrahiert, die vereinigten organischen Phasen wurden über Na₂SO₄ getrocknet und am Rotationsverdampfer eingeengt. Das Rohrprodukt wurde mit CH₂Cl₂/CH₃CN/Et₂O verrührt und eingeengt. 2-[2-Benzylamino-1-(4-methoxy-benzyl)-ethylamino]-3-methyl-buttersäure-t-butylester (1,15 g; 2,5 mmol) wurde als gelblicher Schaum erhalten. MS: 427,20 (M+H); Rₜ: 1,42 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### 6.6 2-[3-Benzyl-5-(4-methoxy-benzyl)-2-oxo-imidazolidin-1 -yl]-3-methyl-buttersäure-t-butylester (42)

2-[2-Benzylamino-1-(4-methoxy-benzyl)-ethylamino]-3-methyl-buttersäure-t-butylester (0,64 g; 1,5 mmol) wurde in Toluol (40 ml) gelöst. Es wurde Triethylamin (457 µL; 3,3 mmol) und Triphosgen (0,49 g; 1,65 mmol) zugegeben. Die Reaktionsmischung wurde 4,5 h bei RT gerührt. Dann wurde mit Wasser (1x), gesättigter NaHCO₃-Lösung (1x) und wieder mit Wasser (1x) gewaschen. Die organische Phase wurde über Na₂SO₄ getrocknet und am Rotationsverdampfer eingeengt. Das Rohprodukt wurde über ein SiO₂-Kartusche (EtOAc/n-Heptan 1:3) chromatographiert. 2-[3-Benzyl-5-(4-methoxy-benzyl)-2-oxo-imidazolidin-1-yl]-3-methyl-buttersäure-t-butylester (0,22 g, 0,48 mmol) wurde als gelbes Öl erhalten. MS: 453,20 (M+H); Rt: 1,94 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### 6.7 2-[3-Benzyl-5-(4-methoxy-benzyl)-2-oxo-imidazolidin-1-yl]-3-methyl-buttersäure (43)

2-[3-Benzyl-5-(4-methoxy-benzyl)-2-oxo-imidazolidin-1-yl]-3-methyl-buttersäure-t-butylester (0,22 g; 0,48 mmol) wurde in CH₂Cl₂ (2 ml) gelöst und die Reaktionslösung wurde auf 0 °C gekühlt. Dann wurde TFA (2 ml) zugegeben. Die Reaktionsmischung wurde 3 h bei 0 °C gerührt. Danach wurde am Rotationsverdampfer eingeengt, der Rückstand mit Wasser aufgenommen und mit CH₂Cl₂ (3x) extrahiert. Die organische Phase wurde über Na₂SO₄ getrocknet und am Rotationsverdampfer eingeengt. 2-[3-Benzyl-5-(4-methoxy-benzyl)-2-oxo-imidazolidin-1-yl]-3-methyl-buttersäure (0,18 g; 0,45 mmol) wurde als gelbes Öl erhalten. MS: 397,20 (M+H); Rₜ: 1,52 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### 6.8 2-[3-Benzyl-5-(4-methoxy-benzyl)-2-oxo-imidazolidin-1-yl]-N-hydroxy-3-methyl-butyramid (44)

2-[3-Benzyl-5-(4-methoxy-benzyl)-2-oxo-imidazolidin-1-yl]-3-methyl-buttersäure (174 mg; 0,44 mmol) wurde in THF (4 ml) gelöst. Chlorameisensäureethylester (42 µL; 0,53 mmol), N-Ethylmorpholin (112 µL; 0,88 mmol) und O-Trimethylsilylhydroxalamin (90%) wurden zugegeben. Die Reaktionsmischung wurde 3 h bei RT gerührt und dann am Rotationsverdampfer eingeengt. Der Rückstand wurde in CH₂Cl₂ aufgenommen, mit H₂O (1x) extrahiert, die organische Phase wurde über Na₂SO₄ getrocknet und am Rotationsverdampfer eingeengt. Das Rohprodukt wurde über ein SiO₂-Kartusche (50 g) (CH₂Cl₂/MeOH 50:1) gereinigt.

Nach anschließender Kristallisation aus Diethylether/n-Pentan wurde 2-[3-Benzyl-5-(4-methoxy-benzyl)-2-oxo-imidazolidin-1-yl]-N-hydroxy-3-methyl-butyramid (92 mg; 0,23 mmol) als weiße Kristalle erhalten. MS: 412,20 (M+H); Rₜ: 1,35 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### Beispiel 1: 2-[3-(4-Benzyloxy-benzyl)-2-oxo-imidazolidin-1-yl]-N-hydroxy-3-methyl-butyramid

2-[3-(4-Benzyloxy-benzyl)-2-oxo-imidazolidin-1-yl]-N-hydroxy-3-methyl-butyramid (25 mg) wurde analog zur Durchführung in 3.1 aus Imidazolidin-2-on gewonnen. MS: 398,15 (M+H); Rₜ: 1,30 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### Beispiel 2: 2-[3-(4-Benzyloxy-phenyl)-2-oxo-imidazolidin-1-yl]-N-hydroxy-3-methyl-butyramid

2-[3-(4-Benzyloxy-phenyl)-2-oxo-imidazolidin-1-yl]-N-hydroxy-3-methyl-butyramid (22 mg) wurde analog zur Durchführung in 3.2 aus 2-Oxazolidon gewonnen. MS: 384,25 (M+H); Rₜ: 1,94 min [Methode: Gradient Acetonitril + 0,08% Ameisensäure: H₂O + 0,1% Ameisensäure von 5:95 (0 min) bis 95:5 (2,5 min) bis 95:5 (3 min); Flussrate 1,3 ml/min; Säule YMC Jsphere 33*2.1]

### Beispiel 3 2-[3-(3-Benzyloxy-benzyl)-2-oxo-imidazolidin-1-yl]-N-hydroxy-3-methyl-butyramid

2-[3-(3-Benzyloxy-benzyl)-2-oxo-imidazolidin-1-yl]-N-hydroxy-3-methyl-butyramid (8 mg) wurde analog zur Durchführung in 3.3 aus Valin-t-butyl-ester Hydrochlorid gewonnen. MS: 398,15 (M+H); Rₜ: 1,46 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X21,4 µ); 30 °C]

### Beispiel 4 N-Hydroxy-3-methyl-2-[2-oxo-3-(4-phenoxy-benzyl)-imidazolidin-1-yl]-butyramid

N-Hydroxy-3-methyl-2-[2-oxo-3-(4-phenoxy-benzyl)-imidazolidin-1-yl]-butyramid (14 mg) wurde analog zur Durchführung in 3.3 aus Valin-t-butyl-ester Hydrochlorid gewonnen. MS: 384,15 (M+H); Rₜ: 1,45 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ);. 30 °C]

### Beispiel 5 2-[3-(6-Benzyloxy-pyridin-3-ylmethyl)-2-oxo-imidazolidin-1-yl]-N-hydroxy-3-methyl-butyramid

2-[3-(6-Benzyloxy-pyridin-3-ylmethyl)-2-oxo-imidazolidin-1-yl]-N-hydroxy-3-methyl-butyramid (31 mg) wurde analog zur Durchführung in 3.3 aus Valin-t-butyl-ester Hydrochlorid gewonnen. MS: 399,25 (M+H); Rₜ: 1,43 min [Methode: Gradient Acetonitril + 0,08% Ameisensäure : H₂O + 0,1% Ameisensäure von 5:95 (0 min) bis 95:5 (2,5 min) bis 95:5 (3 min); Flussrate 1,3 ml/min; Säule YMC Jsphere 33*2.1]

### Beispiel 6 2-(3-Biphenyl-4-ylmethyl-2-oxo-imidazolidin-1-yl)-N-hydroxy-3-methyl-butyramid

2-(3-Biphenyl-4-ylmethyl-2-oxo-imidazolidin-1-yl)-N-hydroxy-3-methyl-butyramid (101 mg) wurde analog zur Durchführung in 3.3 aus Valin-t-butyl-ester Hydrochlorid gewonnen. MS: 398,29 (M+H); Rₜ: 1,92 min [Methode: Gradient Acetonitril + 0,08% Ameisensäure : H₂O + 0,1% Ameisensäure von 5:95 (0 min) bis 95:5 (2,5 min) bis 95:5 (3 min); Flussrate 1,3 ml/min; Säule YMC Jsphere 33*2.1]

### Beispiel 7 2-[3-Benzyl-5-(4-methoxy-phenyl)-2-oxo-imidazolidin-1-yl]-N-hydroxy-3-methyl-butyramid

2-[3-Benzyl-5-(4-methoxy-phenyl)-2-oxo-imidazolidin-1-yl]-N-hydroxy-3-methyl-butyramid (16 mg) wurde analog zur Durchführung in 3.4 aus 1-(4-Methoxy-phenyl)-ethanon gewonnen. MS: 398,15 (M+H); Rₜ: 1,31 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; säule 0,4 µL (YMC J'sphere ODS H80 20X2 1,4 p); 30 °C]

### Beispiel 8 N-Hydroxy-2-[3-(4-hydroxy-benzyl)-2-oxo-imidazolid in-1-yl]- 3-methyl-butyramid

N-Hydroxy-2-[3-(4-hydroxy-benzyl)-2-oxo-imidazolidin-1-yl]-3-methyl-butyramid (5 mg) wurde analog zur Durchführung in 3.5 aus 2-[3-(4-Benzyloxy-benzyl)-2-oxo-imidazolidin-1-yl]-3-methyl-buttersäureethylester gewonnen. MS: 308,21 (M+H); Rₜ: 1,29 min [Methode: Gradient Acetonitril + 0,08% Ameisensäure : H₂O + 0,1% Ameisensäure von 5:95 (0 min) bis 95:5 (2,5 min) bis 95:5 (3 min); Flussrate 1,3 ml/min; Säule YMC Jsphere 33*2.1]

### Beispiel 9 N-Hydroxy-2-[3-(3-hydroxy-benzyl)-2-oxo-imidazolidin-1-yl]-3-methyl-butyramid

N-Hydroxy-2-[3-(3-hydroxy-benzyl)-2-oxo-imidazolidin-1-yl]-3-methyl-butyramid (8 mg) wurde analog zur Durchführung in 3.5 aus 2-[3-(3-Benzyloxy-benzyl)-2-oxo-imidazolidin-1-yl]-3-methyl-buttersäure-t-butylester gewonnen. MS: 308,15 (M+H); Rt: 0,97 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### Beispiel 10 N-Hydroxy-3-methyl-2-{2-oxo-3-[4-(pyridin-4-ylmethoxy)-benzyl]-imidazolidin-1-yl}-butyramid; Verbindung mit TFA

N-Hydroxy-3-methyl-2-{2-oxo-3-[4-(pyridin-4-ylmethoxy)-benzyl]-imidazolidin-1-yl}-butyramid; Verbindung mit TFA (9 mg) wurde analog zur Durchführung in **3.5** aus 2-[3-(4-Benzyloxy-benzyl)-2-oxo-imidazolidin-1-yl]-3-methyl-buttersäureethylester gewonnen. MS: 399,40 (M+H); Rₜ: 0,78 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X21,4 µ); 30 °C]

### Beispiel 11 N-Hydroxy-3-methyl-2-{2-oxo-3-[4-(pyridin-3-ylmethoxy)-benzyl]-imidazolidin-1-yl}-butyramid mit TFA

N-Hydroxy-3-methyl-2-{2-oxo-3-[4-(pyridin-3-ylmethoxy)-benzyl]-imidazolidin-1-yl}-butyramid mit TFA (6 mg) wurde analog zur Durchführung in **3.5** aus 2-[3-(4-Benzyloxy-benzyl)-2-oxo-imidazolidin-1-yl]-3-methyl-buttersäureethylester gewonnen. MS: 399,45 (M+H); Rt: 0,77 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### Beispiel 12 N-Hydroxy-3-methyl-2-{2-oxo-3-[4-(pyridin-2-ylmethoxy)-benzyl]-imidazolidin-1-yl}-butyramid mit TFA

N-Hydroxy-3-methyl-2-(2-oxo-3-[4-(pyridin-2-ylmethoxy)-benzyl]-imidazolidin-1-yl)-butyramid mit TFA (6 mg) wurde analog zur Durchführung in **3.5** aus 2-[3-(4-Benzyloxy-benzyl)-2-oxo-imidazolidin-1-yl]-3-methyl-buttersäureethylester gewonnen. MS: 399,45 (M+H); Rₜ: 0,79 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X21,4 µ); 30°C]

### Beispiel 13 2-[3-(4-But-2-ynyloxy-benzyl)-2-oxo-imidazolidin-1-yl]-N-hydroxy-3-methyl-butyramid

2-[3-(4-But-2-ynyloxy-benzyl)-2-oxo-imidazolidin-1-yl]-N-hydroxy-3-methyl-butyramid (4 mg) wurde analog zur Durchführung in 3.5 aus 2-[3-(4-Benzyloxy-benzyl)-2-oxo-imidazolidin-1-yl]-3-methyl-buttersäureethylester gewonnen. MS: 360,45 (M+H); Rₜ: 1,15 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X21,4 p); 30 °C]

### Beispiel 14 N-Hydroxy-3-methyl-2-{3-[4-(2-methyl-quinolin-4-ylmethoxy)-benzyl]-2-oxo-imidazolidin-1-yl}-butyramid mit TFA

N-Hydroxy-3-methyl-2-{3-[4-(2-methyl-quinolin-4-ylmethoxy)-benzyl]-2-oxo-imidazolidin-1-yl}-butyramid mit TFA (4 mg) wurde analog zur Durchführung in 3.5 aus 2-[3-(4-Benzyloxy-benzyl)-2-oxo-imidazolidin-1-yl]-3-methyl-buttersäureethylester gewonnen. MS: 463,55 (M+H); Rₜ: 0,89 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### Beispiel 15 2-[3-Benzyl-5-(4-methoxy-benzyl)-2-oxo-imidazolidin-1-yl]-N-hydroxy-3-methyl-butyramid

2-[3-Benzyl-5-(4-methoxy-benzyl)-2-oxo-imidazolidin-1-yl]-N-hydroxy-3-methyl-butyramid (92 mg) wurde analog zur Durchführung in 3.6 aus 4-Allylanisol gewonnen. MS: 412,20 (M+H); Rₜ: 1,35 min [Methode: Gradient 0 min 96% H₂O (0,05% TFA) 2,0 min 95% Acetonitril, 95% Acetonitril bis 2,4 min, 4% Acetonitril 2,45 min; Flussrate 1 ml/min; Säule 0,4 µL (YMC J'sphere ODS H80 20X2 1,4 µ); 30 °C]

### Beispiel 16 2-[3-(4-Benzyloxy-benzyl)-5-(4-methoxy-benzyl)-2-oxo-imidazolidin-1-yl]-N-hydroxy-3-methyl-butyramid

2-[3-(4-Benzyloxy-benzyl)-5-(4-methoxy-benzyl)-2-oxo-imidazolidin-1-yl]-N-hydroxy-3-methyl-butyramid (55 mg) wurde analog zur Durchführung in **3.6** aus 4-Allylanisol gewonnen. MS: 518,29 (M+H); Rₜ: 2,54 min [Methode: Gradient Acetonitril + 0,05% TFA: H₂O + 0,05% TFA von 5:95 (0 min) bis 95:5 (3,4 min) bis 95:5 (4,4 min); Flussrate 1 ml/min; Säule YMC Jsphere 33*2]

### Beispiel 17 N-Hydroxy-2-[5-(4-methoxy-benryl)-3-methyl-2-oxo-imidazolidin-1-yl]-3-methyl-butyramid

N-Hydroxy-2-[5-(4-methoxy-benzyl)-3-methyl-2-oxo-imidazolidin-1-yl]-3-methyl-butyramid (80 mg) wurde analog zur Durchführung in **3.6** aus 4-Allylanisol gewonnen. MS: 336,22 (M+H); Rₜ: 1,62 min [Methode: Gradient Acetonitril + 0,05% TFA : H₂O + 0,05% TFA von 5:95 (0 min) bis 95:5 (3,4 min) bis 95:5 (4,4 min); Flussrate 1 ml/min; Säule YMC Jsphere 33*2]

### Beispiel 18 2-[5-Benzo[1,3]dioxol-5-ylmethyl-3-(4-benzyloxy-benzyl)-2-oxo-imidazolidin-1-yl]-N-hydroxy-3-methyl-butyramid

2-[5-Benzo[1,3]dioxol-5-ylmethyl-3-(4-benzyloxy-benzyl)-2-oxo-imidazolidin-1-yl]-N-hydroxy-3-methyl-butyramid (110 mg) wurde analog zur Durchführung in **3.6** aus 4-Allylanisol gewonnen. MS: 532,31 (M+H); Rₜ: 2,62 min [Methode: Gradient Acetonitril + 0,05% TFA: H₂O + 0,05% TFA von 5:95 (0 min) bis 95:5 (3,4 min) bis 95:5 (4,4 min); Flussrate 1 ml/min; Säule YMC Jsphere 33*2]

### Beispiel 19 2-(5-Benzo[1,3]dioxol-5-ylmethyl-3-benzyl-2-oxo-imidazolidin-1-yl)-N-hydroxy-3-methyl-butyramid

2-(5-Benzo[1,3]dioxol-5-ylmethyl-3-benzyl-2-oxo-imidazolidin-1-yl)-N-hydroxy-3-methyl-butyramid (100 mg) wurde analog zur Durchführung in **3.6** aus 4-Allylanisol gewonnen. MS: 426,26 (M+H); Rₜ: 2,07 min [Methode: Gradient Acetonitril +0,05% TFA : H₂O + 0,05% TFA von 5:95 (0 min) bis 95:5 (3,4 min) bis 95:5 (4,4 min); Flussrate 1 ml/min; Säule YMC Jsphere 33*2]

### Pharmakologische Beispiele:

Die Aktivität der erfindungsgemäßen cyclischen Harnstoff Derivate wurden in verschiedenen in-vitro Assay Systemen auf inhibitorische Aktivität gegen die Proteasen ADAMTS-4 und TNFαconverting enzyme (TACE), sowie gegen Matrix-abbauende Metalloproteasen (MMP13) getestet. Die ADAMTS-4 Aktivität wurde mittels einer rekombinant hergestellten humanen ADAMTS-4 Protease und dem rAgg1mut Substrat gemessen. Das rAgg1mut Substrat enthält die interglobuläre Domäne des humanen Aggrecanmoleküles N-terminal fusioniert mit einer FLAG-Sequenz und C-terminal fusioniert mit einem humanen IgG-Fc Anteil. In der interglobulären Domäne befindet sich eine spezifische Spaltstelle für ADAMTS-4, deren Spaltung ein neues N-terminales Epitope generiert, das mittels eines neo-epitope spezifischen monoklonalen Antikörpers in einem ELISA Testsystem gemessen werden kann (Hörber, C., Büttner, FH., Kern, C., Schmiedeknecht, G. & Bartnik, E. (2000), Matrix Biology 19, 533-543).

Die Aktivität gegen TACE wurde mit einer kommerziell erhältlichen, rekombinant hergestellten TACE Protease (R&D Systems) und dem Substrat MCA-ProLeuAlaGInAlaVal-Dpa-ArgSerSerSerArg-NH2 (Bachem) gemessen. Die Spaltung des TACE-spezifischen Substrates wird im Fluorimeter bei den Wellenlängen Ex 320nm / Em 405nm gemessen und die Menge an gespaltenem Substrat über eine Eichkurve bestimmt.

Die Aktivitäten gegen MMP13 wurden mit rekombinant hergestellten Enzymen verschiedener Hersteller (Biotrend, Roche, Boehringer Mannheim) und mit verschiedenen MMP-spezifischen Peptid-Substraten (Bachem) gemessen. Die Spaltung der MMP-spezifischen Substrate wurde nach APMA Aktivierung der Proteasen bei pH 7,5 bzw. pH 6,5 im Fluorimeter bei den Wellenlängen von Ex 340nm / Em 405 nm gemessen.

Die Bestimmung des Proteoglykanabbaus erfolgte unter Verwendung von primären bovinen Chondrocyten, die aus dem Knorpel des Metacarpophalangeal Gelenks von etwa 6 Monate alten Rindern isoliert und 3 Wochen in einer Alginat Matrix bei 37 °C und 5 % CO₂ kultiviert wurden. Nach Stimulation von ∼160.000 Zellen mit 5 ng/ml humanem IL1α wurde nach 16 Stunden die freigesetzte Menge an Proteoglykan mittels eines kommerziell erhältlichen Dimethyl-Methylen-Blau Färbetestsystems (Biocolor Ltd.) bestimmt.

**Hemmung der ADAMTS-4 Aktivität (IC50 in µM):**

| Beispiel | 1 | 2 | 4 | 5 | 6 | 9 | 10 | 11 | 12 | 14 | 15 | 16 | 18 |
|---|---|---|---|---|---|---|---|---|---|---|---|---|---|
| ADAMTS-4 | 2,1 | 55 | 1,33 | 10 | 9,06 | 72,39 | 0,9 | 7,8 | 7,0 | 1,14 | 17 | 1,39 | 3,16 |

**Hemmung der MMP 13 Aktivität (IC50 in µM):**

| Beispiel | 1 | 4 | 7 | 10 | 11 | 15 | 16 | 17 | 18 | 19 |
|---|---|---|---|---|---|---|---|---|---|---|
| MMP13 | 5,0 | 0,08 | 0,01 | 4,0 | 5,4 | 0,4 | 0,42 | 3,17 | 0,6 | 0,34 |

**Hemmung der TACE Aktivität (IC50 in µM):**

| Beispiel | 1 | 3 | 4 | 7 | 9 | 10 | 11 | 12 | 13 | 14 | 15 | 16 | 18 | 19 |
|---|---|---|---|---|---|---|---|---|---|---|---|---|---|---|
| TACE | 2,47 | 2,0 | 5,0 | 9,0 | 3,04 | 1,14 | 3 | 8 | 5 | 0,01 | 8 | 3,08 | 1,9 | 9,29 |

**Inhibition der Proteoglykanfreisetzung aus IL1α-stimulierten primären, bovinen Chondrozyten Kulturen:**

| Beispiel | 1 | 10 | 14 | 16 |
|---|---|---|---|---|
| Hemmung der Proteoglykanfreisetzung (IC50 in µM) | >50 | >50 | 12,9 | 25,5 |

| | | | | |
|---|---|---|---|---|
| (> = mehr als) | | | | |

## Patentansprüche

1. Verbindung der Formel I 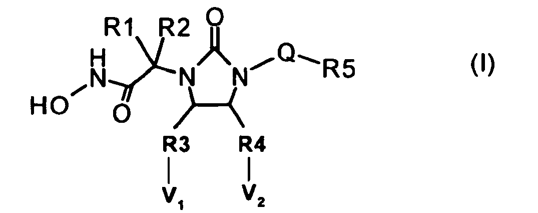 und/oder alle stereoisomeren Formen der Verbindung der Formel I und/oder Gemische diese Formen in jedem Verhältnis, und/oder ein physiologisch verträgliches Salz der Verbindung der Formel I, wobei
R1 und R2 gleich oder verschieden sind und unabhängig voneinander für
a) Wasserstoffatom,
b) -(C₁-C₆)-Alkyl,
c) -(C₃-C₆)-Cycloalkyl,
d) -(C₂-C₄)-Alkyl-Het, worin Het für einen mono- oder bizyklischen 4- bis 15-gliedrigen Heterozyklus steht, der mindestens ein Kohlenstoffatom und ein, zwei, drei oder vier Heteroatome aus der Reihe Stickstoff, Schwefel oder Sauerstoff enthält, worin der Heterozyklus unsubstituiert oder ein-, zwei- oder dreifach durch R8 substituiert ist, oder
e) -(C₂-C₄)-Alkyl-(C₆-C₁₄)-Aryl, worin Aryl unsubstituiert oder ein- oder zweifach durch R8 substituiert ist, stehen, oder
R1 und R2 bilden zusammen mit dem Kohlenstoffatom an das sie jeweils gebunden sind
a) -(C₃-C₆)-Cycloalkyl oder
b) einen mono- oder bizyklischen 4- bis 15-gliedrigen Heterozyklus, der mindestens ein Kohlenstoffatom und ein, zwei, drei oder vier Heteroatome aus der Reihe Stickstoff, Schwefel oder Sauerstoff enthält, worin der Heterozyklus unsubstituiert oder ein-, zwei- oder dreifach durch R8 substituiert ist,
R3 und R4 gleich oder verschieden sind und unabhängig voneinander für eine kovalente Bindung, -(CH₂)ₘ-, -(C₁-C₃)-Alkylen-O-(C₀-C₃)-Alkylen-, -(C₀-C₃)-Alkylen-C(O)-O-(CH₂)ₙ-, -(C₀-C₃)-Alkylen-C(O)-NR¹⁰-(CH₂)ₙ-, -(CH₂)ₘ-CH(OH)-(CH₂)ₙ-, -(C₁-C₃)-Alkylen-N(R¹⁰)-(CH₂)ₙ-, -(CH₂)ₘ-NR¹⁰-C(O)-(CH₂)ₙ-, -(CH₂)ₘ-NR¹⁰-C(O)-NR¹⁰-(CH₂)ₙ-, -(CH₂)ₘ-O-C(O)-NR¹⁰-(CH₂)ₙ-, -(CH₂)ₘ-NR¹⁰-C(O)-O-(CH₂)ₙ-, -(CH₂)ₘ-S-(CH₂)ₙ-, -(C₁-C₃)-Alkylen-S(O)-(CH₂)ₙ-, -(C₁-C₃)-Alkylen-SO₂-(CH₂)ₙ-, -(C₁-C₃)-Alkylen-SO₂-NH-(R¹⁰), -(CH₂)ₘ-SO₂-NR¹⁰-(CH₂)ₙ-, -(CH₂)ₘ-NR¹⁰-SO₂-(CH₂)ₙ- oder -(CH₂)ₘ-NR¹⁰-SO₂-NR¹⁰-(CH₂)ₙ- stehen, worin
n und m unabhängig voneinander gleich oder verschieden sind und m für die ganzen Zahlen 1, 2, 3, 4, 5 oder 6 steht und n für die ganzen Zahlen Null, 1, 2, 3, 4, 5 oder 6 steht und worin die Alkylenreste, die durch -(CH₂)ₘ- oder -(CH₂)ₙ- gebildet werden unsubstituiert oder ein-, zwei- oder dreifach substituiert sind durch Halogen, -NH₂ oder -OH oder ein -(C₃-C₆)-Cycloalkyl bilden, worin Cycloalkyl unsubstituiert oder ein-, zwei- oder dreifach durch Halogen, -NH₂ oder-OH substituiert ist,
R¹⁰ für Wasserstoffatom, -(C₁-C₆)-Alkyl, -(C₀-C₄)-Alkyl-OH, -(C₀-C₄)-Alkyl-O-(C₁-C₄)-Alkyl oder -(C₁-C₃)-Perfluoralkyl steht,
V₁, V₂ und R5 gleich oder verschieden sind und unabhängig voneinander für
a) Wasserstoffatom,
b) -(C₆-C₁₄)-Aryl, worin Aryl unsubstituiert oder ein- oder zweifach durch R8 oder den Rest -G-M substituiert ist oder
c) einen mono- oder bizyklischen 4- bis 15-gliedrigen Heterozyklus stehen, der mindestens ein Kohlenstoffatom und ein, zwei, drei oder vier Heteroatome aus der Reihe Stickstoff, Schwefel oder Sauerstoff enthält, worin der Heterozyklus unsubstituiert oder ein-, zwei- oder dreifach durch R8 oder den Rest -G-M substituiert ist,
M für
a) Wasserstoffatom,
b) -(C₆-C₁₄)-Aryl, worin Aryl unsubstituiert oder ein- oder zweifach durch R8 substituiert ist oder
c) einen mono- oder bizyklischen 4- bis 15-gliedrigen Heterozyklus steht, der mindestens ein Kohlenstoffatom und ein, zwei, drei oder vier Heteroatome aus der Reihe Stickstoff, Schwefel oder Sauerstoff enthält und worin der Heterozyklus unsubstituiert oder ein-, zwei- oder dreifach durch R8 substituiert ist,
R8 für
1) Halogen,
2) -NO₂,
3) -CN,
4) -C(O)-NH₂,
5) -SO₂-NH₂,
6) -OH,
7) -NH₂,
8) -O-CF₃,
9) -(C₆-C₁₄)-Aryl, worin Aryl unsubstituiert oder ein- oder zweifach durch Halogen oder -O-(C₁-C₈)-Alkyl substituiert ist,
10) -(C₁-C₈)-Alkyl, worin Alkyl unsubstituiert oder ein-, zwei- oder dreifach durch Halogen, NH₂, -OH oder Methoxy substituiert ist,
11) -O-(C₁-C₈)-Alkyl, worin Alkyl unsubstituiert oder ein-, zwei- oder dreifach durch Halogen, NH₂, -OH oder Methoxy substituiert ist,
12) -SO₂-CH₃ oder
13) -SO₂-CF₃ steht,
G für kovalente Bindung, -(CH₂)ₒ-, -(C₀-C₃)-Alkylen-O-(C₀-C₃)-Alkylen-, -(C₀-C₃)-Alkylen-C(O)-O-(CH₂)ₚ-, -(C₀-C₃)-Alkylen-C(O)-NR¹⁰-(CH₂)ₚ-, -(CH₂)ₒ-CH(OH)-(CH₂)ₚ-, -(C₀-C₃)-Alkylen-N(R¹⁰)-(CH₂)ₚ-, -(CH₂)ₒ-NR¹⁰-C(O)-(CH₂)ₚ-, -(CH₂)ₒ-NR¹⁰-C(O)-NR¹⁰-(CH₂)ₚ-, -(CH₂)₀-O-C(0)-NR¹⁰-(CH₂)ₚ-, -(CH₂)ₒ-NR¹⁰-C(O)-O-(CH₂)ₚ-, -(CH₂)ₒ-S-(CH₂)ₚ-, -(C₀-C₃)-Alkylen-S(O)-(CH₂)ₚ-, -(C₀-C₃)-Alkylen-SO₂-(CH₂)ₚ-, -(C₀-C₃)-Alkylen-SO₂-NH-(R¹⁰), -(CH₂)ₒ-SO₂-NR¹⁰-(CH₂)ₚ-, -(CH₂)ₒ-NR¹⁰-SO₂-(CH₂)ₚ-, -(C₀-C₃)-Alkylen-O-(C₂-C₄)-Alkenylen- oder-(CH₂)ₒ-NR¹⁰-SO₂-NR¹⁰-SO₂-(CH₂)ₚ- steht, worin
o und p gleich oder verschieden sind und unabhängig voneinander für die ganzen Zahlen Null, 1, 2, 3, 4, 5 oder 6 stehen und worin die Alkylenreste die durch - (CH₂)ₒ- oder -(CH₂)ₚ- gebildet werden unsubstituiert oder ein-, zwei- oder dreifach substituiert sind durch Halogen, -NH₂ oder -OH oder ein
-(C₃-C₆)-Cycloalkyl bilden, worin Cycloalkyl unsubstituiert oder ein-, zwei- oder dreifach durch Halogen, -NH₂ oder-OH substituiert ist, und R10 wie oben definiert ist, und
Q für kovalente Bindung, -(C₁-C₃)-Alkylen oder -(C₃-C₆)-Cycloalkyl steht, unter der Bedingung, dass wenigstens einer der Reste V₁, V₂ oder R5 für -(C₆-C₁₄)-Aryl oder einen mono- oder bizyklischen 4- bis 15-gliedrigen Heterozyklus steht, worin Aryl oder Heterozyklus unsubstituiert oder ein- oder zweifach durch R8 oder den Rest -G-M substituiert sind.

2. Verbindung der Formel I gemäß Anspruch 1, wobei
R1 und R2 gleich oder verschieden sind und unabhängig voneinander für Wasserstoffatom oder -(C₁-C₄)-Alkyl stehen, oder
R1 und R2 bilden zusammen mit dem Kohlenstoffatom an das sie jeweils gebunden sind -(C₃-C₆)-Cycloalkyl,
R3 und R4 gleich oder verschieden sind und unabhängig voneinander für eine kovalente Bindung, -(CH₂)ₘ-, -(C₁-C₃)-Alkylen-O-(C₀-C₃)-Alkylen-, -(C₀-C₃)-Alkylen-C(O)-O-(CH₂)ₙ-, -(C₀-C₂)-Alkylen-C(O)-NR¹⁰-(CH₂)ₙ-, -(CH₂)ₘ-CH(OH)-(CH₂)ₙ-, -(C₁-C₃)-Alkylen-N(R¹⁰)-(CH₂)ₙ-, -(CH₂)ₘ-NR¹⁰-C(O)-(CH₂)ₙ-, -(CH₂)ₘ-NR¹⁰-C(O)-NR¹⁰-(CH₂)ₙ-, -(CH₂)ₘ-O-C(O)-NR¹⁰-(CH₂)ₙ-, -(CH₂)ₘ-NR¹⁰-C(O)-O-(CH₂)ₙ-. -(CH₂)ₘ-S-(CH₂)ₙ-, -(C₁-C₃)-Alkylen-S(O)-(CH₂)ₙ-, -(C₁-C₃)-Alkylen-SO₂-(CH₂)ₙ-, -(C₁-C₃)-Alkylen-SO₂-NH-(R¹⁰), -(CH₂)ₘ-SO₂-NR¹⁰-(CH₂)ₙ-, -(CH₂)ₘ-NR¹⁰-SO₂-(CH₂)ₙ- oder -(CH₂)ₘ-NR¹⁰-SO₂-NR¹⁰-(CH₂)ₙ- stehen, worin
n und m unabhängig voneinander gleich oder verschieden sind und m für die ganzen Zahlen 1, 2, 3, 4, 5 oder 6 steht und n für die ganzen Zahlen Null, 1, 2, 3, 4, 5 oder 6 steht und worin die Alkylenreste die durch -(CH₂)ₘ- oder -(CH₂)ₙ- gebildet werden unsubstituiert oder ein-, zwei- oder dreifach substituiert sind durch Halogen, -NH₂ oder-OH oder ein -(C₃-C₆)-Cycloalkyl bilden, worin Cycloalkyl unsubstituiert oder ein-, zwei- oder dreifach durch Halogen, -NH₂ oder-OH substituiert ist,
R¹⁰ für Wasserstoffatom, -(C₁-C₆)-Alkyl, -(C₀-C₄)-Alkyl-OH, -(C₀-C₄)-Alkyl-O-(C₁-C₄)-Alkyl oder -(C₁-C₃)-Perfluoralkyl steht, V₁, V₂ und R5 gleich oder verschieden sind und unabhängig voneinander für
a) Wasserstoffatom,
b) -(C₆-C₁₄)-Aryl, worin Aryl ein Rest aus der Reihe Phenyl, Naphthyl, 1-Naphthyl, 2-Naphthyl, Anthryl oder Fluorenyl bedeutet unsubstituiert oder ein- oder zweifach durch R8 oder den Rest -G-M substituiert ist oder
c) einen mono- oder bizyklischen 4- bis 15-gliedrigen Heterozyklus stehen, worin Heterozyklus ein Rest aus der Reihe Acridinyl, Azetidinyl, Benzimidazolyl, Benzodioxol, Benzodiazin, Benzofuranyl, Benzothiofuranyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, beta-Carbolinyl, Chinazolinyl, Chinolinyl, 4H-Chinolizinyl, Chinoxalinyl, Chinuclidinyl, Chromanyl, Chromenyl, Decahydrochinolinyl, Dihydrofuran, Dithiazinyl, Dithiazolly, Fuaranyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl, Isothiazolyl, Isoxazolyl, Morpholinyl, Naphthyridinyl, Octahydr-isochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Phenanthridinyl, Phenanthrolinyl, Phenazinyl, Phenothiazinyl, Phenoxathiinyl. Phenoxazinyl, Phthalazinyl, Piperazinyl, Piperidinyl, Pteridinyl, Purinyl, Pyranyl, Pyrazinyl, Pyroazolidinyl, Pyrazolinyl, Pyrazolyl, Pyridazinyl, Pryidooxazolyl, Pyridoimidazolyl, Pyridothiazolyl, Pyridothiophenyl, Pyridyl, Pyrimidinyl, Pyrrolidinyl, Pyrrolinyl, Pyrrolyl, Tetrahydrofuranyl, Tetrahydroisochinolinyl, Tetrahydrochinolinyl, 1,2,3-Thiadiazolyl, 1,2,4-Thiadiazolyl, 1,2,5-Thiadiazolyl, 1,3,4-Thiadiazolyl, Thianthrenyl, Thiazolyl, Thienyl, Thienothiazolyl, Thienooxazolyl, Thienoimidazolyl, Triazinyl, 1,2,3-Triazolyl, 1,2,4-Triazolyl, 1,2,5-Triazolyl, 1,3,4-Triazolyl und Xanthenyl bedeutet, worin der Heterozyklus unsubstituiert oder ein-, zwei- oder dreifach durch R8 oder den Rest -G-M substituiert ist,
M für
a) Wasserstoffatom,
b) -(C₆-C₁₄)-Aryl, worin Aryl wie oben definiert ist und unsubstituiert oder ein- oder zweifach durch R8 substituiert ist oder
c) einen mono- oder bizyklischen 4- bis 15-gliedrigen Heterozyklus steht, worin Heterozyklus wie oben definiert ist und worin der Heterozyklus unsubstituiert oder ein-, zwei- oder dreifach durch R8 substituiert ist,
R8 für
1) Halogen,
2) -NO₂,
3) -CN,
4) -C(O)-NH₂,
5) -SO₂-NH₂,
6) -OH,
7) -NH₂,
8) -O-CF₃,
9) -(C₆-C₁₄)-Aryl, worin Aryl unsubstituiert oder ein- oder zweifach durch Halogen oder -O-(C₁-C₈)-Alkyl substituiert ist,
10) -(C₁-C₈)-Alkyl, worin Alkyl unsubstituiert oder ein-, zwei- oder dreifach durch Halogen, NH₂, -OH oder Methoxy substituiert ist,
11) -O-(C₁-C₈)-Alkyl, worin Alkyl unsubstituiert oder ein-, zwei- oder dreifach durch Halogen, NH₂, -OH oder Methoxy substituiert ist,
12) -SO₂-CH₃ oder
13) -SO₂-CF₃ steht,
G für kovalente Bindung, -(C₀-C₃)-Alkylen-O-(C₀-C₃)-Alkylen-, -(CH₂)ₒ-CH(OH)-(CH₂)ₚ-, -(C₀-C₃)-Alkylen-C(O)-O-(CH₂)ₚ-, -(C₀-C₃)-Alkylen-C(O)-NR¹⁰-(CH₂)ₚ-, -(CH₂)ₒ-, -(C₀-C₃)-Alkylen-N(R¹⁰)-(CH₂)ₚ-, -(CH₂)ₒ-NR¹⁰-C(O)-(CH₂)ₚ-, -(CH₂)₀-O-C(O)-NR¹⁰-(CH₂)ₚ-, -(CH₂)ₒ-S-(CH₂)ₚ-, -(CH₂)ₒ-NR¹⁰-C(O)-NR¹⁰-(CH₂)ₚ-, -(CH₂)ₒ-NR¹⁰-C(O)-O-(CH₂)ₚ-, -(C₀-C₃)-Alkylen-S(O)-(CH₂)ₚ-_{,} -(C₀-C₃)-Alkylen-SO₂-(CH₂)ₚ-, -(C₀-C₃)-Alkylen-SO₂-NH-(R¹⁰), -(CH₂)ₒ-SO₂-NR¹⁰-(CH₂)ₚ-, -(CH₂)ₒ-NR¹⁰-SO₂-(CH₂)ₚ-, -(C₀-C₃)-Alkylen-O-(C₂-C₄)-Alkenylen-, oder -(CH₂)ₒ-NR¹⁰-SO₂-NR¹⁰-(CH₂)ₚ- steht, worin
o und p gleich oder verschieden sind und unabhängig voneinander für die ganzen Zahlen Null, 1, 2, 3, 4, 5 oder 6 stehen und worin die Alkylenreste die durch -(CH₂)ₒ- oder -(CH₂)ₚ- gebildet werden unsubstituiert oder ein-, zwei- oder dreifach substituiert sind durch Halogen, -NH₂ oder -OH oder ein -(C₃-C₆)-Cycloalkyl bilden, worin Cycloalkyl unsubstituiert oder ein-, zwei- oder dreifach durch Halogen, -NH₂ oder -OH substituiert ist, und R10 wie oben definiert ist, und
Q für kovalente Bindung, -(C₁-C₃)-Alkylen oder -(C₃-C₆)-Cycloalkyl steht, unter der Bedingung, dass wenigstens einer der Reste V₁, V₂ oder R5 für -(C₆-C₁₄)-Aryl oder einen mono- oder bizyklischen 4- bis 15-gliedrigen Heterozyklus steht, worin Aryl oder Heterozyklus unsubstituiert oder ein- oder zweifach durch R8 oder den Rest -G-M substituiert sind.

3. Verbindung der Formel I gemäß den Ansprüchen 1 oder 2, wobei
R1 und R2 gleich oder verschieden sind und unabhängig voneinander für Wasserstoffatom oder -(C₁-C₄)-Alkyl stehen, oder
R1 und R2 bilden zusammen mit dem Kohlenstoffatom an das sie jeweils gebunden sind -(C₃-C₆)-Cycloalkyl,
R3 und R4 gleich oder verschieden sind und unabhängig voneinander für eine kovalente Bindung, -(CH₂)ₘ- oder -(C₁-C₃)-Alkylen-O-(C₀-C₃)-Alkylen-, worin m für die ganze Zahl 1 steht, und worin der Alkylenrest, der durch -(CH₂)ₘ- gebildet wird unsubstituiert ist oder einfach mit -OH substituiert ist,
V₂ für Wasserstoffatom steht,
V₁ und R5 gleich oder verschieden sind und unabhängig voneinander für
a) Wasserstoffatom,
b) -(C₆-C₁₄)-Aryl, worin Aryl Phenyl bedeutet und unsubstituiert oder ein- oder zweifach durch R8 oder den Rest -G-M substituiert ist oder
c) einen mono- oder bizyklischen 4- bis 15-gliedrigen Heterozyklus stehen, worin Heterozyklus ein Rest aus der Reihe Benzodioxol, Chinolinyl oder Pyridyl, bedeutet, worin der Heterozyklus unsubstituiert oder ein-, zwei- oder dreifach durch R8 oder den Rest -G-M substituiert ist,
M für
a) Wasserstoffatom,
b) -(C₆-C₁₄)-Aryl, worin Aryl Phenyl bedeutet und unsubstituiert oder ein- oder zweifach durch R8 substituiert ist oder
c) einen mono- oder bizyklischen 4- bis 15-gliedrigen Heterozyklus steht, worin Heterozyklus wie oben definiert ist und worin der Heterozyklus unsubstituiert oder ein-, zwei- oder dreifach durch R8 substituiert ist,
R8 für Halogen, -OH oder -(C₁-C₄)-Alkyl oder-O-(C₁₋C₄₎-Alkyl steht,
G für kovalente Bindung, -(C₀-C₃)-Alkylen-O-(C₀-C₃)-alkylen- oder -(C₀-C₃)-Alkylen-O-(C₂-C₄)-Alkenylen- steht, und
Q für kovalente Bindung oder -(C₁-C₃)-Alkylen steht, unter der Bedingung, dass wenigstens einer der Reste V₁ oder R5 für -(C₆-C₁₄)-Aryl oder einen mono- oder bizyklischen 4- bis 15-gliedrigen Heterozyklus steht, worin Aryl oder Heterozyklus unsubstituiert oder ein- oder zweifach durch R8 oder den Rest -G-M substituiert sind.

4. Verbindung der Formel I gemäß einem oder mehreren der Ansprüche 1 bis 3, **dadurch gekennzeichnet, dass** es die Verbindung
2-[3-(4-Benzyloxy-benzyl)-2-oxo-imidazol id in-1-yl]-N-hydroxy-3-methyl-butyram id,
2-[3-(4-Benzyloxy-phenyl)-2-oxo-imidazolidin-1-yl]-N-hydroxy-3-methyl-butyramid,
2-[3-(3-Benzyloxy-benzyl)-2-oxo-imidazolidin-1-yl]-N-hydroxy-3-methyl-butyramid,
N-Hydroxy-3-methyl-2-[2-oxo-3-(4-phenoxy-benzyl)-imidazolidin-1-yl]-butyramid,
2-[3-(6-Benzyloxy-pyridin-3-ylmethyl)-2-oxo-imidazolidin-1-yl]-N-hydroxy-3-methyl-butyramid,
2-(3-Biphenyl-4-ylmethyl-2-oxo-imidazolidin-1-yl)-N-hydroxy-3-methyl-butyramid,
2-[3-Benzyl-5-(4-methoxy-phenyl)-2-oxo-imidazolid in-1-yl]-N-hydroxy-3-methyl-butyramid,
N-Hydroxy-2-[3-(4-hydroxy-benzyl)-2-oxo-imidazolidin-1-yl]-3-methyl-butyramid,
N-Hydroxy-2-[3-(3-hydroxy-benzyl)-2-oxo-imidazolidin-1-yl]-3-methyl-butyramid,
N-Hydroxy-3-methyl-2-{2-oxo-3-[4-(pyridin-4-ylmethoxy)-benzyl]-imidazolidin-1-yl}-butyramid mit Trifluoressigsäure (TFA),
N-Hydroxy-3-methyl-2-{2-oxo-3-[4-(pyridin-3-ylmethoxy)-benzyl]-imidazolidin-1-yl}-butyramid mit TFA,
N-Hydroxy-3-methyl-2-{2-oxo-3-[4-(pyridin-2-ylmethoxy)-benzyl]-im idazolidin-1-yl}-butyramid mit TFA,
2-[3-(4-But-2-ynyloxy-benzyl)-2-oxo-imidazolidin-1-yl]-N-hydroxy-3-methyl-butyramid,
N-Hydroxy-3-methyl-2-{3-[4-(2-methyl-quinolin-4-ylmethoxy)-benzyl]-2-oxo-imidazolidin-1-yl}-butyramid mit TFA,
2-[3-Benzyl-5-(4-methoxy-benzyl)-2-oxo-imidazolidin-1-yl]-N-hydroxy-3-methyl-butyramid,
2-[3-(4-Benzyloxy-benzyl)-5-(4-methoxy-benzyl)-2-oxo-imidazolidin-1-yl]-N-hydroxy-3-methyl-butyramid,
N-Hydroxy-2-[5-(4-methoxy-benzyl)-3-methyl-2-oxo-im idazolidin-1-yl]-3-methyl-butyramid,
2-[5-Benzo[1,3]dioxol-5-ylmethyl-3-(4-benzyloxy-benzyl)-2-oxo-imidazolidin-1-yl]-N-hydroxy-3-methyl-butyramid oder
2-(5-Benzo[1,3]dioxol-5-ylmethyl-3-benzyl-2-oxo-imidazolidin-1-yl)-N-hydroxy-3-methyl-butyramid.

5. Verfahren zur Herstellung der Verbindung der Formel I gemäß einem oder mehreren der Ansprüche 1 bis 4, **dadurch gekennzeichnet, dass** man
a) eine Verbindung der Formel II 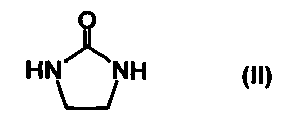 mit einer Verbindung X-Q-R5, worin Q und R5 wie in der Verbindung der Formel I definiert sind und X ein Halogen bedeutet,
in eine Verbindung der Formel III überführt, 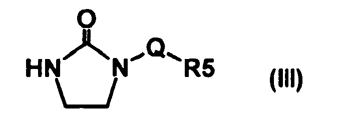 und mit einer Verbindung der Formel IV, 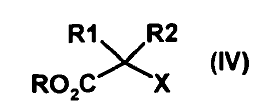 worin R1 und R2 wie in Formel I definiert sind, X ein Halogen und R eine Carboxyl-Schutzgruppe bedeutet,
in eine Verbindung der Formel V überführt, 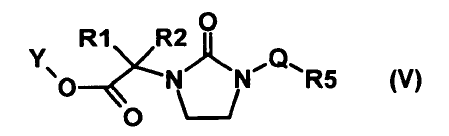 und anschließend die Verbindung der Formel V in die Hydroxamsäure, worin Y = NH-OH ist, der Formel I umwandelt, oder
b) eine Verbindung der Formel VI 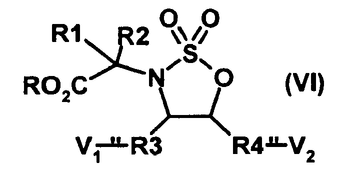 worin R1, R2, R3 und V1 wie in Formel I definiert sind und R eine CarboxySchutzgruppe bedeutet,
mit einer Verbindung NH₂-Q-R5, worin Q und R5 wie in der Verbindung der Formel I definiert sind, in eine Verbindung der Formel VII überführt, 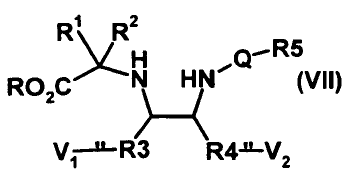 und anschließen mit COCl₂ in eine Verbindung der Formel VIII überführt, 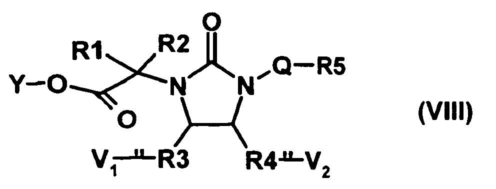 und anschließend die Verbindung der Formel VIII in die Hydroxamsäure, worin Y = NH-OH ist, der Formel I umwandelt, oder
c) eine nach Verfahren a) oder b) hergestellte Verbindung der Formel I, die aufgrund ihrer chemischen Struktur in enantiomeren Formen auftritt, durch Salzbildung mit enantiomerenreinen Säuren oder Basen, Chromatographie an chiralen Stationärphasen oder Derivatisierung mittels chiraler enantiomerenreinen Verbindungen wie Aminosäuren, Trennung der somit erhaltenen Diastereomeren, und Abspaltung der chiralen Hilfsgruppen in die reinen Enantiomeren auftrennt, oder
d) die nach den Verfahren a), b) oder c) hergestellte Verbindung der Formel I entweder in freier Form isoliert oder im Falle des Vorliegens von sauren oder basischen Gruppen in physiologisch verträgliche Salze umwandelt.

6. Arzneimittel, **gekennzeichnet durch** einen wirksamen Gehalt an mindestens einer Verbindung der Formel I gemäß einem oder mehreren der Ansprüche 1 bis 4 zusammen mit einem pharmazeutisch geeigneten und physiologisch verträglichen Trägerstoff, Zusatzstoff und/oder anderen Wirk- und Hilfsstoffen.

7. Verwendung der Verbindung der Formel I gemäß einem oder mehreren der Ansprüche 1 bis 4 zur Herstellung eines Arzneimittels zur selektiven Prophylaxe und Therapie all solcher Erkrankungen, an deren Verlauf eine verstärkte Aktivität der Metalloproteinasen wie Aggrecanase oder TNF-α converting enzyme beteiligt sind, **dadurch gekennzeichnet, dass** die Erkrankung eine degenerative Gelenkerkrankungen wie Osteoarthrosen, Spondylosen, Knorpelschwund nach Gelenktrauma oder längerer Gelenksruhigstellung nach Meniskus- oder Patellaverletzungen oder Bänderrissen ist oder eine Erkrankung des Bindegewebes wie Kollagenosen, Periodontalerkrankungen oder Wundheilungsstörungen ist oder eine chronische Erkrankung des Bewegungsapparates wie entzündliche, immunologisch oder stoffwechselbedingte akute und chronische Arthritiden, Arthropathien oder Myalgien ist oder eine Störungen des Knochenstoffwechsels ist oder eine Ulceration, Atherosklerose, Stenose, Krebserkrankung, Tumormetastasenbildung, Kachexie, Anorexie oder septischer Schock ist.

## Claims

1. A compound of the formula I 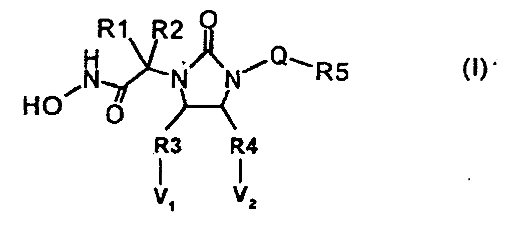 and/or all stereoisomeric forms of the compound of the formula I and/or mixtures of these forms in any ratio, and/or a physiologically tolerated salt of the compound of the formula I, where
R1 and R2 are identical or different and are independently of one another
a) hydrogen atom,
b) -(C₁-C₆)-alkyl,
c) -(C₃-C₆)-cycloalkyl,
d) -(C₂-C₄)-alkyl-Het, in which Het is a mono- or bicyclic 4- to 15-membered heterocycle which comprises at least one carbon atom and one, two, three or four heteroatoms from the series nitrogen, sulfur or oxygen, in which the heterocycle is unsubstituted or substituted once, twice or three times by R8, or
e) -(C₂-C₄)-alkyl-(C₆-C₁₄)-aryl in which aryl is unsubstituted or substituted once or twice by R8, or
R1 and R2 form together with the carbon atom to which they are respectively bonded
a) -(C₃-C₆)-cycloalkyl or
b) a mono- or bicyclic 4- to 15-membered heterocycle which comprises at least one carbon atom and one, two, three or four heteroatoms from the series nitrogen, sulfur or oxygen, in which the heterocycle is unsubstituted or substituted once, twice or three times by R8,
R3 and R4 are identical or different and are independently of one another
a covalent bond, -(CH₂)ₘ-, -(C₁-C₃)-alkylene-O-(C₀-C₃)-alkylene, -(C₀-C₃)-alkylene-C(O)-O-(CH₂)ₙ-, -(C₀-C₃)-alkylene-C(O)-NR¹⁰-(CH₂)ₙ-, -(CH₂)ₘ-CH(OH)-(CH₂)ₙ-, -(C₁-C₃)-alkylene-N(R¹⁰)-(CH₂)ₙ, -(CH₂)ₘ-NR¹⁰-C(O)-(CH₂)ₙ-, -(CH₂)ₘ-NR¹⁰-C(O)-NR¹⁰-(CH₂)ₙ-, -(CH₂)ₘ-O-C(O)-NR¹⁰-(CH₂)ₙ-, -(CH₂)ₘ-NR¹⁰-C(O)-O-(CH₂)ₙ-, -(CH₂)ₘ-S-(CH₂)ₙ-, -(C₁-C₃)-alkylene-S(O)-(CH₂)ₙ-, -(C₁-C₃)-alkylene-SO₂-(CH₂)ₙ-, -(C₁-C₃)-alkylene-SO₂-NH-(R¹⁰), -(CH₂)ₘ-SO₂-NR¹⁰-(CH₂)ₙ-, -(CH₂)ₘ-NR¹⁰-SO₂-(CH₂)ₙ- or -(CH₂)ₘ-NR¹⁰-SO₂-NR¹⁰-(CH₂)ₙ-, in which
n and m are independently of one another identical or different, and m is the integers 1, 2, 3, 4, 5 or 6, and n is the integers zero 1, 2, 3, 4, 5 or 6, and in which the alkylene radicals which are formed by -(CH₂)ₘ- or -(CH₂)ₙ- are unsubstituted or substituted once, twice or three times by halogen, -NH₂ or -OH or form a -(C₃-C₆)-cycloalkyl in which cycloalkyl is unsubstituted or substituted once, twice or three times by halogen, -NH₂ or -OH,
R¹⁰ is hydrogen atom, -(C₁-C₆)-alkyl, -(C₀-C₄)-alkyl-OH, -(C₀-C₄)-alkyl-0-(C₁-C₄)-alkyl or -(C₁-C₃)-perfluoroalkyl,
V₁, V₂ and R5 are identical or different and are independently of one another
a) hydrogen atom,
b) -(C₆-C₁₄)-aryl in which aryl is unsubstituted or substituted once or twice by R8 or the radical -G-M, or
c) a mono- or bicyclic 4- to 15-membered heterocycle which comprises at least one carbon atom and one, two, three or four heteroatoms from the series nitrogen, sulfur or oxygen, in which the heterocycle is unsubstituted or substituted once, twice or three times by R8 or the radical -G-M,
M is
a) hydrogen atom,
b) -(C₆-C₁₄)-aryl in which aryl is unsubstituted or substituted once or twice by R8, or
c) a mono- or bicyclic 4- to 15-membered heterocycle which comprises at least one carbon atom and one, two, three or four heteroatoms from the series nitrogen, sulfur or oxygen, and in which the heterocycle is unsubstituted or substituted once, twice or three times by R8,
R8 is
1) halogen,
2) -NO₂,
3) -CN,
4) -C(O)-NH₂,
5) -SO₂-NH₂,
6) -OH,
7) -NH₂,
8) -O-CF₃,
9) -(C₆-C₁₄)-aryl in which aryl is unsubstituted or substituted once or twice by halogen or -O-(C₁-C₈)-alkyl,
10) -(C₁-C₈)-alkyl in which alkyl is unsubstituted or substituted once, twice or three times by halogen, NH₂, -OH or methoxy,
11) -O-(C₁-C₈)-alkyl in which alkyl is unsubstituted or substituted once, twice or three times by halogen, NH₂, -OH or methoxy,
12) -SO₂-CH₃ or
13) -SO₂-CF₃,
G is covalent bond, -(CH₂)ₒ-, -(C₀-C₃)-alkylene-O-(C₀-C₃)-alkylene-, -(C₀-C₃)-alkylene-C(O)-O-(CH₂)ₚ-, -(C₀-C₃)-alkylene-C(O)-NR¹⁰-(CH₂)ₚ-, -(CH₂)ₒ-CH(OH)-(CH₂)ₚ-, -(C₀-C₃)-alkylene-N(R¹⁰)-(CH₂)ₚ-, -(CH₂)ₒ-NR¹⁰-C(O)-(CH₂)ₚ-, -(CH₂)ₒ-NR¹⁰-C(O)-NR¹⁰-(CH₂)ₚ-, -(CH₂)₀-O-C(O)-NR¹⁰-(CH₂)ₚ-, -(CH₂)ₒ-NR¹⁰-C(O)-O-(CH₂)ₚ-, -(CH₂)ₒ-S-(CH₂)ₚ-, -(C₀-C₃)-alkylene-S(O)-(CH₂)ₚ-, -(C₀-C₃)-alkylene-SO₂-(CH₂)ₚ-, -(C₀-C₃)-alkylene-SO₂-NH-(R¹⁰), -(CH₂)ₒ-SO₂-NR¹⁰-(CH₂)ₚ-, -(CH₂)ₒ-NR¹⁰-SO₂-(CH₂)ₚ-, -(C₀-C₃)-alkylene-O-(C₂-C₄)-alkenylene- or -(CH₂)ₒ-NR¹⁰-SO₂-NR¹⁰-(CH₂)ₚ-, in which
o and p are identical or different and are independently of one another the integers zero, 1, 2, 3, 4, 5 or 6, and in which the alkylene radicals which are formed by -(CH₂)ₒ- or -(CH₂)ₚ- are unsubstituted or substituted once, twice or three times by halogen, -NH₂ or -OH or form a -(C₃-C₆)-cycloalkyl in which cycloalkyl is unsubstituted or substituted once, twice or three times by halogen, -NH₂ or -OH, and R10 is as defined above, and
Q is covalent bond, -(C₁-C₃)-alkylene or -(C₃-C₆)-cycloalkyl, on condition that at least one of the radicals V₁, V₂ or R5 is -(C₆-C₁₄) -aryl or a mono- or bicyclic 4- to 15-membered heterocycle, in which aryl or heterocycle are unsubstituted or substituted once or twice by R8 or the radical -G-M.

2. A compound of the formula I as claimed in claim 1, where
R1 and R2 are identical or different and are independently of one another hydrogen atom or -(C₁-C₄)-alkyl, or
R1 and R2 form together with the carbon atom to which they are respectively bonded -(C₃-C₆)-cycloalkyl,
R3 and R4 are identical or different and are independently of one another
a covalent bond, -(CH₂)ₘ-, -(C₁-C₃)-alkylene-O-(C₀-C₃)-alkylene-, -(C₀-C₃)-alkylene-C(O)-O-(CH₂)ₙ-, -(C₀-C₂)-alkylene-C(O)-NR¹⁰-(CH₂)ₙ-, -(CH₂)ₘ-CH(OH)-(CH₂)ₙ-, -(C₁-C₃)-alkylene-N(R¹⁰)-(CH₂)ₙ-, -(CH₂)ₘ-NR¹⁰-C(O)-(CH₂)ₙ-, -(CH₂)ₘ-NR¹⁰-C(O)-NR¹⁰-(CH₂)ₙ-, -(CH₂)ₘ-O-C(O)-NR¹⁰-(CH₂)ₙ-, -(CH₂)ₘ-NR¹⁰-C(O)-O-(CH₂)ₙ-, -(CH₂)ₘ-S-(CH₂)ₙ-, -(C₁-C₃)-alkylene-S(O)-(CH₂)ₙ-, -(C₁-C₃)-alkylene-SO₂-(CH₂)ₙ-, -(C₁-C₃)-alkylene-SO₂-NH-(R¹⁰), -(CH₂)ₘ-SO₂-NR¹⁰-(CH₂)ₙ-, -(CH₂)ₘ-NR¹⁰-SO₂-(CH₂)n₋ or -(CH₂)ₘ-NR¹⁰-SO₂-NR¹⁰-(CH₂)ₙ-, in which
n and m are independently of one another identical or different, and m is the integers 1, 2, 3, 4, 5 or 6, and n is the integers zero, 1, 2, 3, 4, 5 or 6, and in which the alkylene radicals which are formed by -(CH₂)ₘ- or -(CH₂)ₙ- are unsubstituted or substituted once, twice or three times by halogen, -NH₂ or -OH or form a -(C₃-C₆)-cycloalkyl in which cycloalkyl is unsubstituted or substituted once, twice or three times by halogen, -NH₂ or -OH,
R¹⁰ is hydrogen atom, -(C₁-C₆)₋alkyl, -(C₀-C₄)-alkyl-OH, -(C₀-C₄)-alkyl-O-(C₁-C₄)-alkyl or -(C₁-C₃)-perfluoroalkyl,
V₁, V₂ and R5 are identical or different and are independently of one another
a) hydrogen atom,
b) -(C₆-C₁₄)-aryl in which aryl is a radical from the series phenyl, naphthyl, 1-naphthyl, 2-naphthyl, anthryl or fluorenyl, is unsubstituted or substituted once or twice by R8 or the radical -G-M, or
c) a mono- or bicyclic 4- to 15-membered heterocycle in which heterocycle is a radical from the series acridinyl, azetidinyl, benzimidazolyl, benzodioxol, benzodiazin, benzofuranyl, benzothiofuranyl, benzothiophenyl, benzoxazolyl, benzthiazolyl, benztriazolyl, benztetrazolyl, benzisoxazolyl, benzisothiazolyl, benzimidazalinyl, carbazolyl, beta-carbolinyl, quinazolinyl, quinolinyl, 4H-quinolizinyl, quinoxalinyl, quinuclidinyl, chromanyl, chromenyl, decahydroquinolinyl, dihydrofuran, dithiazinyl, dithiazolly, fuaranyl, furazanyl, imidazolidinyl, imidazolinyl, imidazolyl, 1H-indazolyl, indolinyl, indolizinyl, indolyl, 3H-indolyl, isobenzofuranyl, isochromanyl, isoindazolyl, isoindolinyl, isoindolyl, isoquinolinyl, isothiazolyl, isoxazolyl, morpholinyl, naphthyridinyl, octahydr-isoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyro-azolidinyl, pyrazolinyl, pyrazolyl, pyridazinyl, pryidooxazolyl, pyridoimidazolyl, pyridothiazolyl, pyridothiophenyl, pyridyl, pyrimidinyl, pyrrolidinyl, pyrrolinyl, pyrrolyl, tetrahydrofuranyl, tetrahydroisoquinolinyl, tetrahydroquinolinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, triazinyl, 1,2,3-triazolyl, 1,2,4-triazolyl, 1,2,5-triazolyl, 1,3,4-triazolyl and xanthenyl, in which the heterocycle is unsubstituted or substituted once, twice or three times by R8 or the radical -G-M,
M is
a) hydrogen atom,
b) -(C₆-C₁₄)-aryl in which aryl is as defined above and is unsubstituted or substituted once or twice by R8, or
c) a mono- or bicyclic 4- to 15-membered heterocycle in which
heterocycle is as defined above, and in which the heterocycle is unsubstituted or substituted once, twice or three times by R8,
R8 is
1) halogen,
2) -NO₂,
3) -CN,
4) -C(O)-NH₂,
5) -SO₂-NH₂,
6) -OH,
7) -NH₂,
8) -O-CF₃,
9) -(C₆₋C₁₄)-aryl in which aryl is unsubstituted or substituted once or twice by halogen or -O-(C₁-C₈)-alkyl,
10) -(C₁-C₈)-alkyl in which alkyl is unsubstituted or substituted once, twice or three times by halogen, NH₂, -OH or methoxy,
11) -O-(C₁-C₈)-alkyl in which alkyl is unsubstituted or substituted once, twice or three times by halogen, NH₂, -OH or methoxy,
12) -SO₂-CH₃ or
13) -SO₂-CF₃,
G is covalent bond, -(C₀-C₃)-alkylene-O-(C₀-C₃)-alkylene, -(CH₂)ₒ-CH(OH)-(CH₂)ₚ-, -(C₀-C₃)-alkylene-C(O)-O-(CH₂)ₚ-, -(C₀-C₃)-alkylene-C(O)-NR¹⁰-(CH₂)ₚ-, -(CH₂)ₒ-, -(C₀-C₃)-alkylene-N(R¹⁰)-(CH₂)ₚ-, -(CH₂)ₒ-NR¹⁰-C(O)-(CH₂)ₚ-, -(CH₂)ₒ-O-C(O)-NR¹⁰-(CH₂)ₚ-, (CH₂)ₒ-S-(CH₂)ₚ-, -(CH₂)ₒ-NR¹⁰-C(O)-NR¹⁰-(CH₂)ₚ-, -(CH₂)ₒ-NR¹⁰-C(O)-O-(CH₂)ₚ-, -(C₀-C₃)-alkylene-S(O)-(CH₂)ₚ-, -(C₀-C₃)-alkylene-SO₂-(CH₂)ₚ-, -(C₀-C₃)-alkylene-SO₂-NH-(R¹⁰), -(CH₂)ₒ-SO₂-NR¹⁰-(CH₂)ₚ-, -(CH₂)ₒ-NR¹⁰-SO₂-(CH₂)ₚ-, -(C₀-C₃)-alkylene-O-(C₂-C₄)-alkenylene-, or -(CH₂)ₒ-NR¹⁰-SO₂-NR¹⁰-(CH₂)ₚ-, in which
o and p are identical or different and are independently of one another the integers zero, 1, 2, 3, 4, 5 or 6, and in which the alkylene radicals which are formed by -(CH₂)ₒ- or -(CH₂)ₚ- are unsubstituted or substituted once, twice or three times by halogen, -NH₂ or -OH or form a -(C₃-C₆)-cycloalkyl in which cycloalkyl is unsubstituted or substituted once, twice or three times by halogen, -NH₂ or -OH, and R10 is as defined above, and
Q is covalent bond, -(C₁-C₃)-alkylene or -(C₃-C₆)-cycloalkyl,
on condition that at least one of the radicals V₁, V₂ or R5 is -(C₆-C₁₄)-aryl or a mono- or bicyclic 4- to 15-membered heterocycle, in which aryl or heterocycle are unsubstituted or substituted once or twice by R8 or the radical -G-M.

3. A compound of the formula I as claimed in claims 1 or 2, where
R1 and R2 are identical or different and are independently of one another hydrogen atom or -(C₁-C₄)-alkyl, or
R1 and R2 form together with the carbon atom to which they are respectively bonded -(C₃-C₆)-cycloalkyl,
R3 and R4 are identical or different and are independently of one another
a covalent bond, -(CH₂)ₘ₋ or -(C₁-C₃)-alkylene-O-(C₀-C₃)-alkylene-, in which
m is the integer 1, and in which the alkylene radical which is formed by -(CH₂)ₘ- is unsubstituted or substituted once by -OH,
V₂ is hydrogen atom,
V₁ and R5 are identical or different and are independently of one another
a) hydrogen atom,
b) -(C₆-C₁₄)-aryl in which aryl is phenyl and is unsubstituted or substituted once or twice by R8 or the radical -G-M, or
c) a mono- or bicyclic 4- to 15-membered heterocycle in which heterocycle is a radical from the series benzodioxol, quinolinyl or pyridyl, in which the heterocycle is unsubstituted or substituted once, twice or three times by R8 or the radical -G-M,
M is
a) hydrogen atom,
b) -(C₆-C₁₄)-aryl in which aryl is phenyl and is unsubstituted or substituted once or twice by R8, or
c) a mono- or bicyclic 4- to 15-membered heterocycle in which
heterocycle is as defined above and in which the heterocycle is unsubstituted or substituted once, twice or three times by R8,
R8 is halogen, -OH or -(C₁-C₄)-alkyl or -O-(C₁-C₄)-alkyl,
G is covalent bond, -(C₀-C₃)-alkylene-O-(C₀-C₃)-alkylene- or -(C₀-C₃)-alkylene-O-(C₂-C₄)-alkenylene-, and
Q is covalent bond or -(C₁-C₃)-alkylene,
on condition that at least one of the radicals V₁ or R5 is - (C₆-C₁₄) -aryl or a mono- or bicyclic 4-to 15-membered heterocycle, in which aryl or heterocycle are unsubstituted or substituted once or twice by R8 or the radical -G-M.

4. A compound of the formula I as claimed in one or more of claims 1 to 3, which is the compound
2-[3-(4-benzyloxybenzyl)-2-oxoimidazolidin-1-yl]-N-hydroxy-3-methylbutyramide,
2-[3-(4-benzyloxyphenyl)-2-oxoimidazolidin-1-yl]-N-hydroxy-3-methylbutyramide,
2-[3-(3-benzyloxybenzyl)-2-oxoimidazolidin-1-yl]-N-hydroxy-3-methylbutyramide,
N-hydroxy-3-methyl-2-[2-oxo-3-(4-phenoxybenzyl)imidazolidin-1-yl]-butyramide,
2-[3-(6-benzyloxypyridin-3-ylmethyl)-2-oxoimidazolidin-1-yl]-N-hydroxy-3-methylbutyramide,
2-(3-biphenyl-4-ylmethyl-2-oxoimidazolidin-1-yl)-N-hydroxy-3-methylbutyramide,
2-[3-benzyl-5-(4-methoxyphenyl)-2-oxoimidazolidin-1-yl]-N-hydroxy-3-methylbutyramide,
N-hydroxy-2-[3-(4-hydroxybenzyl)-2-oxoimidazolidin-1-yl]-3-methylbutyramide,
N-hydroxy-2-[3-(3-hydroxybenzyl)-2-oxoimidazolidin-1-yl]-3-methylbutyramide,
N-hydroxy-3-methyl-2-{2-oxo-3-[4-(pyridin-4-ylmethoxy)benzyl]imidazolidin-1-yl}butyramide with trifluoroacetic acid (TFA),
N-hydroxy-3-methyl-2-{2-oxo-3-[4-(pyridin-3-ylmethoxy)benzyl]imidazolidin-1-yl}butyramide with TFA,
N-hydroxy-3-methyl-2-{2-oxo-3-[4-(pyridin-2-ylmethoxy)benzyl]imidazolidin-1-yl}butyramide with TFA,
2-[3-(4-but-2-ynyloxybenzyl)-2-oxoimidazolidin-1-yl]-N-hydroxy-3-methylbutyramide,
N-hydroxy-3-methyl-2-{3-[4-(2-methylquinolin-4-ylmethoxy)benzyl]-2-oxoimidazolidin-1-yl}butyramide with TFA,
2-[3-benzyl-5-(4-methoxybenzyl)-2-oxoimidazolidin-1-yl]-N-hydroxy-3-methylbutyramide,
2-[3-(4-benzyloxybenzyl)-5-(4-methoxybenzyl)-2-oxoimidazolidin-1-yl]-N-hydroxy-3-methylbutyramide,
N-hydroxy-2-[5-(4-methoxybenzyl)-3-methyl-2-oxoimidazolidin-1-yl]-3-methylbutyramide,
2-[5-benzo[1,3]dioxol-5-ylmethyl-3-(4-benzyloxybenzyl)-2-oxoimidazolidin-1-yl]-N-hydroxy-3-methylbutyramide or
2-(5-benzo[1,3]dioxol-5-ylmethyl-3-benzyl-2-oxoimidazolidin-1-yl)-N-hydroxy-3-methylbutyramide.

5. A method for preparing the compound of the formula I as claimed in one or more of claims 1 to 4, which comprises
a) converting a compound of the formula II 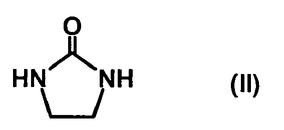 with a compound X-Q-R5 in which Q and R5 are defined as in the compound of the formula I, and X is a halogen,
into a compound of the formula III 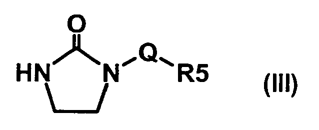 and converting with a compound of the formula IV 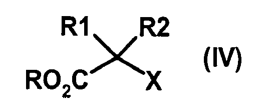 in which R1 and R2 are defined as in formula I, X is a halogen, and R is a carboxyl protective group,
into a compound of the formula V 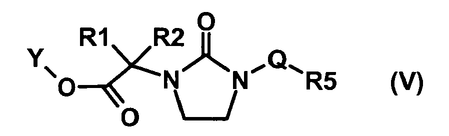 and subsequently converting the compound of the formula V into the hydroxamic acid, in which Y is NH-OH, of the formula I, or
b) converting a compound of the formula VI 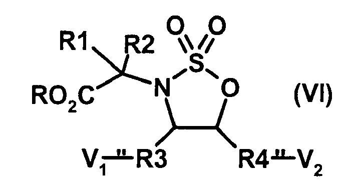 in which R1, R2, R3 and V1 are defined as in formula I, and R is a carboxy protective group, with a compound NH₂-Q-R5 in which Q and R5 are defined as in the compound of the formula I, into a compound of the formula VII 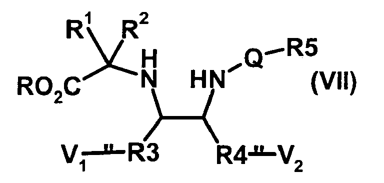 and subsequently converting with COCl₂ into a compound of the formula VIII 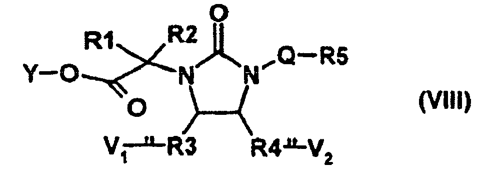 and subsequently converting the compound of the formula VIII into the hydroxamic acid, in which Y is NH-OH, of the formula I, or
c) fractionating a compound of the formula I which has been prepared by method a) or b) and which, owing to its chemical structure, occurs in enantiomeric forms into the pure enantiomers by salt formation with enantiopure acids or bases, chromatography on chiral stationary phases or derivatization using chiral enantiopure compounds such as amino acids, separating the diastereomers obtained thus, and eliminating the chiral auxiliary groups, or
d) either isolating in free form the compound of the formula I which has been prepared by methods a), b), or c) or, in the case where acidic or basic groups are present, converting into physiologically tolerated salts.

6. A medicament having an effective content of at least one compound of the formula I as claimed in one or more of claims 1 to 4 together with a pharmaceutically suitable and physiologically tolerated carrier, additive and/or other active substances and excipients.

7. The use of the compound of the formula I as claimed in one or more of claims 1 to 4 for producing a medicament for the selective prophylaxis and therapy of all disorders in the progression of which an enhanced activity of metalloproteinases such as aggrecanase or TNF-α converting enzyme is involved, wherein the disorder is a degenerative joint disorder such as osteoarthroses, spondyloses, chondrolysis after joint trauma or prolonged joint immobilization after meniscus or patellar injuries or ligament tears, or is a connective tissue disorder such as collagenoses, periodontal disorders or wound-healing disturbances, or is a chronic disorder of the locomotor system such as inflammatory, immunologically or metabolism-related acute and chronic arthritides, arthropathies or myalgias, or is a disturbance of bone metabolism, or is an ulceration, atherosklerosis, stenosis, cancer, tumor metastasis, cachexia, anorexia or septic shock.

## Revendications

1. Composé de formule I 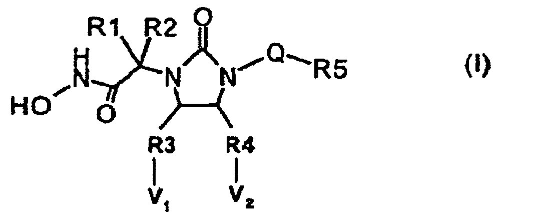 et/ou toutes les formes stéréo-isomères du composé de formule (I) et/ou les mélanges de ces formes, dans tous les rapports, et/ou un sel physiologiquement acceptable du composé de formule (I), où
R1 et R2 sont identiques ou différents et représentent, indépendamment l'un de l'autre
a) un atome d'hydrogène,
b) -(C₁-C₆)-alkyle,
c) -(C₃-C₆)-cycloalkyle,
d) -(C₂-C₄)-alkyl-Het, où Het représente un hétérocycle monocyclique ou bicyclique de 4 à 15 chaînons, qui contient au moins un atome de carbone et un, deux, trois ou quatre hétéroatomes de la série azote, soufre ou oxygène, où l'hétérocycle est non substitué ou monosubstitué, disubstitué ou trisubstitué par R8, ou
e) -(C₂-C₄)-alkyl-(C₆-C₁₄) -aryle, où aryle est non substitué ou monosubstitué ou disubstitué par R8, ou
R1 et R2 forment ensemble avec l'atome de carbone auquel ils sont à chaque fois liés
a) -(C₃-C₆)-cycloalkyle ou
b) un hétérocycle monocyclique ou bicyclique de 4 à 15 chaînons, qui contient au moins un atome de carbone et un, deux, trois ou quatre hétéroatomes de la série azote, soufre ou oxygène, où l'hétérocycle est non substitué ou monosubstitué, disubstitué ou trisubstitué par R8,
R3 et R4 sont identiques ou différents et représentent, indépendamment l'un de l'autre, une liaison covalente, -(CH₂)ₘ-, - (C₁-C₃) -alkylène-O-(C₀-C₃)-alkylène-, -(C₀-C₃)-alkylène-C(O)-O-(CH₂)ₙ-, -(C₀-C₃)-alkylène-C(O)-NR¹⁰- (CH₂)ₙ-, -(CH₂)ₘ-CH(OH)-(CH₂)ₙ-, -(C₁-C₃)-alkylène-N(R¹⁰)-(CH₂ₙ-, -(CH₂)ₘ-NR¹⁰-C(O)-(CH₂)ₙ-, -(CH₂)ₘ-NR¹⁰-C(O)-NR¹⁰-(CH₂)ₙ-, -(CH₂)ₘ-O-C(O)-NR¹⁰-(CH₂)ₙ-, -(CH₂)ₘ-NR¹⁰-C(O)-O-(CH₂)ₙ-, -(CH₂)ₘ-S-(CH₂)ₙ-, -(C₁-C₃)-alkylène-S(O)-(CH₂)ₙ-, -(C₁-C₃)-alkylène-SO₂-(CH₂)ₙ-, -(C₁-C₃)-alkylène-SO₂-NH-(R¹⁰), -(CH₂)ₘ-SO₂-NR¹⁰-(CH₂)ₙ-, -(CH₂)ₘ-NR¹⁰-SO₂- (CH₂)ₙ- ou - (CH₂)ₘ-NR¹⁰-SO₂-NR¹⁰-(CH₂)ₙ- où
n et m indépendamment l'un de l'autre, sont identiques ou différents et m représente les nombres entiers 1, 2, 3, 4, 5 ou 6 et n représente les nombres entiers zéro, 1, 2, 3, 4, 5 ou 6 et les radicaux alkylène, qui sont formés par -(CH₂)ₘ- ou -(CH₂)ₙ-, sont non substitués ou monosubstitués, disubstitués ou trisubstitués par halogène, -NH₂ ou -OH ou forment un -(C₃-C₆)-cycloalkyle, où cycloalkyle est non substitué ou monosubstitué, disubstitué ou trisubstitué par halogène, -NH₂ ou -OH,
R10 représente un atome d'hydrogène, -(C₁-C₆)-alkyle, -(C₀-C₄)-alkyl-OH, -(C₀-C₄)-alkyl-O-(C₁-C₄)-alkyle ou -(C₁-C₃)-perfluoroalkyle,
V₁, V₂ et R5 sont identiques ou différents et représentent, indépendamment l'un de l'autre
a) un atome d'hydrogène,
b) - (C₆-C₁₄) -aryle, où aryle est non substitué ou monosubstitué ou disubstitué par R8 ou le radical -G-M ou
c) un hétérocycle monocyclique ou bicyclique de 4 à 15 chaînons, qui contient au moins un atome de carbone et un, deux, trois ou quatre hétéroatomes de la série azote, soufre ou oxygène, où l'hétérocycle est non substitué ou monosubstitué, disubstitué ou trisubstitué par R8 ou le radical -G-M,
M représente
a) un atome d'hydrogène,
b) - (C₆-C₁₄) -aryle, où aryle est non substitué ou monosubstitué ou disubstitué par R8 ou
c) un hétérocycle monocyclique ou bicyclique de 4 à 15 chaînons, qui contient au moins un atome de carbone et un, deux, trois ou quatre hétéroatomes de la série azote, soufre ou oxygène, où l'hétérocycle est non substitué ou monosubstitué, disubstitué ou trisubstitué par R8,
R8 représente
1) halogène,
2) -NO₂,
3) -CN,
4) -C(O)-NH₂,
5) -SO₂-NH₂,
6) -OH,
7) -NH₂,
8) -O-CF₃ ,
9) -(C₆-C₁₄)-aryle, où aryle est non substitué ou monosubstitué ou disubstitué par halogène ou -O-(C₁-C₈)-alkyle,
10) -(C₁-C₈)-alkyle, où alkyle est non substitué ou monosubstitué, disubstitué ou trisubstitué par halogène, NH₂, -OH ou méthoxy,
11) -O-(C₁-C₈)-alkyle, où alkyle est non substitué ou monosubstitué, disubstitué ou trisubstitué par halogène, NH₂, -OH ou méthoxy,
12) -SO₂-CH₃ ou
13) -SO₂-CF₃,
G représente une liaison covalente, -(CH₂)ₒ-, -(C₀-C₃)-alkylène-O-(C₀-C₃)-alkylène-, -(C₀-C₃)-alkylène-C(O)-O-(CH₂)ₚ-, -(C₀-C₃)-alkylène-C(O)-NR¹⁰- (CH₂)ₚ-, -(CH₂)ₒ-CH(OH)-(CH₂)ₚ-, -(C₀-C₃)-alkylène-N(R¹⁰)-(CH₂)ₚ-, -(CH₂)ₒ-NR¹⁰-C(O) -(CH₂)ₚ-, -(CH₂)ₒ-NR¹⁰-C (O)-NR¹⁰- (CH₂)ₚ-, -(CH₂)ₒ-O-C (O)-NR¹⁰-(CH₂)ₚ-, -(CH₂)ₒ-NR¹⁰-C(O)-O-(CH₂)ₚ-, -(CH₂)ₒ-S-(CH₂)ₚ-, -(C₀-C₃)-alkylène-S(O)-(CH₂)ₚ-, -(C₀-C₃)-alkylène-SO₂-(CH₂)ₚ-, -(C₀-C₃)-alkylène-SO₂-NH-(R¹⁰), -(CH₂)o-SO₂-NR¹⁰-(CH₂)ₚ-, -(CH₂)ₒ-NR¹⁰-SO₂-(CH₂)ₚ-, -(C₀-C₃)-alkylène-O-(C₂-C₄)-alcénylène- ou -(CH₂)ₒ-NR¹⁰-SO₂NR¹⁰-(CH₂)ₚ- où
o et p sont identiques ou différents et représentent, indépendamment l'un de l'autre, les nombres entiers zéro, 1, 2, 3, 4, 5 ou 6 et les radicaux alkylène qui sont formés par -(CH₂)ₒ- ou -(CH₂)ₚ- sont non substitués ou monosubstitués, disubstitués ou trisubstitués par halogène, -NH₂ ou -OH ou forment un -(C₃-C₆)-cycloalkyle, où cycloalkyle est non substitué ou monosubstitué, disubstitué ou trisubstitué par halogène, -NH₂ ou -OH, et R10 est défini comme ci-dessus, et
Q représente une liaison covalente, -(C₁-C₃)-alkylène ou - (C₃-C₆) -cycloalkyle, à condition qu'au moins un des radicaux V1, V2 ou R5 représente -(C₆-C₁₄)-aryle ou un hétérocycle monocyclique ou bicyclique de 4 à 15 chaînons, où aryle ou hétérocycle sont non substitués ou monosubstitués ou disubstitués par R8 ou le radical -G-M.

2. Composé de formule I selon la revendication 1, où
R1 et R2 sont identiques ou différents et représentent, indépendamment l'un de l'autre, un atome d'hydrogène ou -(C₁-C₄)-alkyle, ou
R1 et R2 forment ensemble avec l'atome de carbone auquel ils sont à chaque fois liés -(C₃-C₆)-cycloalkyle,
R3 et R4 sont identiques ou différents et représentent indépendamment l'un de l'autre une liaison covalente, -(CH₂)ₘ₋, -(C₁-C₃)-alkylène-O-(C₀-C₃)-alkylène-, -(C₀-C₃)-alkylène-C(O)-O-(CH₂)ₙ-, -(C₀-C₂)-alkylène-C(O)-NR¹⁰-(CH₂)ₙ-, - (CH₂)ₘ-CH(OH)-(CH₂)ₙ-, -(C₁-C₃)-alkylène-N(R¹⁰)-(CH₂)ₙ-, -(CH₂)ₘ-NR¹⁰-C(O)-(CH₂)ₙ-, - (CH₂)ₘ-NR¹⁰-C(O)-NR¹⁰-(CH₂)ₙ-, -(CH₂)ₘ-O-C(O)-NR¹⁰-(CH₂)ₙ-, -(CH₂)ₘ-NR¹⁰-C(O)-O-(CH₂)ₙ-, -(CH₂)ₘ-S-(CH₂)ₙ-, -(C₁-C₃)-alkylène-S(O)-(CH₂)ₙ-, - (C₁-C₃)-alkylène-SO₂-(CH₂)ₙ-, -(C₁-C₃)-alkylène-SO₂-NH-(R¹⁰), -(CH₂)ₘ-SO₂-NR¹⁰-(CH₂)ₙ-, -(CH₂)ₘ-NR-SO₂-CH₂)ₙ- ou -(CH₂)ₘ-NR¹⁰-SO₂-NR¹⁰-(CH₂)ₙ-, où
n et m indépendamment l'un de l'autre, sont identiques ou différents et m représente les nombres entiers 1, 2, 3, 4, 5 ou 6 et n représente les nombres entiers zéro, 1, 2, 3, 4, 5 ou 6 et les radicaux alkylène, qui sont formés par -(CH₂)ₘ- ou -(CH₂)ₙ-, sont non substitués ou monosubstitués, disubstitués ou trisubstitués par halogène, -NH₂ ou -OH ou forment un -(C₃-C₆)-cycloalkyle, où cycloalkyle est non substitué ou monosubstitué, disubstitué ou trisubstitué par halogène, -NH₂ ou -OH,
R10 représente un atome d'hydrogène, -(C₁-C₆)-alkyle, -(C₀-C₄)-alkyl-OH, -(C₀-C₄)-alkyl-O-(C₁-C₄)-alkyle ou -(C₁-C₃)-perfluoroalkyle,
V₁, V₂ et R5 sont identiques ou différents et représentent, indépendamment l'un de l'autre
a) un atome d'hydrogène,
b) - (C₆-C₁₄) -aryle, où aryle signifie un radical de la série phényle, naphtyle, 1-naphtyle, 2-naphtyle, anthryle ou fluorényle, non substitué ou monosubstitué ou disubstitué par R8 ou le radical -G-M, ou
c) un hétérocycle monocyclique ou bicyclique de 4 à 15 chaînons, où l'hétérocycle signifie un radical de la série acridinyle, azétidinyle, benzimidazolyle, benzodioxole, benzodiazine, benzofurannyle, benzothiofurannyle, benzothiophényle, benzoxazolyle, benzothiazolyle, benzotriazolyle, benzotétrazolyle, benzo-isoxazolyle, benzo-isothiazolyle, benzo-imidazolinyle, carbazolyle, bêta-carbolinyle, quinazolinyle, quinoléinyle, 4H-quinoléizinyle, quinoxalinyle, quinuclidinyle, chromanyle, chroményle, décahydroquinoléinyle, dihydrofuranne, dithiazinyle, dithiazolyle, furannyle, furazanyle, imidazolidinyle, imidazolinyle, imidazolyle, 1H-indazolyle, indolinyle, indolizinyle, indolyle, 3H-indolyle, isobenzofurannyle, isochromanyle, iso-indazolyle, iso-indolinyle, iso-indolyle, isoquinoléinyle, isothiazolyle, isoxazolyle, morpholinyle, naphtyridinyle, octahydro-isoquinoléinyle, oxadiazolyle, 1,2,3-oxadiazolyle, 1,2,4-oxadiazolyle, 1,2,5-oxadiazolyle, 1,3,4-oxadiazolyle, oxazolidinyle, oxazolyle, oxazolidinyle, phénanthridinyle, phénanthrolinyle, phénazinyle, phénothiazinyle, phénoxathiinyle, phénoxazinyle, phtalazinyle, pipérazinyle, pipéridinyle, ptéridinyle, purinyle, pyrannyle, pyrazinyle, pyroazolidinyle, pyrazolinyle, pyrazolyle, pyridazinyle, pyridooxazolyle, pyridoimidazolyle, pyridothiazolyle, pyridothiophényle, pyridyle, pyrimidinyle, pyrrolidinyle, pyrrolinyle, pyrrolyle, tétrahydrofurannyle, tétrahydroisoquinoléinyle, tétrahydroquinoléinyle, 1,2,3-thiadiazolyle, 1,2,4-thiadiazolyle, 1,2,5-thiadiazolyle, 1,3,4-thiadiazolyle, thianthrényle, thiazolyle, thiényle, thiénothiazolyle, thiénooxazolyle, thiénoimidazolyle, triazinyle, 1,2,3-triazolyle, 1,2,4-triazolyle, 1,2,5-triazolyle, 1,3,4-triazolyle et xanthényle, où l'hétérocycle est non substitué ou monosubstitué, disubstitué ou trisubstitué par R8 ou le radical -G-M,
M représente
a) un atome d'hydrogène,
b) -(C₆-C₁₄)-aryle, où aryle est défini comme ci-dessus et est non substitué ou monosubstitué ou disubstitué par R8 ou
c) un hétérocycle monocyclique ou bicyclique de 4 à 15 chaînons, où hétérocycle est défini comme ci-dessus et où l'hétérocycle est non substitué ou monosubstitué, disubstitué ou trisubstitué par R8,
R8 représente
1) halogène,
2) -NO₂,
3) -CN,
4) -C(O)-NH₂,
5) -SO₂-NH₂,
6) -OH,
7) NH₂,
8) -O-CF₃,
9) -(C₆-C₁₄)-aryle, où aryle est non substitué ou monosubstitué ou disubstitué par halogène ou -O-(C₁-C₈)-alkyle,
10) -(C₁-C₈)-alkyle, où alkyle est non substitué ou monosubstitué, disubstitué ou trisubstitué par halogène, NH₂, -OH ou méthoxy,
11) -O-(C₁-C₈)-alkyle, où alkyle est non substitué ou monosubstitué, disubstitué ou trisubstitué par halogène, NH₂, -OH ou méthoxy,
12) -SO₂-CH₃ ou
13) -SO₂-CF₃,
G représente une liaison covalente, -(C₀-C₃)-alkylène-O-(C₀-C₃)-alkylène-, -(CH₂)ₒ-CH(OH)-(CH₂)ₚ-, -(C₀-C₃)-alkylène-C(O)-O-(CH₂)ₚ-, -(C₀-C₃)-alkylène-C(O)-NR¹⁰-(CH₂)ₚ-, -(CH₂)ₒ-, -(C₀-C₃)-alkylène-N(R¹⁰)-(CH₂)ₚ-, -(CH₂)ₒ-NR¹⁰-C(O)-(CH₂)ₚ-, -(CH₂)ₒ-O-C(O)-NR¹⁰-(CH₂)ₚ-, -(CH₂)ₒ-S-(CH₂)ₚ-, -(CH₂)ₒ-NR¹⁰-C(O)-NR¹⁰-(CH₂)ₚ-, - (CH₂)ₒ-NR¹⁰-C(O)-O-(CH₂)ₚ-, -(C₀-C₃)-alkylène-S(O)-(CH₂)ₚ-, -(C₀-C₃)-alkylène-SO₂-(CH₂)ₚ-, -(C₀-C₃)-alkylène-SO₂-NH-(R¹⁰), -(CH₂)ₒ-SO₂-NR¹⁰-(CH₂)ₚ- , -(CH₂)ₒ-NR¹⁰-SO₂-(CH₂)ₚ-, -(C₀-C₃)-alkylène-O-(C₂-C₄)-alcénylène-, ou -(CH₂)ₒ-NR¹⁰- SO₂-NR¹⁰-(CH₂)ₚ- où
o et p sont identiques ou différentes et représentent, indépendamment l'un de l'autre, les nombres entiers zéro, 1, 2, 3, 4, 5 ou 6 et les radicaux alkylène qui sont formés par -(CH₂)ₒ- ou -(CH₂)ₚ- sont non substitués ou monosubstitués, disubstitués ou trisubstitués par halogène, -NH₂ ou -OH ou forment un -(C₃-C₆)-cycloalkyle, où cycloalkyle est non substitué ou monosubstitué, disubstitué ou trisubstitué par halogène, -NH₂ ou -OH, et R10 est défini comme ci-dessus, et
Q représente une liaison covalente, -(C₁-C₃), -alkylène ou -(C₃-C₆)-cycloalkyle, à condition qu'au moins un des radicaux V₁, V₂ ou R5 représente -(C₆-C₁₄)-aryle ou un hétérocycle monocyclique ou bicyclique de 4 à 15 chaînons, où aryle ou hétérocycle sont non substitués ou monosubstitués ou disubstitués par R8 ou le radical -G-M.

3. Composé de formule I selon les revendications 1 ou 2, où
R1 et R2 sont identiques ou différents et représentent, indépendamment l'un de l'autre, un atome d'hydrogène ou - (C₁-C₄) -alkyle, ou
R1 et R2 forment ensemble avec l'atome de carbone auquel ils sont à chaque fois liés - (C₃-C₆) - cycloalkyle,
R3 et R4 sont identiques ou différents et représentent, indépendamment l'un de l'autre, une liaison covalente, - (CH₂)ₘ- ou - (C₁-C₃) -alkylène-O-(C₀-C₃)-alkylène-, où m représente le nombre entier 1, et où le radical alkylène, qui est formé par -(CH₂)ₘ- est non substitué ou monosubstitué par -OH,
V₂ représente un atome d'hydrogène,
V₁ et R5 sont identiques ou différents et représentent, indépendamment l'un de l'autre
a) un atome d'hydrogène,
b) -(C₆-C₁₄) -aryle, où aryle signifie phényle et est non substitué ou monosubstitué ou disubstitué par R8 ou le radical -G-M ou
c) un hétérocycle monocyclique ou bicyclique de 4 à 15 chaînons, où hétérocycle signifie un radical de la série benzodioxole, quinoléinyle ou pyridyle, où l'hétérocycle est non substitué ou monosubstitué, disubstitué ou trisubstitué par R8 ou le radical -G-M,
M représente
a) un atome d'hydrogène,
b) -(C₆-C₁₄)-aryle, où aryle signifie phényle et est non substitué ou monosubstitué ou disubstitué par R8 ou
c) un hétérocycle monocyclique ou bicyclique de 4 à 15 chaînons, où hétérocycle est défini comme ci-dessus et où l'hétérocycle est non substitué ou monosubstitué, disubstitué ou trisubstitué par R8,
R8 représente halogène, -OH ou -(C₁-C₄)-alkyle ou -O-(C₁-C₄) -alkyle,
G représente une liaison covalente, -(C₀-C₃)-alkylène-O-(C₀-C₃)-alkylène- ou - (C₀-C₃)-alkylène-O-(C₂-C₄)-alcénylène-, et
Q représente une liaison covalente ou -(C₁-C₃)-alkylène, à condition qu'au moins un des radicaux V₁ ou R5 représente -(C₆-C₁₄)-aryle ou un hétérocycle monocyclique ou bicyclique de 4 à 15 chaînons, où aryle ou hétérocycle sont non substitués ou monosubstitués ou disubstitués par R8 ou le radical -G-M.

4. Composé de formule I selon l'une ou plusieurs des revendications 1 à 3, **caractérisé en ce qu'**il s'agit du composé
2-[3-(4-benzyloxybenzyl)-2-oxo-imidazolidin-1-yl]-N-hydroxy-3-méthylbutyramide,
2-[3-(4-benzyloxyphényl)-2-oxo-imidazolidin-1-yl]-N-hydroxy-3-méthylbutyramide,
2-[3-(3-benzyloxybenzyl)-2-oxo-imidazolidin-1-yl]-N-hydroxy-3-méthylbutyramide,
N-hydroxy-3-méthyl-2-[2-oxo-3-(4-phénoxybenzyl)-imidazolidin-1-yl]-butyramide,
2-[3-(6-benzyloxypyridin-3-ylméthyl)-2-oxo-imidazolidin-1-yl]-N-hydroxy-3-méthylbutyramide,
2-(3-biphényl-4-ylméthyl-2-oxo-imidazolidin-1-yl)-N-hydroxy-3-méthylbutyramide,
2-[3-benzyl-5-(4-méthoxyphényl)-2-oxo-imidazolidin-1-yl]-N-hydroxy-3-méthylbutyramide,
N-hydroxy-2-[3-(4-hydroxybenzyl)-2-oxo-imidazolidin-1-yl]-3-méthylbutyramide,
N-hydroxy-2-[3-(3-hydroxybenzyl)-2-oxo-imidazolidin-1-yl]-3-méthylbutyramide,
N-hydroxy-3-méthyl-2-{2-oxo-3-[4-(pyridin-4-ylméthoxy)-benzyl]-imidazolidin-1-yl}-butyramide avec de l'acide trifluoroacétique (TFA),
N-hydroxy-3-méthyl-2-{2-oxo-3-[4-(pyridin-3-ylméthoxy)-benzyl]-imidazolidin-1-yl}-butyramide avec du TFA,
N-hydroxy-3-méthyl-2-{2-oxo-3-[4-(pyridin-2-ylméthoxy)-benzyl]-imidazolidin-1-yl}-butyramide avec du TFA,
2-[3-(4-but-2-ynyloxybenzyl)-2-oxo-imidazolidin-1-yl]-N-hydroxy-3-méthylbutyramide,
N-hydroxy-3-méthyl-2-{3-[4-(2-méthylquinoléin-4-ylméthoxy)-benzyl]-2-oxo-imidazolidin-1-yl}-butyramide avec du TFA,
2-[3-benzyl-5-(4-méthoxybenzyl)-2-oxo-imidazolidin-1-yl]-N-hydroxy-3-méthylbutyramide,
2-[3-(4-benzyloxybenzyl)-5-(4-méthoxybenzyl)-2-oxo-imidazolidin-1-yl]-N-hydroxy-3-méthylbutyramide,
N-hydroxy-2-[5-(4-méthoxybenzyl)-3-méthyl-2-oxo-imidazolidin-1-yl]-3-méthylbutyramide,
2-[5-benzo[1,3]dioxol-5-ylméthyl-3-(4-benzyloxybenzyl)-2-oxo-imidazolidin-1-yl]-N-hydroxy-3-méthylbutyramide, ou
2-(5-benzo[1,3]dioxol-5-ylméthyl-3-benzyl-2-oxo-imidazolidin-1-yl)-N-hydroxy-3-méthylbutyramide.

5. Procédé pour la préparation du composé de formule I selon l'une ou plusieurs des revendications 1 à 4, **caractérisé en ce qu'**on transforme
a) un composé de formule II 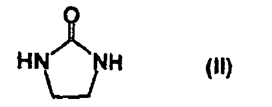 avec un composé X-Q-R5, où Q et R5 sont définis comme dans le composé de formule I et X signifie halogène,
en un composé de formule III 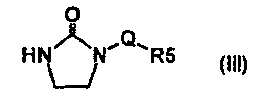 et avec un composé de formule IV 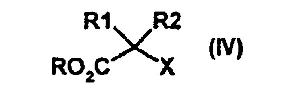 où R1 et R2 sont définis comme dans la formule I, X signifie halogène et R signifie un groupe de protection de la fonction carboxyle,
en un composé de formule V 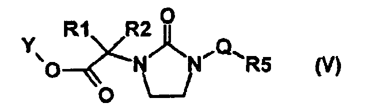 et on transforme ensuite le composé de formule V en acide hydroxamique, où Y = NH-OH, de formule I, ou
b) un composé de formule VI 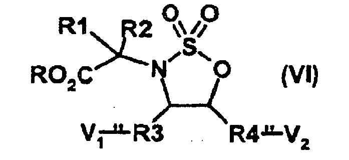 où R1, R2, R3 et V1 sont définis comme dans la formule I et R signifie un groupe de protection de la fonction carboxy,
avec un composé NH₂-Q-R5, où Q et R5 sont définis comme dans le composé de formule I, en un composé de formule VII, 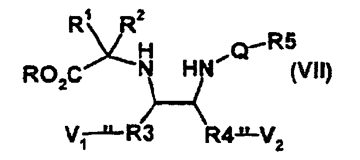 puis avec du COCl₂ en un composé de formule VIII 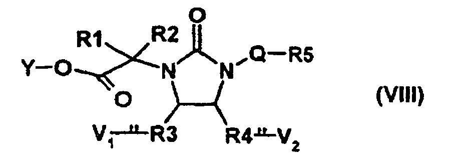 et ce qu'on transforme ensuite le composé de formule VIII en acide hydroxamique, où Y = NH-OH, de formule I, ou
c) on sépare un composé de formule I, préparé selon le procédé a) ou b), qui, en raison de sa structure chimique, apparaît sous des formes énantiomères, par salification avec des acides ou des bases sous forme d'énantiomères purs, chromatographie sur des phases stationnaires chirales ou transformation en dérivés au moyen de composés sous forme d'énantiomères purs, tels que des aminoacides, séparation des diastéréo-isomères ainsi obtenus et élimination des groupes auxiliaires chiraux, en énantiomères purs, ou
d) le composé de formule I, préparé selon les procédés a), b) ou c) est soit isolé sous forme libre, soit, dans le cas de la présence de groupes acides ou basiques, converti en sels physiologiquement acceptables.

6. Médicament, **caractérisé par** une teneur active en au moins un composé de formule I selon l'une ou plusieurs des revendications 1 à 4 avec un support, un additif et/ou d'autres substances actives et adjuvants pharmaceutiquement approprié(s) et physiologiquement compatible(s).

7. Utilisation du composé de formule I selon l'une ou plusieurs des revendications 1 à 4 pour la préparation d'un médicament destiné à la prophylaxie et la thérapie sélective de toutes les maladies à l'évolution desquelles participe une activité renforcée des métalloprotéinases telles que l'Aggrecanase ou l'enzyme de conversion du TNF-α, **caractérisée en ce que** la maladie est une maladie de dégénérescence des articulations, telle que les ostéoarthroses, les spondyloses, l'atrophie du cartilage après un traumatisme articulaire ou une immobilisation prolongée des articulations après des lésions au ménisque ou à la rotule ou des déchirures des ligaments, ou une maladie du tissu conjonctif, tel que les collagénoses, les maladies périodontiques, ou des troubles de guérison de plaies ou une maladie chronique de l'appareil locomoteur telle que les arthrites inflammatoires, immunologiques ou métaboliques, aiguës et chroniques, les arthropathies, ou les myalgies ou un trouble du métabolisme osseux ou une ulcération, une athérosclérose, une sténose, une maladie cancéreuse, une formation de métastases de tumeurs, la cachexie, l'anorexie ou un choc septique.
